(19) Europäisches Patentamt
European Patent Office
Office européen des brevets

(11) EP 4 083 278 A1

(12) EUROPEAN PATENT APPLICATION
published in accordance with Art. 153(4) EPC

(43) Date of publication:
**02.11.2022 Bulletin 2022/44**

(21) Application number: **20886909.9**

(22) Date of filing: **13.11.2020**

(51) International Patent Classification (IPC):
***C40B 40/06*** *(2006.01)* ***C12Q 1/48*** *(2006.01)*
***C12Q 1/6827*** *(2018.01)* ***C12Q 1/6869*** *(2018.01)*
***C12N 9/16*** *(2006.01)* ***G01N 33/48*** *(2006.01)*
***G01N 33/50*** *(2006.01)*

(52) Cooperative Patent Classification (CPC):
**C12N 9/16; C12Q 1/48; C12Q 1/6827; C12Q 1/6869; C40B 40/06; G01N 33/48; G01N 33/50**

(86) International application number:
**PCT/JP2020/042487**

(87) International publication number:
**WO 2021/095866 (20.05.2021 Gazette 2021/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.11.2019 JP 2019207386**
**24.04.2020 JP 2020077622**

(71) Applicant: **Kao Corporation**
**Chuo-ku**
**Tokyo 103-8210 (JP)**

(72) Inventors:
- **OTSUBO, Yuki**
  **Kawasaki-shi, Kanagawa 210-0821 (JP)**
- **MATSUMURA, Shoji**
  **Kawasaki-shi, Kanagawa 210-0821 (JP)**

(74) Representative: **Vossius & Partner**
**Patentanwälte Rechtsanwälte mbB**
**Siebertstraße 3**
**81675 München (DE)**

# (54) METHOD FOR PRODUCING SEQUENCING LIBRARY

(57) Provided is a sequencing library which gives reduced sequencing errors, specifically a method for preparing a sequencing library, the method comprising: fragmenting sample DNA; and treating prepared fragments of the sample DNA with a single-strand-specific nuclease to remove single-stranded moieties from the fragments.



Processed by Luminess, 75001 PARIS (FR)

## Description

Field of the Invention

The present invention relates to a method for preparing a sequencing library.

Background of the Invention

The next-generation sequencing (NGS) technology has significantly developed and has been actively utilized in genomic mutation analysis for cancer cells or the like in recent years, leading to new findings. NGS sequencers which have been frequently used are, for example, the sequencers HiSeq and MiSeq from Illumina, Inc. Sample DNA extracted from cells or tissue as a target of analysis is fragmented into a length of several hundreds of bp, protruding terminals of the fragments are then blunted, and subsequently a sequencing adapter is bound to each terminal to prepare library DNA, and this is sequenced by those sequencers. In the blunting of the terminals, 3' protruding terminals are typically removed by an enzyme such as T4 DNA polymerase, and 5' protruding terminals are, on the other hand, repaired with synthesized strands to be paired with (end repair).

Data from sequencing normally contain errors due to the character of sample DNA or steps for library preparation, and the errors interfere with accurate identification of mutations or the like in a cell population as a target of analysis. For example, sample DNA itself may become single-stranded, for example, by damaging during storage, to result in errors in sequencing. DNA in a formalin-fixed paraffin-embedded (FFPE) sample may be damaged to become single-stranded during sample preparation or storage, and molecules of such single-stranded DNA generate a chimeric fragment through incorrect paring, for example, at parts including repetitive sequences (Patent Literature 1, Non Patent Literature 1). Cell-free DNA (cfDNA) may be decomposed in blood to become single-stranded. Cytosine in single-stranded DNA frequently causes errors in sequencing because of the susceptibility to conversion into thymidine through deamination (Patent Literature 2). To reduce such errors derived from single-stranded DNA, methods of removing single-stranded moieties by treating sample DNA with a single-strand-specific nuclease in the course of library preparation were proposed (Patent Literatures 1, 2, and Non Patent Literature 1). For example, Patent Literature 1 and Non Patent Literature 1 report that treatment of DNA in an FFPE sample with a single-strand-specific nuclease resulted in reduction in the chimeric fragment detection rate and error rate in sequencing. Patent Literature 2 discloses that treatment of cfDNA with a single-strand-specific nuclease allows errors caused by deamination of cytosine in sequencing to be reduced.

In addition, oxidative modification or the like that occurs in DNA as a target of analysis in the course of sample preparation or storage may cause errors in sequencing (Non Patent Literature 2). Kennedy et al. referred to the possibility that the increase in the number of errors in terminal parts of sequence reads is due to the end repair process for fragmented DNA (Non Patent Literature 3).

Methods utilizing information on complementary strands to reduce errors in sequencing have received attention in recent years. For example, because oxidative modification or the like that occurs in DNA in the course of sample preparation or storage occurs only in one of the two strands, identification of mutations fixed in the two complementary strands allows errors due to oxidative modification or the like to be removed (Patent Literatures 3, 4). If a base subjected to oxidative modification is present in a protruding site at a DNA terminal, however, the modified base pairs with an incorrect base in the end repair process, and the incorrect base may be fixed in the two strands of DNA through PCR or the like. Therefore, a base that is present in a protruding site and has undergone the occurrence of oxidative modification or the like may result in an error incapable of being removed even in sequencing utilizing information on complementary strands. Non Patent Literature 3 proposes reduction of errors due to the end repair process for DNA by analysis without five bases from each end of each sequence read.

(Patent Literature 1) WO2015/057985
(Patent Literature 2) WO2019/126803
(Patent Literature 3) WO2013/142389
(Patent Literature 4) WO2019/208827

(Non Patent Literature 1) Nucleic Acids Research, 47(2):e12, 2019
(Non Patent Literature 2) Nucleic Acids Research, 41(6):e67, 2013
(Non Patent Literature 3) Nature Protocols, 9(11):2586-2606, 2014

Summary of the Invention

The present invention provides a method for preparing a sequencing library, the method comprising:

fragmenting sample DNA; and

treating prepared fragments of the sample DNA with a single-strand-specific nuclease to remove single-stranded moieties from the fragments, wherein

the sample DNA is DNA extracted from a living cell, DNA extracted from a frozen cell, or a stored sample of any of the DNAs.

Further, the present invention provides a method for sequencing DNA, the method comprising sequencing the sequencing library.

Furthermore, the present invention provides a method for detecting a mutation in genomic DNA, the method comprising:

preparing a sequencing library by the method for preparing a sequencing library with use of genomic DNA in a cell as sample DNA; and

sequencing the sequencing library.

Brief Description of the Drawings

[Fig. 1]

Figure 1 shows mutation frequencies for six mutation patterns in a DMSO-exposed library, where data are presented as mean and standard deviation for three samples exposed under the same conditions.

[Fig. 2] Figure 2 shows the influence of removal of bases from both terminals of read pairs on mutation frequencies for 12 mutation patterns in a DMSO-exposed library, where data are presented as mean and standard deviation for three samples exposed under the same conditions.

[Fig. 3]

Figure 3 shows the influence of S1 nuclease treatment on mutation frequencies for six mutation patterns in a DMSO-exposed library.

[Fig. 4]

Figure 4 shows mutation frequencies for 12 mutation patterns in a DMSO-exposed library treated with S1 nuclease at different numbers of units.

[Fig. 5]

Figure 5 shows the continuation from Figure 4.

[Fig. 6]

Figure 6 shows the influence of MBN treatment on mutation frequencies for six mutation patterns in a DMSO-exposed library.

[Fig. 7]

Figure 6 shows mutation frequencies for 12 mutation patterns in a DMSO-exposed library treated with MBN at different numbers of units.

[Fig. 8]

Figure 8 shows the continuation from Figure 7.

[Fig. 9]

Figure 9 shows the influence of $RecJ_f$ treatment on mutation frequencies for six mutation patterns in a DMSO-exposed library.

[Fig. 10]

Figure 10 shows mutation frequencies for 12 mutation patterns in a DMSO-exposed library treated with $RecJ_f$ at different numbers of units.

[Fig. 11]

Figure 11 shows the continuation from Figure 10.

[Fig. 12]

Figure 12 shows the influence of S1 nuclease treatment on mutation detection for a sample subjected to mutagen exposure, where mutation frequencies in a DMSO-exposed library (DMSO control) and 3-MC-exposed library (3MC) each treated with S1 nuclease at different numbers of units are presented.

[Fig. 13]

Figure 13 shows the continuation from Figure 12.

[Fig. 14]

Figure 14 shows the influence of MBN treatment on mutation detection for a sample subjected to mutagen exposure, where mutation frequencies in a DMSO-exposed library (DMSO control) and 3-MC-exposed library (3MC) each

treated with MBN at different numbers of units are presented.

[Fig. 15]

Figure 15 shows the influence of $RecJ_f$ treatment on mutation detection for a sample subjected to mutagen exposure, where mutation frequencies in a DMSO-exposed library (DMSO control) and 3-MC-exposed library (3MC) each treated with $RecJ_f$ at different numbers of units are presented.

[Fig. 16]

Figure 16 shows histograms representing genomic coverage in sequencing of a DMSO-exposed library subjected to S1 nuclease treatment, where the horizontal axis represents genomic positions, and the vertical axis represents normalized values of coverage within an interval of approximately 100 bases.

[Fig. 17]

Figure 17 shows histograms representing genomic coverage in sequencing of a DMSO-exposed library subjected to MBN treatment, where the horizontal axis represents genomic positions, and the vertical axis represents normalized values of coverage within an interval of approximately 100 bases.

[Fig. 18]

Figure 18 shows histograms representing genomic coverage in sequencing of a DMSO-exposed library subjected to $RecJ_f$ treatment, where the horizontal axis represents genomic positions, and the vertical axis represents normalized values of coverage within an interval of approximately 100 bases.

[Fig. 19]

Figure 19 shows the influence of S1 nuclease treatment on the frequency of misrecognition of fragments, where the vertical axis represents frequencies of misrecognition of fragments (proportions (%) of cases with inclusion of different indexes in a group of read pairs).

[Fig. 20]

Figure 20 shows the influence of MBN treatment on the frequency of misrecognition of fragments, where the vertical axis represents frequencies of misrecognition of fragments (proportions (%) of cases with inclusion of different indexes in a group of read pairs).

[Fig. 21]

Figure 21 shows the influence of $RecJ_f$ treatment on the frequency of misrecognition of fragments, where the vertical axis represents frequencies of misrecognition of fragments (proportions (%) of cases with inclusion of different indexes in a group of read pairs).

[Fig. 22]

Figure 22 shows the influence of the amount of input DNA on the frequency of misrecognition of fragments subjected to S1 nuclease treatment, where the vertical axis represents frequencies of misrecognition of fragments (proportions (%) of cases with inclusion of different indexes in a group of read pairs).

[Fig. 23]

Figure 23 shows the influence of S1 nuclease + $RecJ_f$ treatment on the frequency of misrecognition of fragments, where the vertical axis represents frequencies of misrecognition of fragments (proportions (%) of cases with inclusion of different indexes in a group of read pairs), and the horizontal axis represents the numbers of units of $RecJ_f$ used.

[Fig. 24]

Figure 24 shows mutation frequencies for six mutation patterns in a DMSO-exposed library subjected to S1 nuclease + $RecJ_f$ treatment, where each legend represents the number of units of $RecJ_f$ used for data represented by the corresponding bar.

## Detailed Description of the Invention

### (1. Definitions)

"Mutation" herein refers to mutation generated in DNA, and examples thereof include deletion, insertion, substitution, addition, inversion, and translocation of a nucleotide or sequence in DNA. The mutation as used herein encompasses deletion, insertion, substitution, and addition of one nucleotide, and deletion, insertion, substitution, addition, inversion, and translocation of a sequence consisting of two or more nucleotides. The mutation as used herein also includes mutation in a gene-coding region and noncoding region, and also includes mutation involving change of an amino acid to be expressed and mutation without involving change of an amino acid to be expressed (silent mutation).

"Genotoxicity" of substance to be evaluated in the present invention refers to a property of the substance to cause mutation (what is called mutagenicity).

A "reference sequence" herein refers to a known sequence included in DNA as a target of analysis. The known sequence to be used is preferably a sequence registered in a public database or the like, a sequence in target DNA which has been sequenced in advance by using a sequencer or the like may also be used as the known sequence. The region, length and number of the reference sequence are not limited, and can be appropriately selected from DNA in

accordance with the purpose of analysis.

An "amplified fragment", which is to be obtained in PCR, herein refers to a double-stranded DNA fragment obtained in PCR amplification of template DNA.

"Two complementary strands" regarding DNA or a fragment thereof herein refer to two single strands constituting double-stranded DNA or a fragment thereof and being complementary to each other.

A "raw read sequence" herein refers to sequence information read out through sequencing of a base sequence. A "read sequence" herein refers to information on a nucleotide sequence targeted in sequencing, which is extracted from the raw read sequence by trimming adapter sequences added for PCR or sequencing reaction or bases of low quality from the raw read sequence. Nevertheless, a raw read sequence can be directly used as a read sequence if the trimming or the like is not needed. In the case that a raw read sequence contains a plurality of pieces of sequence information on base sequences targeted in sequencing, the individual sequence information on the nucleotide sequences targeted in sequencing can be extracted as individual read sequences, respectively, and in this case one or more read sequences can be generated from one raw read sequence. Accordingly, even in the case that an adapter sequence or the like is added to fragments of sample DNA, each read sequence herein fundamentally contains only information on a nucleotide sequence derived from a fragment of sample DNA, without containing sequence information on the adapter sequence or the like. Read sequences each contain information on a sequence starting from a base at one of the terminals of a nucleotide sequence of a target of sequencing (e.g. the nucleotide sequence of a fragment of sample DNA). The length of a read sequence typically depends on the performance and specification of a sequencer. Thus, each read sequence may optionally, but not necessarily need to, contain information on a sequence from a base at one terminal to a base at the other terminal (full sequence) of a nucleotide sequence of a target of sequencing.

The "beginning" and "end" of a read sequence herein refer to a terminal initially read out and terminal finally read out, respectively, in generating the read sequence. "Sequence direction" regarding a read sequence herein refers to the direction from the beginning of the read sequence to the end thereof in a DNA sequence on which the read sequence is mapped.

The situation that two or more read sequences "contain sequence information on the same region in sample DNA" herein refers to a situation that estimated positions of two terminals of the read sequences are identical in the sequence of sample DNA (or a reference sequence). The situation that two or more read sequences "contain sequence information on the same region in sample DNA" does not require a situation that the two or more read sequences are of 100% sequence identity. However, read sequences having different estimated positions of both terminals, even by 1 bp, do not "contain sequence information on the same region in sample DNA".

The situation that two or more read sequences are "mapped on the same position in a reference sequence" herein refers to a situation that on being mapped on a reference sequence, the read sequences have identical positions of two terminals in the reference sequence (wherein their directions are not limited).

A "read pair" herein refers to a pair of two read sequences read out of one sequence of a target of sequencing. One of the two read sequences included in a read pair is a read sequence containing sequence information on reading out of the sequence of a target from the 5'-terminal side to the 3'-terminal side (herein, referred to as "read 1"), and the other is a read sequence containing sequence information on reading out of the sequence of the same one strand from the 3'-terminal side to the 5'-terminal side (herein, referred to as "read 2").

A "region between the beginning of read 1 and the beginning of read 2" in DNA, a sequence, or a fragment herein refers to a region from a site at which the beginning of read 1 is positioned to a site at which the beginning of read 2 is positioned (including the site at which the beginning of read 1 is positioned and the site at which the beginning of read 2 is positioned) in the DNA, sequence, or fragment on which read 1 and read 2 are mapped.

The situation that two or more read pairs "contain sequence information on the same region in sample DNA" herein refers to a situation that a "region between the beginning of read 1 and the beginning of read 2" in the sequence of sample DNA (or a reference sequence) is the same among the read pairs. When two or more read pairs "contain sequence information on the same region in sample DNA", the read sequences of the read pairs are not necessarily required to be of 100% sequence identity. However, read pairs having different terminal position of the "region between the beginning of read 1 and the beginning of read 2", even by 1 bp, do not those "containing sequence information on the same region in sample DNA".

The situation that two or more read pairs are "mapped on the same position in a reference sequence" herein refers to a situation that on being mapped on a reference sequence, the read pairs have identical positions of two terminals in the reference sequence. The "two terminals of a read pair" correspond to the starting positions for reading read 1 and read 2.

The "number of units (U)" of an enzyme herein refers to a unit of the activity (also referred to as catalytic activity) of the enzyme, and the definition varies among enzymes.

All of the patent literatures, the non-patent literatures, and other publications recited herein are totally incorporated herein by reference.

(2. Method for preparing library)

The present invention relates to a method for preparing a sequencing library which gives reduced sequencing errors. The present invention allows reduction of sequencing errors due to the oxidative modification or damage of DNA as a target of analysis that occurs in the course of sample preparation or storage.

If base modification such as oxidative modification has occurred at a single-stranded protruding site in a terminal part of a DNA fragment in the course of sequencing library preparation, pairing between the modified base and the corresponding incorrect base in the end repair process and PCR amplification of the strand having the incorrect base result in preparation of a library having the same information on complementary strands as in the case that mutation has occurred in two complementary strands. Such a library may cause an error incapable of being removed even in sequencing utilizing information on complementary strands. The present inventors found that, in GC → TA and GC → CG mutations in sequencing utilizing information on complementary strands, G → T and G → C mutations are detected highly frequently than C → A and C → G mutations are detected (Figure 1). These mutations of high frequency were considered to be errors due to the oxidative modification of guanine. An estimated cause for the errors was that a single-stranded protrusion was generated at a terminal of sample DNA fragmented in the course of preparation of a sequencing library, and guanine at the single-stranded protruding site underwent oxidative modification (left in Conceptual Diagram 1 shown below).

To remove such errors at terminal protruding sites, the present inventors removed from 10 to 20 bases from each terminal of each read pair obtained through sequencing in accordance with a conventional approach (Non Patent Literature 3). The result showed that the mutation frequencies for G → T and G → C decreased in a manner depending on the number of removed bases (Figure 2). This result indicated that many G → T and G → C mutations are present in both terminal parts of read pairs, supporting that those mutations are errors due to oxidative modification of guanine at terminal single-stranded protruding sites of DNA fragments. However, that conventional approach failed in sufficiently reducing mutations of guanine due to errors, even when 20 bases were removed from each terminal (Figure 2). The increase in the number of bases removed from read pairs is expected to allow errors to be more reduced, whereas the decrease in the number of bases of read pairs lowers the efficiency and accuracy of DNA analysis.

(2-1) Summary

In the present invention, errors in sequencing due to oxidative modification or the like are efficiently reduced in the course of library preparation in such a manner that sample DNA is fragmented by sonicating or the like to prepare DNA fragments, and the fragments of the sample DNA are then treated with a single-strand-specific nuclease to remove their single-stranded moieties (right in Conceptional diagram 1).

[Chem. 1]

Conceptional Diagram 1

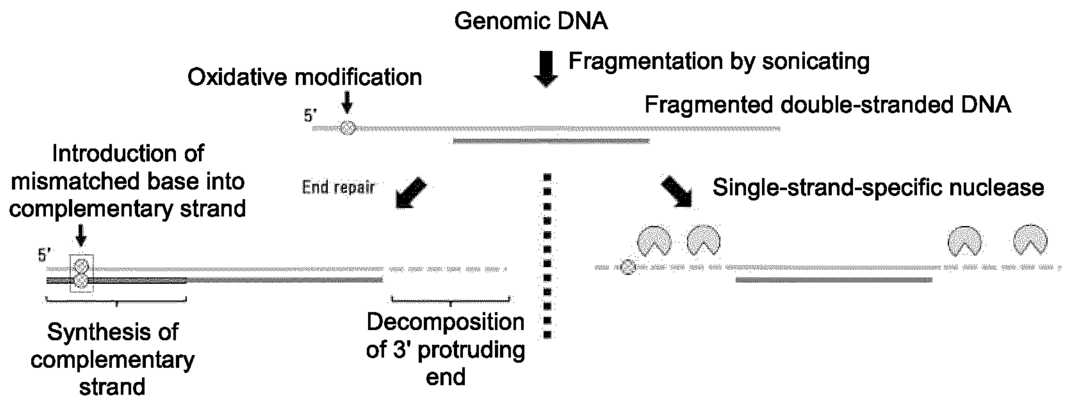


(2-2) Sample DNA

"Sample DNA" to be used in the method for preparing a library according to the present invention is only required to be double-stranded DNA, and examples of the origin thereof include, but are not limited to, animals, plants, and

microorganisms. Examples of types of the sample DNA include, but are not limited to, genomic DNA, mitochondrial genome DNA, chloroplast genome DNA, plasmid DNA, viral genome DNA, and synthesized DNA. Genomic DNA is preferred.

The sample DNA is preferably DNA free of decomposition or damage or having undergone decomposition or damage at low frequency (hereinafter, also referred to as "fresh" DNA) in vivo or in the course of preparation and storage of a cell or tissue sample. The "fresh" DNA preferably refers to DNA being almost completely double-stranded with almost no single-stranded region resulting from decomposition. For example, the "fresh" DNA can be DNA which has not undergone long-term exposure to an environment which promotes the chemical modification or decomposition of DNA, such as DNA extracted from a living cell (e.g., DNA directly extracted from a living body, an organ, a tissue, or a cell collected from any of them, without subjecting to fixing treatment or the like, DNA directly extracted from a cell of a microorganism such as a bacterium), DNA extracted from a frozen cell (e.g., DNA extracted from a cryopreserved living body, DNA extracted from a product obtained by collecting an organ, a tissue, or a cell from a living body and then quickly cryopreserving it), and a stored sample of any of the extracted DNAs (e.g., a sample cryopreserved or stored under low temperature in solvent or inert gas). By contrast, DNA derived from a formalin-fixed cell such as DNA derived from an FFPE sample and DNA which was present in blood for a certain period of time such as cfDNA can be excluded from the scope of the "fresh" DNA. Alternatively, the "fresh" DNA can be defined as such DNA that the DNA Integrity Number (hereinafter, referred to as "DIN") as analyzed with an Agilent 4200 TapeStation or an Agilent 2200 TapeStation (both produced by Agilent Technologies, Inc.) is preferably 6 or more, more preferably 7 or more, even more preferably 7.3 or more, and even more preferably 7.5 or more.

Such sample DNA can be obtained through extraction or isolation from cells using a common method in the art. For the extraction or isolation, for example, commercially available DNA extraction kits can be used. Alternatively, DNA which has been stored after being extracted or isolated from cells may be obtained and used for the method according to the present invention. Synthesized DNA can be obtained through synthesis using any known chemical synthesis method.

Alternatively, double-stranded RNA may be used in place of double-stranded DNA in the method according to the present invention. Double-stranded RNA can be extracted or isolated from viruses or cells possessing it by using a common method in the art such as commercially available RNA extraction kits. Alternatively, double-stranded RNA which has been stored after being extracted or isolated may be obtained and used for the method according to the present invention. In obtaining and analyzing RNA in the method according to the present invention, the RNA obtained is converted into cDNA before PCR, and bases of T in a read sequence derived from the cDNA are read as bases of U.

(2-3) Preparation of DNA fragments

Fragmentation of the sample DNA can be performed by using a common method in the art allowing cutting at random positions, such as sonication and enzymatic treatment. Specific examples of fragmentation treatment for DNA include intensive sonication by using DNA Shearing system from Covaris Inc. The length of each fragment to be prepared can be appropriately selected in accordance with lengths accurately readable for the sequencer. Although from 100 to 10,000 bp can be typically selected, fragments of 10,000 bp or more in length may be prepared if the sequencer can accurately read such fragments, and a more appropriate range can be selected in accordance with the type of the sequencer. In using a sequencer for sequencing reaction involving amplification of fragments, the mean length of fragments is preferably from 100 to 1,000 bp, and more preferably from 200 to 500 bp. Alternatively, longer fragments may be prepared and subjected to PCR described later to prepare PCR products with appropriate lengths for sequencing reaction.

(2-4) Nuclease treatment

In the method according to the present invention, the above-described fresh sample DNA is fragmented, and the resulting fragments are then treated with a single-strand-specific nuclease to remove single-stranded moieties from the fragments. Conventional single-strand-specific nuclease treatment targets DNA which has been comparatively decomposed or damaged, thus being present as fragments in a sample, such as DNA in an FFPE sample and cfDNA. Treating fresh DNA having low degree of decomposition with a single-strand-specific nuclease for library preparation as in the present invention has not been reported yet.

In the present invention, the target of decomposition by a single-strand-specific nuclease can be single-stranded protruding sites typically present at terminals of DNA fragments, but the target is not limited thereto. For example, single-stranded moieties present at nonterminal positions (e.g., center parts) of DNA fragments can be the target of removal by a single-strand-specific nuclease in the present invention, and removal of them can contribute to error reduction. For example, if a nick is present in one strand of a double-stranded DNA fragment, the strand after the nick is resynthesized in an end repair process described later (e.g., treatment with an enzyme in End Repair Cocktail), which may contribute

to the increase in the error rate. Removal of single-stranded moieties resulting from such nicks by a single-strand-specific nuclease can contribute to error reduction.

The single-strand-specific nuclease applicable to the method according to the present invention may be an endonuclease or an exonuclease, as long as the endonuclease or exonuclease acts in a single-strand-specific manner. Examples of single-strand-specific endonuclease include S1 nuclease and Mung Bean Nuclease (MBN), examples of single-strand-specific exonuclease include Exonuclease VII, and examples of single-strand-specific 5' → 3' exonuclease include $RecJ_f$. Among them, S1 nuclease and MBN are preferred because of the high specificity to single strands and the capability to remove even a single strand sandwiched between double strands, and S1 nuclease is more preferred. These single-strand-specific nucleases are commercially available, and can be purchased, for example, from Promega Corporation, Takara Bio Inc., and New England Biolabs. In the single-strand-specific nuclease treatment, only one enzyme may be used, and a plurality of enzymes may be used in combination. For example, it is preferable to treat fragments of the sample DNA with an endonuclease such as S1 nuclease and MBN and then further treat the fragments with an exonuclease such as $RecJ_f$, or vice versa.

The treatment of fragments of the sample DNA with a single-strand-specific nuclease can be performed by using a common procedure, for example, in accordance with a protocol provided by the supplier. Appropriate reaction conditions can be determined in view of optimum conditions for the enzyme and the amount of DNA fragments as the substrate. From the viewpoint of sequencing error reduction, for example, the enzymatic activity unit per 1 ng of fragments of the sample DNA (number of units; U/ng) in reaction solution for S1 nuclease is preferably 0.01 U/ng or more, more preferably 0.02 U/ng or more, even more preferably 0.03 U/ng or more, even more preferably 0.05 U/ng or more, and even more preferably 0.10 U/ng; from the viewpoints of the actually achievable upper limit value and decomposition of double-stranded DNA which occurs in a nonspecific manner in the case of high concentration, on the other hand, the enzymatic activity unit is preferably 16.7 U/ng or less; from the viewpoint of reaction efficiency, the enzymatic activity unit is preferably 5.00 U/ng or less, more preferably 1.67 U/ng or less, even more preferably 1.00 U/ng or less, and even more preferably 0.30 U/ng or less. From the viewpoint of the efficiency of the enzymatic reaction, the range of the amount of the enzyme S1 nuclease is preferably from 0.02 to 5.00 U/ng, more preferably from 0.03 to 1.67 U/ng, even more preferably from 0.03 to 1.00 U/ng, even more preferably from 0.05 to 1.00 U/ng, and even more preferably from 0.10 to 0.30 U/ng. From the viewpoint of sequencing error reduction, for example, the amount of the enzyme MBN in reaction solution is preferably 0.01 U/ng or more, more preferably 0.02 U/ng or more, even more preferably 0.03 U/ng or more, even more preferably 0.05 U/ng or more, and even more preferably 0.10 U/ng or more; from the viewpoints of the actually achievable upper limit value and decomposition of double-stranded DNA which occurs in a nonspecific manner in the case of high concentration, on the other hand, the amount of the enzyme MBN is preferably 16.7 U/ng or less; from the viewpoint of reaction efficiency, the amount of the enzyme MBN is preferably 5.00 U/ng or less, more preferably 1.67 U/ng or less, even more preferably 1.00 U/ng or less, and even more preferably 0.30 U/ng or less. From the viewpoint of the efficiency of the enzymatic reaction, the range of the amount of the enzyme MBN is preferably from 0.02 to 5.00 U/ng, more preferably from 0.03 to 1.67 U/ng, even more preferably from 0.03 to 1.00 U/ng, even more preferably from 0.05 to 1.00 U/ng, and even more preferably from 0.10 to 0.30 U/ng. From the viewpoint of sequencing error reduction, for example, the amount of the enzyme $RecJ_f$ in reaction solution is preferably 0.10 U/ng or more, and more preferably 0.30 U/ng or more; from the viewpoint of the actually achievable upper limit value, on the other hand, the amount of the enzyme $RecJ_f$ is preferably 100 U/ng or less; from the viewpoint of performing the enzymatic reaction with the amount of DNA being close to that recommended (60 ng), the amount of the enzyme $RecJ_f$ is preferably 16.7 U/ng or less; from the viewpoint of reaction efficiency, the amount of the enzyme $RecJ_f$ is preferably 1.00 U/ng. From the viewpoint of the efficiency of the enzymatic reaction, the range of the amount of the enzyme $RecJ_f$ is preferably from 0.10 to 16.7 U/ng, and more preferably from 0.30 to 1.00 U/ng. In the present specification, one enzymatic activity unit (1 U) is defined as follows:

- S1 nuclease: an enzymatic activity such that 1 μg of acid-soluble substance is generated per minute at 37°C in a mixed solution of 30 mM sodium acetate (pH 4.6, 25°C), 50 mM NaCl, 1 mM $ZnCl_2$, 5% glycerol, and 0.5 mg/mL denatured calf thymus DNA.
- MBN: an enzymatic activity such that 1 μg of acid-soluble decomposition product is generated per minute at 37°C and pH 5.0 with thermally denatured calf thymus DNA as a substrate.
- $RecJ_f$: an enzymatic activity such that 0.5 ng of deoxyribonucleotide soluble in trichloroacetic acid is generated per minute at 37°C in 50 μL of whole reaction solution (containing 1× NE Buffer 2 and 1.5 μg of sonicated [$^3$H]-labeled single-stranded E. coli DNA).

Moreover, the number of units of an enzyme to be used for reaction may relate to the amount of input DNA in an amplification (PCR) step described later. For S1 nuclease, for example, if the number of units is more than 0.05 U/ng, an index calculated by the following equation:

$$\text{Index} = \text{amount of input DNA (amol/Mbp sample DNA)} \times 3^{\text{log S1 nuclease (U/ng)}}$$

wherein S1 nuclease (U/ng) > 0.05, and log denotes common logarithm, is preferably 60 or less, more preferably 30 or less, even more preferably 15 or less, and even more preferably 7.5 or less. For MBN, for example, if the number of units is more than 0.05 U/ng, an index calculated by the following equation:

$$\text{Index} = \text{amount of input DNA (amol/Mbp sample DNA)} \times 3^{\text{log MBN (U/ng)}}$$

wherein MBN (U/ng) > 0.05, and log denotes common logarithm, is preferably 60 or less, more preferably 30 or less, even more preferably 15 or less, and even more preferably 7.5 or less. If 0.05 U/ng or less of S1 nuclease or MBN is used, or $RecJ_f$ is used irrespective of the number of units, the equations do not work, and the amount of input DNA in the amplification (PCR) step described later is preferably 250 amol or less, more preferably 125 amol or less, even more preferably 62.5 amol or less, even more preferably 31.3 amol or less, and even more preferably 15.7 amol or less per 1 Mbp of the sample DNA.

It is desirable that the enzyme after reaction be inactivated or removed by washing. DNA fragments subjected to nuclease treatment are purified so that the DNA fragments can be used for the subsequent PCR step. For the purification of DNA, a common technique can be used, such as ethanol precipitation, electrophoresis, column purification, beads purification, and affinity purification.

(2-5) Additional treatment

In the present invention, after the single-strand-specific nuclease treatment for the fragments of the sample DNA, a sequencing library can be prepared in accordance with a common procedure. For example, the DNA fragments subjected to the single-strand-specific nuclease treatment is subjected to treatments including end repair, addition of a base to a terminal, and amplification, as necessary, to prepare a library. Preferably, all of the end repair, addition of a base to a terminal, and amplification are performed in the presented order. The steps of the end repair, addition of a base to a terminal, and amplification can be performed by using a commercially available reagent such as a TruSeq Nano DNA Library Prep Kit (Illumina, Inc.).

(2-5-1) End repair

Each fragment of the sample DNA may have a short single-stranded protruding site remaining at a terminal even after being treated with a single-strand-specific nuclease. The end repair blunts such terminals having a remaining single-stranded protruding site in the DNA fragments after the nuclease treatment. In the blunting treatment, 3'-side protruding terminals are removed by 3' → 5' exonuclease such as T4 DNA polymerase in normal cases; for 5'-side protruding terminals, on the other hand, strands to be paired are synthesized by 5' → 3' polymerase; thereby, both terminals of each DNA fragment are blunted.

(2-5-2) Addition of base

The addition of a base to a terminal is treatment for each terminal-blunted DNA fragment to add a label sequence necessary for sequencing to both terminals or add adenine to the 3'-terminal for adding the label sequence. Amplification of a DNA fragment including a label sequence added thereto and sequencing the resultant allow acquisition of sequence information on the DNA fragment and information on the label sequence, and allow read sequences to be identified or classified on the basis of the information on the label sequence. For example, a label sequence added to each terminal of a DNA fragment serves as an indicator to determine whether a read sequence has information on the whole sequence of the DNA fragment. Alternatively, addition of a label sequence to one terminal of a DNA fragment and sequencing the resultant from the terminal without the label sequence allow determination of whether a read sequence has information on the whole sequence of the fragment of the sample DNA.

In preparing a library for use in sequencing utilizing information on complementary strands, it is preferred to add a label sequence which allows identifying which of two complementary strands of a fragment of the sample DNA a read sequence is derived from to both terminals of each fragment of the sample DNA. For example, different label

sequences are added to the 5'-terminal side and 3'-terminal side of each of the two complementary strands constituting one DNA fragment. In one embodiment, the label sequences in the 5'-terminal side are identical and the label sequences in the 3'-terminal side are also identical between one and the other strand of one DNA fragment, and label sequences added to both terminals of the DNA fragment include sequences not complementary to each other (hereinafter, this is referred to as "label sequences for complementary strands", see Conceptual Diagram 2 shown below). Preferably, in the label sequences for complementary strands, a common label sequence is present in the 5'-terminal side in the DNA fragments labeled, and, similarly, a common label sequence is present in the 3'-terminal side in the DNA fragments labeled. Thus, two single strands constituting each fragment each include different label sequences in the 5'-terminal side and the 3'-terminal side, and the label sequences included in the 5'-terminal side are in common and the label sequences included in the 3'-terminal side are in common among one or the other single strands. On the other hand, it is not required for the label sequences for complementary strands to allow identifying which individual fragment of the sample DNA the read sequence is derived from. Examples of such label sequences for complementary strands include adapter sequences attached to a TruSeq from Illumina, Inc.

In another embodiment, a label sequence that allows individual identification of fragments of the sample DNA (hereinafter, this is referred to as "label sequence for individual fragments"; e.g., a tag sequence unique to fragments of sample DNA as described in PNAS, 109(36): 14508-14513, 2012, or Patent Literature 1) can be added to each DNA fragment. Such labeling allows identifying which of two complementary strands of a DNA fragment a read sequence is derived from, enabling sequencing utilizing information on complementary strands. However, it is preferred from the viewpoint of the efficiency of sequencing utilizing information on complementary strands, particularly in the case of sample DNA of large size, to use label sequences for complementary strands.

(2-5-3) Amplification

For amplification of DNA fragments, an existing method such as PCR can be used. Amplified fragments obtained can be purified with a common procedure, as necessary, and used as a sequencing library. PCR can be performed in accordance with a conventional technique using commercially available reagents and apparatuses for PCR. Alternatively, a sequencer equipped with a PCR amplifier may be used. Such high-throughput sequencers involving PCR amplification of sample DNA fragments in their procedure are available on the market as e.g., HiSeq (produced by Illumina, Inc.) and MiSeq (produced by Illumina, Inc.).

In the PCR, two or more amplified fragments are preferably produced for each of the DNA fragments used as templates. Here, the two or more amplified fragments may be prepared for each of at least some of the fragments of the sample DNA used as templates for the PCR. The two or more amplified fragments may be obtained for all of the fragments of the sample DNA used as templates in the PCR, however, which is not required. Using a certain amount of PCR products is recommended in sequencing reaction with high-throughput sequencers involving PCR amplification of sample DNA fragments in their procedure, from the viewpoint of sequencing efficiency. Accordingly, it is preferred to set the amount of PCR products to the recommended level by changing the number of PCR cycles in accordance with the amount of the sample DNA to be subjected to PCR (amount of input DNA in PCR).

(3. Sequencing method)

Sequencing can be performed by using the library obtained in the above procedure. The library obtained in the present invention is applicable to various sequencing methods. The library obtained in the present invention is preferably used for sequencing utilizing information on complementary strands (e.g., a sequencing method described in Patent Literature 4). With reference to Patent Literature 4, the summary of a sequencing method utilizing information on complementary strands using the library obtained in the present invention (hereinafter, referred to as the present sequencing method) will be described in the following.

(3-1) Summary

Fundamentally, the present sequencing method comprises: sequencing the library obtained in the present invention to generate one or more reading results (read sequences) for each of a plurality of amplified fragments, which are derived from one fragment of the sample DNA, contained in the library, thereby acquiring a plurality of read sequences for the plurality of amplified fragments; collecting, from the read sequences acquired through the sequencing, read sequences containing sequence information on the same region in the sample DNA; and constructing sequence information on the sample DNA by using information on the collected read sequences.

(3-2) Sequencing and generation of read sequences

It is sufficient to conduct the sequencing of the library for a region required for analysis or the like, e.g., for a region to be used for sequence comparison with a reference sequence in the case of mutation analysis described later. For example, it is sufficient to sequence fragments having a region at least partly, preferably totally corresponding to a DNA region of reference sequence. In the case of using mammalian cells or the like, sequencing may be selectively performed for exonic regions. For selection of a region, kits such as a SureSelect (produced by Agilent Technologies, Inc.) are available on the market.

Through the sequencing, raw read sequences for the library are acquired. Adapter sequences added for PCR or sequencing reaction or bases of low quality are trimmed from the raw read sequences to extract a sequence derived from the fragments of the sample DNA, thus generating read sequences. Or, raw read sequences may be directly used as read sequences if the trimming or the like is not needed. The amplified fragments from which the raw read sequences or read sequences derived may be a plurality of amplified fragments of at least some of the amplified fragments contained in the library. The read sequences may be acquired for all of the amplified fragments contained in the library, however, which is not required. One or more read sequences are generated for each of the plurality of amplified fragments. The one or more read sequences thus generated for each one of the amplified fragments contain sequence information on the amplified fragment (i.e., one of two complementary strands of one fragment of the sample DNA from which it derived). Thus, a plurality of read sequences is acquired as a result of sequencing the library. Herein, data including the plurality of read sequences acquired at this stage are occasionally referred to as "sequencing data".

(3-3) Grouping of read sequences

Subsequently, read sequences containing sequence information on the same region in the sample DNA are collected from the plurality of read sequences acquired, on the basis of sequence information on each read sequence. The collected read sequences are put into a group. Accordingly, a "group of read sequences" to be generated in the method according to the present invention is a group of read sequences containing sequence information on the same region in the sample DNA, in other words, a group of read sequences estimated to be derived from the same fragment of the sample DNA. In the method according to the present invention, one or more groups of read sequences can be generated, typically in a manner depending on the number of fragments of sample DNA subjected to PCR in library preparation and the amount of sequencing data.

In an embodiment of the method according to the present invention, one or more read sequences are generated for one amplified fragment contained in the library, and the read sequences acquired are put into a group as described above. In a preferred embodiment, read sequences to be used to generate each of the above-described groups of read sequences are read sequences containing information on the whole sequence of the original fragment of the sample DNA (i.e., the fragment of the sample DNA as an origin of amplified fragments from which the read sequences are derived). Examples of procedures to select the read sequences containing information on the whole sequence of an original fragment of the sample DNA out of the read sequences acquired through sequencing include a method of selecting read sequences with high reading accuracy (quality value) for a base at the end, and a method of preparing a library including a label sequence added at each terminal and sequencing the library followed by selecting read sequences on the basis of the presence or absence of information on the label sequence. Among them, an example of the method using a label sequence will be described hereinafter in more detail: first, different label sequences are added to both terminals of each of the fragments of the sample DNA, and the resulting fragments of the sample DNA are subjected to PCR amplification to prepare a library including amplified fragments including the label sequence at each terminal; and the library obtained is subjected to sequencing to acquire read sequences derived from each of the amplified fragments and information on the label sequences accompanying thereto. A read sequence accompanied by information on both of the label sequences at both terminals is regarded as a read sequence containing information on the whole sequence of the original fragment of the sample DNA. In another example, a label sequence is added to one terminal of each of the fragments of the sample DNA, and the resulting fragments of the sample DNA are subjected to PCR amplification to prepare amplified fragments each including the label sequence; and the amplified fragments obtained are subjected to sequencing from the respective terminals without the label sequence. A read sequence accompanied by information on the label sequence is regarded as a read sequence containing information on the whole sequence of the original fragment of the sample DNA. Here, the information on the label sequence(s) may be acquired from raw read sequences, or from sequence information on a sequencing primer.

Examples of ways to generate groups of read sequences from the collected read sequences include a method of collecting read sequences to be mapped on the same position in a reference sequence, and a method of collecting read sequences identical in sequences at least in both terminal regions. The phrase "identical in sequences at least in both terminal regions" means that the read sequences have sequence identity of 80% or higher, preferably 90% or higher, more preferably 95% or higher, even more preferably 97% or higher, at least in both terminal regions, and both

terminals thereof are aligned at identical positions among the read sequences. The length of the "terminal region" can be appropriately selected, and, for example, may be a length of 10 bases or more, preferably of about from 10 to 30 bases, with the terminal included. Alternatively, groups of read sequences may be generated by collecting read sequences such that the whole-sequence identity thereof is 80% or higher, preferably 90% or higher, more preferably 95% or higher, even more preferably 97% or higher and both terminals thereof are aligned at identical positions among the read sequences.

(3-4) Extraction of sequence information on sample DNA from groups of read sequences

Next, sequence information on the sample DNA is extracted from the groups of read sequences acquired. Specifically, one sequence data is derived for each of the groups of read sequences by using information on read sequences included in the group. The sequence data acquired represents a consensus sequence for a certain fragment of the sample DNA from which the read sequences in the group are derived.

For example, one sequence data can be generated by building consensus of sequence information among read sequences included in a group of read sequences. Examples of specific techniques to build consensus among read sequences include: a method in which read sequences are aligned, and if all the aligned read sequences have a common base at the same position, the base is regarded as a "consensus base"; a method in which read sequences are aligned, and the most frequent base is then determined at each position of the sequence and extracted as a "consensus base"; a method in which read sequences are aligned, and a base for which a sequencer provides the highest reading accuracy (quality value) among bases at the same position is employed as a "consensus base"; a method in which read sequences are aligned, and "consensus bases" are stochastically determined on the basis of quality values, frequencies of appearance of bases, and so forth; and a method of combination of any of them.

In building consensus among read sequences, all of the read sequences included in a group of read sequences may be used; however, only some of the read sequences in the group may be used. Building of consensus among read sequences can exclude errors including read errors in sequencing, thereby providing highly accurate reading results. The resulting sequence data can be acquired as final sequence data representing the sequence of a region of the sample DNA.

(3-5) Sequencing based on information on complementary strands

Base substitution due to oxidative modification of DNA or the like, which causes sequencing error, generally occurs only in one of two strands of DNA. Therefore, use of sequencing information on two complementary strands of DNA enables selective identification of true mutations fixed in the two strands, without detecting a base substitution which occurred only in one strand as a mutation. The sequences of two complementary strands of DNA share equivalent information, in spite of their complementarity. Hence, information on complementary strands can be acquired, theoretically, by searching for sequences containing equivalent information from read sequences acquired through sequencing. If the sample DNA is prepared from a genome sequence of a certain biological species, for example, two read sequences which are derived from two complementary strands constituting a fragment of the sample DNA, and correspond to the same read region, are mapped on the same position in the genome when being mapped on a reference sequence for the biological species which is a subject of analysis. Therefore, read sequences derived from each of two complementary strands can be acquired by collecting read sequences to be mapped on the same position of the genome, and screening the collected read sequences with consideration of complementary strands from which they are derived. Further, consensus is built among the read sequences derived from each of two complementary strands, and thus highly accurate read information reflecting information on a complementary strand can be acquired.

In the present sequencing method, read sequences are generated for each of two complementary strands constituting each of the fragments of the sample DNA in sequencing the library described in (3-2) above. More specifically, in generating read sequences for each of the plurality of amplified fragments contained in the library by sequencing the library, one or more read sequences are generated for each of amplified fragments derived from each of two complementary strands constituting each of the fragments of the sample DNA. That is, two or more read sequences are acquired for one fragment of the sample DNA, and the read sequences contain sequence information on one and the other of two complementary strands of the fragment of the sample DNA from which the read sequences derived.

Subsequently, one or more groups of read sequences are generated from the plurality of read sequences acquired. The way to generate groups of read sequences is as described in (3-3) above. Groups of read sequences to be acquired here each include read sequences containing sequence information on one and the other of two complementary strands of a certain fragment of the sample DNA. Therefore, sequence data reflecting information on complementary strands can be generated by building consensus of sequence information among read sequences included in each of the groups of read sequences. The technique to build consensus among read sequences is specifically as described in (3-4) above. In building consensus among read sequences, all of the read sequences included in a group

of read sequences may be used; however, only some of the read sequences included in the group may be used.

The step of building consensus among read sequences preferably includes collecting, from a group of read sequences, at least one read sequence derived from each of two complementary strands of a fragment of the sample DNA, and building consensus of sequence information among the collected read sequences. Thereby, consensus data using information on complementary strands (herein, also referred to as "consensus read sequence considering complementary strands") can be acquired. The consensus read sequence considering complementary strands thus acquired, which is a highly accurate reading in which errors which occur only in one strand such as read errors in sequencing or errors due to oxidative modification of DNA or the like are excluded, can be acquired as final sequence data representing the sequence of one fragment of the sample DNA.

Examples of procedures to collect read sequences derived from each of two complementary strands of a fragment of the sample DNA include the following: a label sequence, which allows two complementary strands of each of the fragments of the sample DNA to be distinguished, is added to the fragments of the sample DNA in advance to prepare amplified fragments each including the label sequence; the amplified fragments are then subjected to sequencing to acquire read sequences derived from each of the amplified fragments and information on the label sequence accompanying thereto; groups of read sequences are generated from the read sequences acquired; and read sequences derived from each of strands complementary to each other are then collected from each of the groups of read sequences with use of the information on the label sequence accompanying to each read sequence.

In the above procedure, the label sequence described in (2-5-2) in the above (e.g., a label sequence for complementary strands or a label sequence for individual fragments), which allows identifying which of two complementary strands of the fragment a read sequence is derived from, is preferably added to each fragment of the sample DNA. Preferably, a label sequence for complementary strands is used. By sequencing amplified fragments obtained from fragments of the sample DNA each including the label sequences added thereto, read sequences derived from the amplified fragments and information on label sequences accompanying thereto can be acquired.

Next, a preferred procedure in collecting read sequences derived from each of strands complementary to each other from a group of read sequences by using information on the label sequences will be described. The read sequences included in a group of read sequences are mapped on a reference sequence. A read sequence accompanied by information on the label sequence in the 5'-terminal side and positioned in the reference sequence such that the beginning is positioned in the 5'-terminal side to the end, and a read sequence accompanied by information on the label sequence in the 3'-terminal side and positioned in the reference sequence such that the beginning is positioned in the 3'-terminal side to the end are derived from the same one single strand of two complementary strands of a fragment of the sample DNA. On the other hand, a read sequence accompanied by information on the label sequence in the 3'-terminal side and positioned in the reference sequence such that the beginning is positioned in the 5'-terminal side to the end, and a read sequence accompanied by information on the label sequence in the 5'-terminal side and positioned in the reference sequence such that the beginning is positioned in the 3'-terminal side to the end are derived from the other single strand of two complementary strands of the fragment of the sample DNA. Thus, which of two complementary strands constituting a fragment of the sample DNA each read sequence in a group of read sequences is derived from can be identified on the basis of the location in a reference sequence of the read sequence mapped on the reference sequence and information on the accompanying label sequences. Alternatively, sequencing reaction which is initiated only when a specific label sequence has been added to a terminal of an amplified fragment allows identification of a read sequence derived from a certain single strand of each fragment of the sample DNA on the basis of information on the label sequence. Thus, read sequences derived from each of strands complementary to each other can be collected from a group of read sequences by identifying in advance read sequences derived from the same one single strand of a fragment of the sample DNA.

An example of detail procedures to acquire a consensus read sequence considering complementary strands from each of the groups of read sequences as described above includes a procedure in which two read sequences each derived from each of two complementary strands of a fragment of the sample DNA are selected from a group of read sequences and consensus of sequence information is built between the two read sequences. This procedure may be repeated to generate a plurality of consensus read sequences considering complementary strands, among which consensus may be built to generate one consensus read sequence considering complementary strands. Another example of detail procedures to acquire such a consensus read sequence considering complementary strands includes a procedure in which read sequences included in a group of read sequences are classified into groups derived from one and the other of two complementary strands of a fragment of the sample DNA, and consensus is built among read sequences in each group, and consensus is further built between the resulting two consensus data to generate one consensus read sequence considering complementary strands. Still another example includes a procedure in which consensus is built among read sequences included in a group of read sequences to generate a consensus read sequence, without distinguishing read sequences derived from one of two complementary strands of a fragment of the sample DNA from those derived from the other.

(3-6) Extraction of sequence information on sample DNA using read pairs

In an embodiment of the present sequencing method, one pair of read sequences consisting of two read sequences (i.e., "read pair") is generated, instead of one read sequence, for each of the plurality of amplified fragments contained in the library in sequencing of the library described in (3-2) above. From the read pairs generated, sequence information on the sample DNA is extracted on the basis of the above principle.

In the present method, one or more read pairs are generated for each of the amplified fragments through sequencing of the library. The generation of one or more read pairs is performed for each of two or more amplified fragments derived from each of the fragments of the sample DNA. The read pairs contain sequence information on either one of two complementary strands of the corresponding fragment of the sample DNA. Thus, in the present embodiment, the plurality of read sequences acquired in the above-described sequencing of the library includes a plurality of read pairs.

One of two read sequences constituting each of the read pairs is a read sequence containing sequence information on reading out of the sequence of one strand of two complementary strands constituting the amplified fragment, from the 5'-terminal side to the 3'-terminal side (referred to as "read 1"), and the other is a read sequence containing sequence information on reading out of the sequence of the same one strand from the 3'-terminal side to the 5'-terminal side (referred to as "read 2"). Read 1 and read 2 are disposed in the opposite direction with respect to the original strand (a single strand constituting the amplified fragment). Specifically, when being mapped on the original strand, the beginning of read 1 is positioned in the 5'-terminal side to the end in the original strand, and the beginning of read 2 is, on the other hand, positioned in the 3'-terminal side to the end in the original strand (see Schematic Diagram 1 shown later).

Subsequently, read pairs containing sequence information on the same region in the sample DNA are selected out of the plurality of read pairs in the acquired sequencing data. The read pairs collected are put into a group. Examples of ways to generate such groups of read pairs include a method of mapping read 1 and read 2 in each read pair on a reference sequence and collecting read sequence pairs into the same group such that they have the same region between the beginning of read 1 and the beginning of read 2 in the reference sequence. In an example of more detailed procedures, first, read pairs whose positions of the beginning of one read sequence (read 1 or 2) included therein are the same in a reference sequence are collected, and from the collected read sequence pairs, read sequence pairs whose positions of the beginning of the other read sequence (read 2 or 1) therein are the same in the reference sequence are collected into the same group.

Thus, a "group of read sequence pairs (read pairs)" to be generated in the method according to the present invention is a collection of read pairs estimated to contain sequence information on the same region in the sample DNA (i.e., derived from the same fragment of the sample DNA). In the present method, one or more groups of read pairs can be generated, typically in a manner depending on the number of fragments of the sample DNA used in library preparation and the amount of sequencing data.

Subsequently, sequence information on the sample DNA is extracted by using information on read sequences included in the groups of read pairs acquired. For example, one sequence data can be generated by building consensus of sequence information among read sequences included in a group of read pairs. The detail procedure to build consensus among read sequences is as described in (3-4) above. In building consensus among read sequences, the read sequences of all of the read pairs included in a group of read pairs may be used; however, the read sequences of only some of the read pairs included in the group may be used. The resulting sequence data can be acquired as final sequence data representing the sequence of a fragment of the sample DNA.

(3-7) Sequencing with read pairs based on information on complementary strands

A method for sequencing DNA using information on complementary strands can be implemented with the above-described read pairs. In this method, one or more read pairs are generated for each of amplified fragments derived from each of two complementary strands constituting each of the fragments of the sample DNA in the sequencing of the library as described in (3-6) above. That is, two or more read pairs are acquired for one fragment of the sample DNA, and the read pairs contain sequence information on one and the other of two complementary strands of the corresponding fragment of the sample DNA. Hence, the plurality of read sequences acquired through the above-described sequencing includes a plurality of read pairs in the present embodiment.

Subsequently, one or more groups of read pairs are generated from the plurality of read pairs acquired. The way to generate groups of read pairs is as described in (3-5) above. Groups of read pairs to be acquired here each include read pairs containing sequence information on one and the other of two complementary strands of a certain fragment of the sample DNA. Therefore, sequence data reflecting information on complementary strands can be generated by building consensus of sequence information among read sequences included in each of the groups of read pairs. The detail procedure to build consensus among read sequences is as described in (3-4) above. In building consensus among read sequences, the read sequences of all of the read pairs included in a group of read pairs may be used; however, the read sequences of only some of the read pairs included in the group may be used.

Next, one sequence data is derived by using information on read sequences included in each of the groups of read pairs acquired. For example, one sequence data can be generated by building consensus of sequence information among read sequences included in a group of read pairs. The sequence data acquired represents the sequence of a certain fragment of the sample DNA from which the read sequences in the group are derived. If read sequences containing sequence information on two complementary strands of a fragment of the sample DNA are included in a group of read pairs, building of consensus thereamong can exclude errors which occur only in one strand such as read errors in sequencing or errors due to oxidative modification of DNA or the like.

The step of building consensus among read sequences included in a group of read pairs preferably includes collecting at least one read pair derived from each of two complementary strands of a fragment of the sample DNA from a group of read pairs, and building consensus of sequence information among read sequences included in the collected read pairs. Thereby, a consensus read sequence considering complementary strands can be acquired. The resulting consensus read sequence considering complementary strands can be acquired as final sequence data representing the sequence of a fragment of the sample DNA.

Examples of procedures to collect read pairs derived from each of two complementary strands of a fragment of the sample DNA from a group of read pairs include the following: a label sequence, which allows two complementary strands of each of the fragments of the sample DNA to be distinguished, is added to the fragments of the sample DNA in advance to prepare amplified fragments each including the label sequence; the amplified fragments are then subjected to sequencing to acquire read pairs derived from each of the amplified fragments and information on the label sequence accompanying thereto; groups of read pairs are generated from the read pairs acquired; and read pairs derived from each of strands complementary to each other are then collected from each of the groups of read pairs with use of the information on the label sequence accompanying to each read pair.

In the above procedure, the label sequence described in (2-5-2) in the above (e.g., a label sequence for complementary strands or a label sequence for individual fragments), which allows identifying which of two complementary strands of the fragment a read sequence is derived from, is preferably added to each fragment of the sample DNA. Preferably, a label sequence for complementary strands is used. By sequencing amplified fragments obtained from fragments of the sample DNA each including the label sequences added thereto, read pairs derived from the amplified fragments and information on the label sequences accompanying to each read sequence included therein can be acquired. In this case, either one of read 1 or read 2 in each read pair is accompanied by information on the label sequence in the 5'-terminal side, and the other is accompanied by information on the label sequence in the 3'-terminal side.

Next, a preferred procedure in collecting read pairs derived from each of strands complementary to each other from a group of read pairs by using information on the label sequences will be described. The read pairs included in a group of read pairs are mapped on a reference sequence. A read pair such that the beginning of the read sequence accompanied by information on the label sequence in the 5'-terminal side is positioned in the 5'-terminal side to the beginning of the other read sequence in the reference sequence (i.e., the beginning of the read sequence accompanied by information on the label sequence in the 3'-terminal side is positioned in the 3'-terminal side to the beginning of the other read sequence in the reference sequence), and a read pair such that the beginning of the read sequence accompanied by information on the label sequence in the 5'-terminal side is positioned in the 3'-terminal side to the beginning of the other read sequence in the reference sequence (i.e., the beginning of the read sequence accompanied by information on the label sequence in the 3'-terminal side is positioned in the 5'-terminal side to the beginning of the other read sequence in the reference sequence) are distinguished from each other. The former read pair and the latter read pair are derived from two complementary strands of a fragment of the sample DNA (see Conceptual Diagram 2 shown below). Thus, which of two complementary strands constituting a fragment of the sample DNA each read pair in a group of read pairs is derived from can be identified on the basis of information on the label sequences accompanying to two read sequences included in the read pair and positional relation between the two read sequences in a reference sequence. Alternatively, sequencing reaction which is initiated only when a specific label sequence has been added to a terminal of an amplified fragment allows identification of a read pair derived from a certain single strand of each fragment of the sample DNA on the basis of information on the label sequence. Thus, read pairs derived from each of strands complementary to each other can be collected from a group of read pairs by identifying in advance read pairs derived from the same one single strand of a fragment of the sample DNA.

[Chem. 2]

Conceptual Diagram 2

Identification of read pair based on information on label sequences

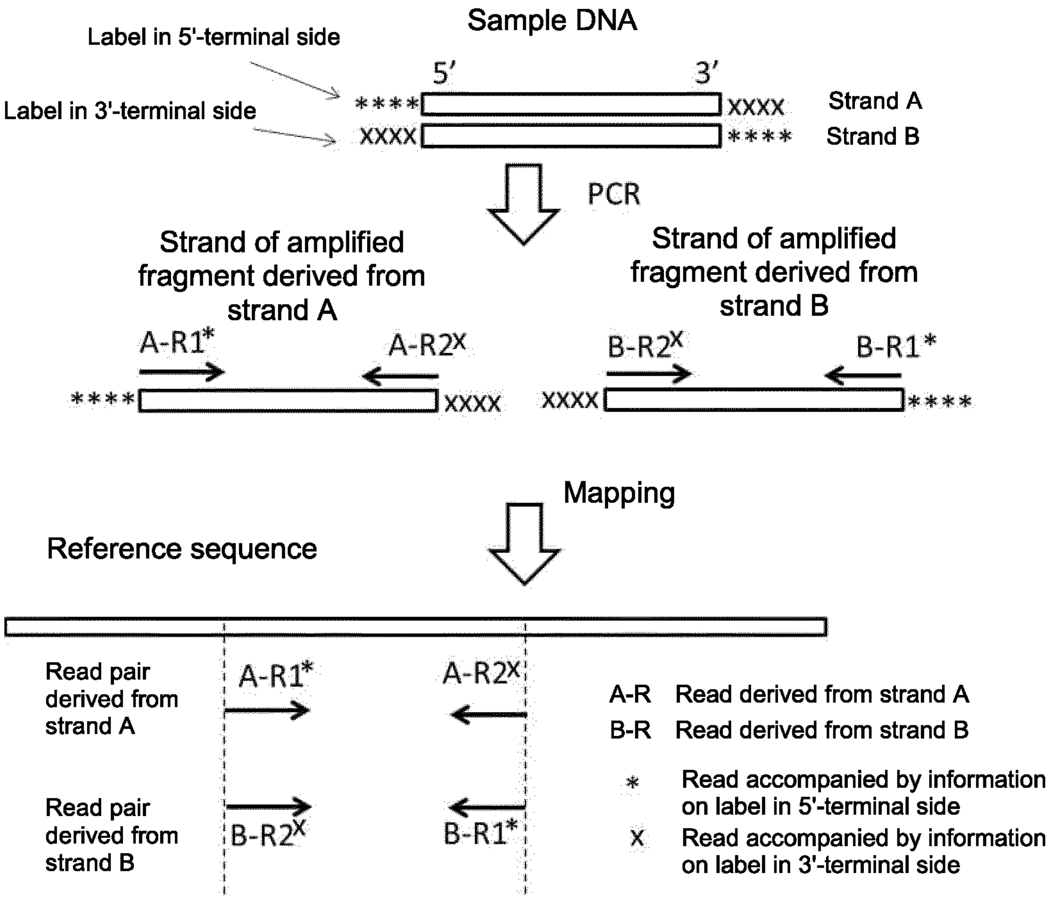


An example of detail procedures to acquire a consensus read sequence considering complementary strands from each of the groups of read pairs as described above includes a procedure in which two read pairs each derived from each of two complementary strands of a fragment of the sample DNA are selected from a group of read pairs and consensus of sequence information is built among read sequences included in the read pairs. This procedure may be repeated to generate a plurality of consensus read sequences considering complementary strands, among which consensus may be built to generate one consensus read sequence considering complementary strands. Another example of detail procedures to acquire such a consensus read sequence considering complementary strands includes a procedure in which read pairs included in a group of read pairs are classified into groups derived from one and the other of two complementary strands of a fragment of the sample DNA, and consensus is built among read sequences included in read pairs of each group and consensus is further built between the resulting two consensus data to generate one consensus read sequence considering complementary strands. Still another example includes a procedure in which consensus is built among read sequences included in a group of read pairs to generate a consensus read sequence, without distinguishing read sequences derived from one of two complementary strands of a fragment of the sample DNA from those derived from the other. An example of more detail procedures to generate a consensus read sequence considering complementary strands from a group of read pairs is described later in Example 1 (Schematic Diagram 3) .

The above-described grouping of read sequences or read pairs can be performed on the basis of sequence information on the sample DNA itself contained in read sequences. Read sequences containing sequence information on strands complementary to each other can be distinguished, for example, on the basis of sequence information on label sequences added to each fragment of the sample DNA.

(3-8) Extraction of information on complementary strands with label sequences for individual fragments

Alternatively, read sequences derived from two complementary strands of a fragment of the sample DNA can

be identified by using the above-described label sequences for individual fragments. In this case, generation of a group of read sequences or read pairs is not necessarily needed, and read sequences derived from two complementary strands of one DNA fragment can be extracted on the basis of information on individual label sequences. Consensus is built among the read sequences extracted, and thus highly accurate read information reflecting information on a complementary strand can be acquired.

(4. Optimum conditions for sequencing)

In the present sequencing method, if labels for individually identifying fragments of the sample DNA (label sequences for individual fragments) are not used, there is the possibility of misrecognizing sequences actually derived from different DNA fragments as those derived from the same DNA fragment, which may lead to the possibility that mutations to be detected in proper cases are regarded as errors and missed.

The efficiency of acquiring sequence data for the sample DNA (e.g., consensus data for each group of read sequences or a consensus read sequence considering complementary strands) from sequencing data (data efficiency) depends on fractions of amplified products (including forward strands and complementary strands) derived from the same DNA fragment in the library and the amount of sequencing data (the number of reads or bp) from the library. For example, the amount of input DNA in the amplification step (e.g., PCR) in library preparation and the amount of sequencing data affect the number of read sequences or read pairs included in each of the above-described groups of read sequences or read pairs, and thus the data efficiency.

Misrecognition of fragments can occur when two fragments derived from different fragments of sample DNA and involving overlapping in their sequences are included in a library and the fragments both have been subjected to sequencing. Hence, misrecognition of fragments is associated with the diversity of DNA sequences in a library (the amount of input DNA in the amplification step). The diversity of DNA sequences in a library is affected by the diversity of the sequence of sample DNA, and the diversity of the sequence of sample DNA generally depends on the size (total bp) of the sample DNA. Therefore, the size of sample DNA also has influence on misrecognition of fragments. In addition, single-strand-specific nuclease treatment may have influence on misrecognition of fragments. Because of the sequence specificity of a single-strand-specific nuclease, sequences which are difficult to be removed by the nuclease may remain in DNA fragments after the nuclease treatment. Specifically, each DNA fragment after being subjected to the nuclease treatment may have increased probability of having identical sequences at two terminals of the fragment, which may increase the fraction of misrecognized fragments.

Accordingly, the amount of input DNA in the amplification step (e.g., PCR) in library preparation, the amount of sequencing data, and the size of the sample DNA are primarily important as factors which may have influence on the efficiency and accuracy of sequencing. Further, it is desired to additionally consider the number of units (U/ng) of a single-strand-specific nuclease per unit weight of DNA in the reaction solution in the single-strand-specific nuclease treatment as a factor which may have influence on the efficiency and accuracy of sequencing. Moreover, the number of read sequences or read pairs included in each group of read sequences or read pairs, which depends on those factors, can serve as an index to determine the efficiency and accuracy of sequencing.

The proper range of the amount of input DNA in the amplification (e.g., PCR) step in library preparation (hereinafter, simply referred to as the amount of input DNA) may depend on the size of the sample DNA, and is preferably 250 amol or less, more preferably 125 amol or less, even more preferably 62.5 amol or less, even more preferably 31.3 amol or less, even more preferably 15.6 amol or less, even more preferably 7.8 amol or less, even more preferably 3.9 amol or less, even more preferably 1.7 amol or less, even more preferably 0.83 amol or less, even more preferably 0.42 amol or less, and even more preferably 0.21 amol or less, for example, per 1 Mbp of the sample DNA. For ensuring the comprehensiveness for genomes, on the other hand, the amount of input DNA in PCR is preferably 0.0003 amol or more, more preferably 0.0007 amol or more, even more preferably 0.002 amol or more, even more preferably 0.005 amol or more, even more preferably 0.01 amol or more, even more preferably 0.03 amol or more, even more preferably 0.05 amol or more, even more preferably 0.1 amol or more, even more preferably 0.3 amol or more, even more preferably 1 amol or more, even more preferably 2 amol or more, even more preferably 3.9 amol or more, and even more preferably 7.8 amol or more, per 1 Mbp of the sample DNA.

In an example, the amount of input DNA in the present sequencing method is as follows: for bacteria, the genome size of which is approximately 5 Mbp, the amount of input DNA is preferably from 0.1 to 250 amol, more preferably from 0.3 to 250 amol, even more preferably from 1 to 250 amol, even more preferably from 2 to 125 amol, even more preferably from 3.9 to 62.5 amol, and even more preferably from 7.8 to 31.3 amol, per 1 Mbp of sample DNA; for yeasts, the genome size of which is approximately 10 Mbp, the amount of input DNA is preferably from 0.05 to 250 amol, more preferably from 0.1 to 250 amol, even more preferably from 0.3 to 125 amol, even more preferably from 1 to 62.5 amol, and even more preferably from 2 to 31.3 amol, per 1 Mbp of sample DNA; for nematodes, the genome size of which is approximately 100 Mbp, the amount of input DNA is preferably from 0.005 to 31.3 amol, more preferably from 0.01 to 31.3 amol, even more preferably from 0.03 to 15.6 amol, even more preferably from 0.1 to 7.8 amol, and

even more preferably from 0.3 to 3.9 amol, per 1 Mbp of sample DNA; and for mice, the genome size of which is approximately 3 Gbp, the amount of input DNA in PCR is preferably from 0.0003 to 1.7 amol, more preferably from 0.0007 to 1.7 amol, even more preferably from 0.002 to 1.7 amol, even more preferably from 0.005 to 0.83 amol, even more preferably from 0.01 to 0.42 amol, and even more preferably from 0.03 to 0.21 amol, per 1 Mbp of sample DNA. Herein, the amount of input DNA is the amount of DNA in a DNA sample to be used for the amplification step, and does not include the amount of DNA from primers or the like.

However, the above-described range of the amount of input DNA per 1 Mbp of the sample DNA may depend on the number of units of a single-strand-specific nuclease in the above-described single-strand-specific nuclease treatment in library preparation. If 0.05 U/ng or less of S1 nuclease is used in the single-strand-specific nuclease treatment, for example, the influence on misrecognition of fragments is sufficiently small, and hence the proper range of the amount of input DNA is as described above.

If S1 nuclease with the number of units being more than 0.05 U/ng is used in the single-strand-specific nuclease treatment, on the other hand, the frequency of misrecognition of fragments may increase as the number of units increases. Hence, it is desired to set the amount of input DNA in accordance with the number of units (U/ng) of S1 nuclease in the reaction solution in the nuclease treatment. Proper conditions for the number of units (>0.05 U/ng) of S1 nuclease and the amount of input DNA are represented by an index calculated by the following equation:

$$\text{Index} = \text{amount of input DNA (amol/Mbp)} \times 3^{\log \text{S1 nuclease (U/ng)}}$$

wherein S1 nuclease (U/ng) > 0.05, and log denotes common logarithm, and

the index is preferably 60 or less, more preferably 30 or less, even more preferably 15 or less, and even more preferably 7.5 or less.

If 0.05 U/ng or less of MBN is used in the single-strand-specific nuclease treatment, the influence on misrecognition of fragments is sufficiently small, and hence the proper range of the amount of input DNA is as described above.

If MBN with the number of units being more than 0.05 U/ng is used in the single-strand-specific nuclease treatment, on the other hand, the frequency of misrecognition of fragments may increase as the number of units increases. Hence, it is desired to set the amount of input DNA in accordance with the number of units (U/ng) of MBN in the reaction solution in the nuclease treatment. Proper conditions for the number of units (>0.05 U/ng) of MBN and the amount of input DNA are represented by an index calculated by the following equation:

$$\text{Index} = \text{amount of input DNA (amol/Mbp)} \times 3^{\log \text{MBN (U/ng)}}$$

wherein MBN (U/ng) > 0.05, and log denotes common logarithm,

and

the index is preferably 60 or less, more preferably 30 or less, even more preferably 15 or less, and even more preferably 7.5 or less.

If RecJf is used in the single-strand-specific nuclease treatment, on the other hand, the influence on misrecognition of fragments is sufficiently small irrespective of the number of units, and hence the proper range of the amount of input DNA is as described above.

To ensure a sufficient amount of data (sequence information) for analysis, the amount of input DNA in the present sequencing method is preferably 0.1 amol or more, more preferably 1 amol or more, even more preferably 5 amol or more, even more preferably 20 amol or more, even more preferably 39 amol or more, and even more preferably 78 amol or more. For data efficiency, on the other hand, the amount of input DNA is preferably 100,000 amol or less, more preferably 20,000 amol or less, and even more preferably 5,000 amol or less. For example, the amount of input DNA in the present sequencing method is preferably from 0.1 to 100,000 amol, more preferably from 1 to 100,000 amol, even more preferably from 5 to 100,000 amol, even more preferably from 20 to 100,000 amol, even more preferably from 20 to 20,000 amol, even more preferably from 39 to 20,000 amol, even more preferably from 78 to 20,000 amol, even more preferably from 20 to 5,000 amol, even more preferably from 39 to 5,000 amol, and even more preferably from 78 to 5,000 amol.

An amount of sequencing data which is excessively large or excessively small with respect to the amount of input DNA may result in lowered data efficiency. The amount of sequencing data in the present sequencing method as the number of read pairs or the number of read sequences per 1 amol of the amount of input DNA is preferably 0.02 ×

$10^6$ (4 Mbp, as the amount of base pairs in read sequences or read pairs wherein the mean of the lengths of read sequences or of the total lengths of read sequences included in read pairs is 200 bp, and the value can vary depending on the mean of the lengths of read sequences or of the total lengths of read sequences included in read pairs, the same applies hereinafter) or larger, more preferably 0.04 × $10^6$ (8 Mbp) or larger, even more preferably 0.08 × $10^6$ (16 Mbp) or larger, even more preferably 0.16 × $10^6$ (32 Mbp) or larger, and, preferably 10 × $10^6$ (2,000 Mbp) or smaller, more preferably 5 × $10^6$ (1,000 Mbp) or smaller, even more preferably 2.5 × $10^6$ (500 Mbp) or smaller, even more preferably 2 × $10^6$ (400 Mbp) or smaller. For example, the amount of sequencing data in the present sequencing method as the number of read pairs or the number of read sequences per 1 amol of the amount of input DNA is preferably from 0.02 to 10 × $10^6$ (from 4 to 2,000 Mbp), more preferably from 0.04 to 5 × $10^6$ (from 8 to 1,000 Mbp), even more preferably from 0.08 to 2.5 × $10^6$ (from 16 to 500 Mbp), and even more preferably from 0.16 to 2 × $10^6$ (from 32 to 400 Mbp).

In the present sequencing method, the mean of the numbers of read sequences or read pairs included in groups of read sequences or read pairs which provide the maximum data efficiency is almost constant irrespective of the amount of input DNA and the amount of sequencing data (see Patent Literature 4). In the present sequencing method, the number of read sequences included in each group of read sequences, or the number of read pairs included in each group of read pairs is, as the mean among the groups, preferably 1.05 or larger, more preferably 1.1 or larger, even more preferably 1.2 or larger, even more preferably 1.4 or larger, and, preferably 30 or smaller, more preferably 20 or smaller, even more preferably 10 or smaller, even more preferably 5 or smaller. In the present sequencing method, for example, the number of read sequences or read pairs included in each group of read sequences or read pairs is, as the mean among the groups, preferably from 1.05 to 30, more preferably from 1.1 to 20, even more preferably from 1.2 to 10, and even more preferably from 1.4 to 5.

Appropriate amount of sequencing data can depend on the size of sample DNA. A larger amount of input DNA is required for sample DNA of larger size. On the other hand, the data efficiency is lowered if the amount of sequencing data is excessively large for the size of sample DNA. The amount of sequencing data as the number of read sequences or read pairs per 1 Mbp of sample DNA in the present sequencing method is preferably 0.05 × $10^6$ (10 Mbp) or larger, more preferably 0.1 × $10^6$ (20 Mbp) or larger, even more preferably 0.2 × $10^6$ (40 Mbp) or larger, even more preferably 0.5 × $10^6$ (100 Mbp) or larger, even more preferably 1 × $10^6$ (200 Mbp) or larger, even more preferably 2 × $10^6$ (0.4 Gbp) or larger, and, preferably 1,600 × $10^6$ (320 Gbp) or smaller, more preferably 800 × $10^6$ (160 Gbp) or smaller, even more preferably 400 × $10^6$ (80 Gbp) or smaller, even more preferably 200 × $10^6$ (40 Gbp) or smaller, even more preferably 100 × $10^6$ (20 Gbp) or smaller, even more preferably 50 × $10^6$ (10 Gbp) or smaller. For example, the amount of sequencing data as the number of read sequences or read pairs per 1 Mbp of sample DNA in the present sequencing method is preferably from 0.05 to 1,600 × $10^6$ (from 0.01 to 320 Gbp), more preferably from 0.1 to 800 × $10^6$ (from 0.02 to 160 Gbp), even more preferably from 0.2 to 400 × $10^6$ (from 0.04 to 80 Gbp), even more preferably from 0.5 to 200 × $10^6$ (from 0.1 to 40 Gbp), even more preferably from 1 to 100 × $10^6$ (from 0.2 to 20 Gbp), and even more preferably from 2 to 50 × $10^6$ (from 0.4 to 10 Gbp). If the size of sample DNA is large, for example, as with the case of genomic DNA derived from a mammal and the comprehensiveness of sequence data for the whole sequence of sample DNA does not matter, the amount of sequencing data in the present sequencing method may be smaller than 0.05 × $10^6$ (10 Mbp), as the number of read sequences or read pairs per 1 Mbp of sample DNA. For mice, the genome size of which is approximately 3 Gbp, for example, the amount of sequencing data as the number of read sequences or read pairs per 1 Mbp of sample DNA is preferably from 0.00003 to 16 × $10^6$ (from 0.006 to 3,200 Mbp), more preferably from 0.00007 to 8 × $10^6$ (from 0.014 to 1,600 Mbp), even more preferably from 0.0001 to 4 × $10^6$ (from 0.02 to 800 Mbp), even more preferably from 0.0003 to 2 × $10^6$ (from 0.06 to 400 Mbp), even more preferably from 0.0005 to 1 × $10^6$ (from 0.1 to 200 Mbp), and even more preferably from 0.001 to 0.5 × $10^6$ (from 0.2 to 100 Mbp).

If the size of sample DNA is excessively small, the diversity of sequences in a library for sequencing is lower and the probability of misrecognition of fragments may increase. The size of the sample DNA in the present sequencing method is preferably 10 kbp or more, more preferably 100 kbp or more, even more preferably 1 Mbp or more, and even more preferably 4 Mbp or more, though the size of the sample DNA may depend, for example, on the size of genomic DNA of an organism from which the sample DNA is derived.

In a preferred embodiment of the present sequencing method, the size of sample DNA is approximately 5 Mbp, the amount of input DNA in PCR is preferably from 10 to 1,250 amol, and the amount of sequencing data as the number of read sequences or read pairs is from 0.2 to 12,500 × $10^6$ (from 0.04 to 2,500 Gbp), preferably from 0.4 to 6,250 × $10^6$ (from 0.08 to 1,250 Gbp), more preferably from 0.8 to 3,125 × $10^6$ (from 0.16 to 625 Gbp), and even more preferably from 1.6 to 2,500 × $10^6$ (from 0.32 to 500 Gbp).

More preferably, the size of sample DNA is approximately 5 Mbp, the amount of input DNA in PCR is from 20 to 625 amol, and the amount of sequencing data as the number of read sequences or read pairs is from 0.4 to 6,250 × $10^6$ (from 0.08 to 1,250 Gbp), preferably from 0.8 to 3,125 × $10^6$ (from 0.16 to 625 Gbp), more preferably from 1.6 to 1,563 × $10^6$ (from 0.32 to 313 Gbp), and even more preferably from 3.2 to 1,250 × $10^6$ (from 0.64 to 250 Gbp) .

Even more preferably, the size of sample DNA is approximately 5 Mbp, the amount of input DNA in PCR is from 39 to 313 amol, and the amount of sequencing data as the number of read sequences or read pairs is from 0.78

to 3,130 × $10^6$ (from 0.156 to 626 Gbp), preferably from 1.56 to 1,565 × $10^6$ (from 0.312 to 313 Gbp), more preferably from 3.12 to 783 × $10^6$ (from 0.624 to 157 Gbp), and even more preferably from 6.24 to 626 × $10^6$ (from 1.248 to 125 Gbp).

In another preferred embodiment of the present sequencing method, the size of sample DNA is approximately 5 Mbp, and the number of read sequences or read pairs per group of read sequences or read pairs is, as the mean among the groups, from 1.05 to 30, preferably from 1.1 to 20, even more preferably from 1.2 to 10, and even more preferably from 1.4 to 5.

As described above, the amount of input DNA in PCR may depend on the number of units of a single-strand-specific nuclease in the single-strand-specific nuclease treatment in library preparation.

In still another preferred embodiment of the present sequencing method, the size of sample DNA is approximately 3 Gbp, the amount of input DNA in PCR is preferably from 10 to 5,000 amol, and the amount of sequencing data as the number of read sequences or read pairs is from 0.2 to 50,000 × $10^6$ (from 0.04 to 10,000 Gbp), preferably from 0.4 to 25,000 × $10^6$ (from 0.08 to 5,000 Gbp), more preferably from 0.8 to 12,500 × $10^6$ (from 0.16 to 2,500 Gbp), and even more preferably from 1.6 to 10,000 × $10^6$ (from 0.32 to 2,000 Gbp).

More preferably, the size of sample DNA is approximately 3 Gbp, the amount of input DNA in PCR is from 20 to 2,500 amol, and the amount of sequencing data as the number of read sequences or read pairs is from 0.4 to 25,000 × $10^6$ (from 0.08 to 5,000 Gbp), preferably from 0.8 to 12,500 × $10^6$ (from 0.16 to 2,500 Gbp), more preferably from 1.6 to 6,250 × $10^6$ (from 0.32 to 1,250 Gbp), and even more preferably from 3.2 to 5,000 × $10^6$ (from 0.64 to 1,000 Gbp).

Even more preferably, the size of sample DNA is approximately 3 Gbp, the amount of input DNA in PCR is from 39 to 1,250 amol, and the amount of sequencing data as the number of read sequences or read pairs is from 0.78 to 12,500 × $10^6$ (from 0.156 to 2,500 Gbp), preferably from 1.56 to 6,250 × $10^6$ (from 0.312 to 1,250 Gbp), more preferably from 3.12 to 3,125 × $10^6$ (from 0.624 to 625 Gbp), and even more preferably from 6.24 to 2,500 × $10^6$ (from 1.248 to 500 Gbp).

In still another preferred embodiment of the present sequencing method, the size of sample DNA is approximately 3 Gbp, and the number of read sequences or read pairs per group of read sequences or read pairs is, as the mean among the groups, from 1.05 to 30, preferably from 1.1 to 20, even more preferably from 1.2 to 10, and even more preferably from 1.4 to 5.

As described above, the amount of input DNA in PCR may depend on the number of units of a single-strand-specific nuclease in the single-strand-specific nuclease treatment in library preparation.

Examples of sample DNA having a size of approximately 5 Mbp include the genome of Salmonella (approximately 4.86 Mbp). Preferred examples of Salmonella include LT-2 strain, TA100 strain, TA98 strain, TA1535 strain, TA1538 strain, and TA1537 strain of S. typhimurium, which are applicable for the Ames test.

(5. Applications of sequencing method)

Sequence data acquired in sequencing using the library according to the present invention are highly accurate sequence data excluding sequencing errors due to oxidative modification of single-stranded moieties of DNA fragments or the like. Hence, sequencing using the library according to the present invention may be applied to mutation analysis, though the application is not limited thereto. More specifically, for example, the method for sequencing DNA according to the present invention may be applied to genotoxicity evaluation for a test substance, evaluation of other toxicity such as reproductive and developmental toxicity, evaluation of impacts of change over time, living environments, genetic factors, and so forth on genomic DNA, and quality evaluation for cultured cells, through mutation analysis for genomic DNA. In these applications, the library according to the present invention is prepared from genomic DNA, which is a subject of mutation analysis, and the library is subjected to sequencing to acquire sequence data. Subsequently, mutation analysis is performed by using the sequence data acquired to detect a mutation in the genomic DNA of the subject of analysis.

Thus, the present invention also provides a method for detecting a mutation in genomic DNA. This method comprises: preparing a sequencing library by the method for preparing a sequencing library according to the present invention with use of genomic DNA in a cell as sample DNA; and sequencing the sequencing library. Through the sequencing, sequence data on the genomic DNA are generated. A mutation in the genomic DNA can be detected by comparing the sequence data with a reference sequence to detect a site of mismatch base between the sequence data and the reference sequence as a site of mutation.

In an embodiment, the method for detecting a mutation in genomic DNA according to the present invention is used for genotoxicity evaluation for a test substance. In the present embodiment, the genomic DNA is genomic DNA from cells exposed to a test substance. Preferably, the genomic DNA comprises genomic DNA from cells exposed to a test substance (subject cells) and genomic DNA from cells not exposed to the test substance (control cells). Preferably, the genomic DNA is fresh DNA. The fresh DNA is preferably DNA having a DIN of 6 or more, more preferably DNA having a DIN of 7 or more, more preferably DNA having a DIN of 7.3 or more, and even more preferably DNA having a DIN of 7.5 or more. In the present embodiment, a mutation detected in the genomic DNA from the subject cells is

compared with a mutation detected in the genomic DNA from the control cells. For example, a mutation detected only in the subject cells can be identified as a mutation generated by exposure to the test substance. The cells to be used in the present embodiment are not particularly limited, and examples thereof include microbial cells, animal cells, and plant cells. Preferred examples of animals include, but are not limited to, mammals, birds, silkworms, and nematodes, and examples of microbes include, but are not limited to, Escherichia such as Escherichia coli, Salmonella, and yeast. Preferred examples of cells to be used in the present embodiment include, but are not limited to, cells of Salmonella and cells of Escherichia coli. Preferred examples of cells of Salmonella include LT-2 strain, TA100 strain, TA98 strain, TA1535 strain, TA1538 strain, and TA1537 strain of Salmonella typhimurium, which are used in the Ames test. Preferred examples of Escherichia coli include K-12 strain, which is widely used for molecular biology study, and WP2 strain and WP2 uvrA strain, which are used in the Ames test. Other preferred examples of cells to be used in the present embodiment include cells of mammals collected from the living body and cultured cells derived from mammals. Preferred examples of mammals include mice, rats, hamsters, Chinese hamsters, rabbits, and humans, among which mice and humans are preferred. Other preferred examples of cells to be used in the present embodiment include cells of birds collected from the living body and cultured cells derived from birds. Preferred examples of birds include chickens, and examples of cultured cells derived from birds include DT40.

The test substance may be, for example, any substance the genotoxicity of which is desired to be evaluated. Examples thereof include substances suspected to have genotoxicity, substances for which the presence or absence of genotoxicity is desired to be determined, and substances for which the type of mutation to be induced is desired to be examined. The test substance may be a naturally occurring substance, or a substance artificially synthesized, for example, through a chemical or biological method, or a compound, or a composition or mixture. Alternatively, the test substance may be an ultraviolet ray or radiation. Any means to expose cells to the test substance can be appropriately selected in accordance with the type of the test substance, and the means is not particularly limited. Examples thereof include a method of adding the test substance to a medium containing cells, and a method of subjecting cells under an atmosphere in which the test substance is present.

In another embodiment, the method for detecting a mutation in genomic DNA according to the present invention is used for evaluation of impacts of change over time, living environments, genetic factors, and so forth on genomic DNA. Examples of change over time include growth, aging, senility, and subculture of cells or individuals, examples of living environments include lifestyle such as dietary habit and exercise, and residence, and examples of genetic factors include sex, species, and deletion or base-pair substitution of a specific gene, however any of the above examples are not limited thereto. A preferred example of the present embodiment is evaluation of impacts of change over time on genomic DNA, and genomic DNA from cells experienced change over time is used therefor. More preferably, the genomic DNA comprises genomic DNA from cells experienced change over time (subject cells) and genomic DNA from cells experienced change over time to a lesser extent (control cells). Preferably, the genomic DNA is fresh DNA. The fresh DNA is preferably DNA having a DIN of 6 or more, more preferably DNA having a DIN of 7 or more, more preferably DNA having a DIN of 7.3 or more, and even more preferably DNA having a DIN of 7.5 or more. In the present embodiment, a mutation detected in the genomic DNA from the subject cells is compared with a mutation detected in the genomic DNA from the control cells. Examples of cells experienced change over time to a lesser extent to be used as the control cells include cells in which the degree of growth, aging, senility, or subculture is smaller than that of the subject cells (e.g., younger cells, cells without aging treatment, cells not subcultured or of smaller passage number). For example, a mutation detected only in the subject cells can be identified as a mutation generated by change over time. Preferred examples of cells to be used in the present embodiment include cells of mammals collected from the living body and cultured cells derived from mammals. Preferred examples of mammals are as described above.

In another embodiment, the method for detecting a mutation in genomic DNA according to the present invention is used for quality evaluation for cultured cells. The genomic DNA to be used in the present embodiment can be genomic DNA from cultured cells for which the presence or absence of mutation is desired to be examined. Examples of the cultured cells for which the presence or absence of mutation is desired to be examined include cells cultured for a certain period of time for which the tendency of mutation is desired to be checked. Preferably, the genomic DNA comprises genomic DNA from the cells for which the tendency of mutation is desired to be examined (subject cells) and genomic DNA from control cells. For the control cells, cultured cells of the same type whose genetic information is known (e.g., for which the presence or absence of mutation and the mutation type have been confirmed) are used. Preferably, the genomic DNA is fresh DNA. The fresh DNA is preferably DNA having a DIN of 6 or more, more preferably DNA having a DIN of 7 or more, more preferably DNA having a DIN of 7.3 or more, and even more preferably DNA having a DIN of 7.5 or more. In the present embodiment, a mutation detected in the genomic DNA from the subject cells is compared with a mutation detected in the genomic DNA from the control cells. For example, a mutation detected only for the subject cells can be identified as a mutation generated during culturing.

Examples of mutation detected in the method for detecting a mutation in genomic DNA according to the present invention include base-pair substitution mutation and short insertion or deletion mutation. Base-pair substitution mutation is mutation which changes a base pair to another base pair in base-pair information of DNA, and examples thereof

include single base-pair substitution mutation and multiple base-pair substitution mutation, which includes substitution of two base pairs or three or more base pairs. In the present invention, single base-pair substitution mutation is preferably detected. Short insertion or deletion mutation is mutation which causes insertion or deletion of a short base sequence into or from a DNA sequence, where the length of bases inserted or deleted is preferably 10 bp or smaller, and more preferably from 1 to 5 bp.

Detection of base-pair substitution mutation and short insertion or deletion mutation can be performed in accordance with a procedure described in WO/2018/150513 (totally incorporated herein by reference). As an example, a preferred procedure in detecting patterns of single base-pair substitution mutation in subject genomic DNA for analysis will be described in the following. In detecting base-pair substitution mutation, sequence data acquired in the sequencing are compared with a reference sequence, and a site of mismatch base between the sequence data and the reference sequence is detected as a site of mutation. The site detected is acquired as a site of mutation with base-pair substitution mutation. In the present invention, a part or all of the sequence data acquired may be used for comparison with the reference sequence in accordance with the purpose of mutation analysis.

Subsequently, mutations are classified by mutation patterns of bases on the basis of the type of a base at a detected site of mutation and the type of a base before mutation. Further, frequency of appearance can be determined for each of the mutation patterns of bases. The procedure can be performed, for example, by using programs written in a programming language such as Python.

In a more specific example, bases contained in sequence data are classified into the following (i) to (iv) .

(i) a base located at a position where the base on the reference sequence is A,
(ii) a base located at a position where the base on the reference sequence is T,
(iii) a base located at a position where the base on the reference sequence is G, and
(iv) a base located at a position where the base on the reference sequence is C.

The bases (i) and (ii) are bases present at sites where the base pair on the reference sequence is AT. The bases (iii) and (iv) are bases present at sites where the base pair on the reference sequence is GC. Among these bases, a mismatch base with respect to the reference sequence (i.e., base with base-pair substitution mutation) is detected. Subsequently, base pairs before and after mutation are determined for each of the detected sites of mutation on the basis of information on the reference sequence and sequence data. From these data, mutations can be classified into six mutation patterns of base pairs in total, namely, three patterns, [AT → TA, AT → CG, and AT → GC], for the case that the base pair before mutation is AT and three patterns, [GC → TA, GC → CG, and GC → AT], for the case that the base pair before mutation is GC. Further, frequency of appearance can be determined for each mutation pattern on the basis of the total number of mutations belonging to each mutation pattern and the total number of bases analyzed. For example, on the basis of the total number of bases analyzed for each of the base pairs AT and GC, frequency of appearance can be calculated for three mutation patterns for each of the base pairs.

Further, each of the above mutation patterns can be classified into two patterns by the base on the reference sequence on which read sequences have been mapped in mutation detection. For example, mutations whose mutation pattern is GC → TA can be classified into cases in which T is detected on G in the reference sequence and cases in which A is detected on C in the reference sequence. These are defined as mutations from G to T (G → T) and mutations from C to A (C → A), respectively. Accordingly, mutation frequencies can be calculated separately for G → T and C → A. The same is applied to AT → TA, AT → CG, AT → GC, GC → CG, and GC → AT. In the case of true mutation fixed in double-stranded DNA, the mutation frequencies for such two mutation patterns are equal to each other. If biased mutation frequencies are found in such two patterns, this indicates the difference in mutation frequency between the two strands of the sample DNA from which read sequences are derived, and this mutation is probably an error due to mutation of a base caused by oxidative modification or the like. Thus, the classification into two patterns as shown above can be used for detection of sequencing errors.

Further, the present invention allows analysis of multi-base-pair substitution mutation. Examples of multi-base-pair substitution mutation include two-base-pair substitution mutation and three-base-pair substitution mutation. In analysis of multi-base-pair substitution mutation, for example, mutation patterns are classified on the basis of a base sequence before mutation (e.g., $4 \times 4 = 16$ patterns for two-base-pair substitution mutation), and then frequency of appearance can be determined for each mutation pattern on the basis of the total number of mutations belonging to each mutation pattern and the total number of mutations analyzed.

Further, the present invention allows sequence context analysis of single-base-pair substitution mutation. In this analysis, single-base-pair substitution mutations are detected in the above procedure, and a sequence including a base before mutation and the adjacent bases in the upstream and downstream of the base before mutation (what is called context) is then determined for each of the detected mutations on the basis of a reference sequence. Subsequently, each mutation is typed by mutation patterns of base pairs and the context. Specifically, the mutations detected are classified into six mutation patterns of base pairs, [AT → TA, AT → CG, AT → GC, GC → TA, GC → CG, and GC →

AT], by using the above-described procedure. Meanwhile, each mutation detected is classified by contexts. For example, contexts having a length of three bases including adjacent one base in each side of a site of mutation are classified into $4 \times 4 = 16$ groups, [e.g., for mutations from C, ACA, ACC, ACG, ACT, CCA, CCC, CCG, CCT, GCA, GCC, GCG, GCT, TCA, TCC, TCG, and TCT]. As a result, each mutation is classified into 96 ($4 \times 6 \times 4$) types in total by mutation patterns of base pairs and contexts. Analysis with longer contexts is also applicable. For example, each mutation is classified into 256 ($4 \times 4 \times 4 \times 4$) groups by contexts having a length of five bases including adjacent two bases in each side of a site of mutation, and through the classification and the six base pair patterns, each mutation is ultimately classified into 1536 ($4 \times 4 \times 6 \times 4 \times 4$) types in total. Further, each mutation is classified into $4^{2n}$ groups by contexts having a length of (2n + 1) bases including n adjacent bases in each side of a site of mutation, and through the classification and the six base pair patterns, each mutation is ultimately classified into ($4^{2n} \times 6$) types in total. Subsequently, frequency of mutation can be determined for each of the mutation types on the basis of the total number of mutations belonging to each mutation type and the total number of bases analyzed.

Next, a preferred procedure in detecting short insertion or deletion mutation in genomic DNA as a target of analysis will be described. In detecting short insertion or deletion mutation, each sequence data is compared with a reference sequence to detect a site where a base is inserted into or deleted from the reference sequence in the sequence data. In comparing with the reference sequence, sequence data acquired may be partly used or totally used. The site of insertion or deletion to be detected is a site where the length of inserted or deleted bases is preferably 10 bp or smaller, and more preferably from 1 to 5 bp, though the length is not limited thereto. Sites detected are acquired as sites of mutation with insertion or deletion mutation.

For each of the mutations acquired, the type of mutation (insertion mutation or deletion mutation), the base length of the site of insertion or deletion, or the type of a base(s) inserted or deleted can be determined. The procedure to detect a site of insertion or deletion with a specific base length can be performed by using the above-described programs written in a programming language such as Python. Further, the type of a base(s) inserted or deleted can be identified by comparing sequence data with a reference sequence. Through these operations, the base length of each site of insertion or deletion in sequence data or the type of a base(s) at each site of insertion or deletion can be determined. In addition, frequency of insertion or deletion may be determined for each base length and/or each type of base. For example, insertion or deletion mutations acquired for read sequences can be classified by base length to determine frequency for each base length. For example, bases inserted or deleted can be classified by type (A, T, G, and C) to determine frequency for each type. Further, mutations can be more finely classified by combination of the classifications by base length and type of base to determine frequency for each of the combined classifications.

The following substances, production methods, applications, methods, and so forth are disclosed herein as exemplary embodiments of the present invention. However, the present invention is not limited to these embodiments.

[1] A method for preparing a sequencing library, the method comprising:

fragmenting sample DNA; and
treating prepared fragments of the sample DNA with a single-strand-specific nuclease to remove single-stranded moieties from the fragments.

[2] The method according to [1], wherein

the sample DNA is
preferably not DNA of a formalin-fixed cell or cfDNA, and more preferably DNA extracted from a living cell, DNA extracted from a frozen cell, or a stored sample of any of the DNAs, and
the DIN of the sample DNA is preferably 6 or more, more preferably 7 or more, even more preferably 7.3 or more, and even more preferably 7.5 or more.

[3] The method according to [1] or [2], wherein

the single-strand-specific nuclease is
preferably a single-strand-specific endonuclease, a single-strand-specific exonuclease, or a combination thereof, and
more preferably at least one selected from the group consisting of S1 nuclease, Mung Bean Nuclease (MBN), $RecJ_f$, and Exonuclease VII.

[4] The method according to [3], wherein, preferably, the treating with the single-strand-specific nuclease includes treating the fragments of the sample DNA with a single-strand-specific endonuclease and then further treating the

fragments of the sample DNA with a single-strand-specific exonuclease, or includes treating the fragments of the sample DNA with a single-strand-specific exonuclease and then further treating the fragments of the sample DNA with a single-strand-specific endonuclease.

[5] The method according to [3] or [4], wherein, preferably,

the single-strand-specific endonuclease is S1 nuclease,
the number of units (U/ng) of the S1 nuclease per 1 ng of the fragments of the sample DNA is
preferably 0.01 U/ng or more, more preferably 0.02 U/ng or more, even more preferably 0.05 U/ng or more, and preferably 16.7 U/ng or less, more preferably 5.00 U/ng or less, even more preferably 1.67 U/ng or less, or preferably from 0.02 to 5.00 U/ng, more preferably from 0.05 to 1.67 U/ng.

[6] The method according to [3] or [4], wherein, preferably,

the single-strand-specific endonuclease is MBN,
the number of units (U/ng) of the MBN per 1 ng of the fragments of the sample DNA is
preferably 0.01 U/ng or more, more preferably 0.02 U/ng or more, even more preferably 0.03 U/ng or more, even more preferably 0.05 U/ng or more, even more preferably 0.10 U/ng or more, and preferably 16.7 U/ng or less, more preferably 5.00 U/ng or less, even more preferably 1.67 U/ng or less, even more preferably 1.00 U/ng or less, even more preferably 0.30 U/ng or less, or
preferably from 0.02 to 5.00 U/ng, more preferably from 0.03 to 1.67 U/ng, even more preferably from 0.03 to 1.00 U/ng, even more preferably from 0.05 to 1.00 U/ng, even more preferably from 0.10 to 0.30 U/ng.

[7] The method according to [3] or [4], wherein, preferably,

the single-strand-specific exonuclease is RecJf,
the number of units (U/ng) of the $RecJ_f$ per 1 ng of the fragments of the sample DNA is
preferably 0.10 U/ng or more, more preferably 0.30 U/ng or more, and preferably 100 U/ng or less, more preferably 16.7 U/ng or less, even more preferably 1.00 U/ng or less, or
preferably from 0.10 to 16.7 U/ng, more preferably from 0.30 to 1.00 U/ng.

[8] The method according to any one of [1] to [7], preferably, further comprising subjecting the fragments of the sample DNA treated with the single-strand-specific nuclease to one or more treatments selected from the group consisting of end repair, addition of a base to a terminal, and amplification, and
more preferably, further comprising subjecting the fragments of the sample DNA treated with the single-strand-specific nuclease to end repair, addition of a base to a terminal, and amplification.

[9] The method according to [8], wherein, preferably, the addition of a base to a terminal is addition of a label sequence to each terminal of each of the fragments of the sample DNA.

[10] The method according to [8] or [9], wherein, preferably, the amplification is PCR.

[11] The method according to [10], wherein

the single-strand-specific nuclease is S1 nuclease, and if the number of units (U/ng) of the nuclease per 1 ng of the fragments of the sample DNA is 0.05 U/ng or less, the amount of input DNA in the PCR per 1 Mbp of the sample DNA is preferably 250 amol or less, more preferably 125 amol or less, even more preferably 62.5 amol or less, even more preferably 31.3 amol or less, and even more preferably 15.7 amol; or
the single-strand-specific nuclease is S1 nuclease, and if the number of units (U/ng) of the nuclease per 1 ng of the fragments of the sample DNA is more than 0.05 U/ng, an index represented by the following equation:

$$\text{Index} = \text{amount of input DNA in PCR (amol/Mbp sample DNA)} \times 3^{\log \text{S1 nuclease (U/ng)}}$$

wherein S1 nuclease (U/ng) > 0.05, and log denotes common logarithm,
is preferably 60 or less, more preferably 30 or less, even more preferably 15 or less, and even more preferably 7.5 or less; or
the single-strand-specific nuclease is MBN, and if the number of units (U/ng) of the nuclease per 1 ng of the fragments of the sample DNA is 0.05 U/ng or less, the amount of input DNA in the PCR per 1 Mbp of the sample DNA is preferably 250 amol or less, more preferably 125 amol or less, even more preferably 62.5 amol or less,

even more preferably 31.3 amol or less, and even more preferably 15.7 amol; or
the single-strand-specific nuclease is MBN, and if the number of units (U/ng) of the nuclease per 1 ng of the fragments of the sample DNA is more than 0.05 U/ng, an index represented by the following equation:

$$\text{Index} = \text{amount of input DNA in PCR (amol/Mbp sample DNA)} \times 3^{\log \text{MBN (U/ng)}}$$

wherein MBN (U/ng) > 0.05, and log denotes common logarithm,
is preferably 60 or less, more preferably 30 or less, even more preferably 15 or less, and even more preferably 7.5 or less.

[12] A method for sequencing DNA, the method comprising sequencing a sequencing library prepared by the method according to any one of [1] to [11].
[13] The method according to [12], wherein, preferably, the method for sequencing comprises:

(1) sequencing the library to generate one or more read sequences for each of a plurality of amplified fragments contained in the library, and acquiring a plurality of read sequences for the plurality of amplified fragments;
(2) collecting, from the plurality of read sequences acquired, read sequences containing sequence information on the same region in the sample DNA used in preparation of the library into a group to generate one or more groups of read sequences; and
(3) building consensus of sequence information among read sequences included in each of the groups of read sequences.

[14] The method according to [13], wherein, preferably, the step (1) includes generating one or more read sequences for each of amplified fragments derived from one and the other of two complementary strands constituting one fragment of the sample DNA.
[15] The method according to [14], wherein, preferably, the step (2) includes classifying read sequences to be mapped on the same position in a reference sequence into the same group.
[16] The method according to [15], wherein, preferably, the step (3) includes collecting, from each of the groups of read sequences, at least one read sequence derived from each of the two complementary strands constituting one fragment of the sample DNA, and building consensus of sequence information among the collected read sequences.
[17] The method according to [13], wherein, preferably,

the plurality of read sequences in the step (1) includes a plurality of read sequence pairs each consisting of read 1 and read 2, wherein
read 1 is a read sequence containing sequence information on reading out of the sequence of one strand of two complementary strands constituting one of the amplified fragments, from the 5'-terminal side to the 3'-terminal side, and
read 2 is a read sequence containing sequence information on reading out of the sequence of the one strand from the 3'-terminal side to the 5'-terminal side;
the step (2) includes collecting, from the read sequence pairs acquired, read sequence pairs containing sequence information on the same region in the sample DNA into a group to generate one or more groups of read sequence pairs; and
the step (3) includes building consensus of sequence information among read sequences included in each of the groups of read sequence pairs.

[18] The method according to <17>, wherein, preferably, the step (1) includes generating the one or more read sequence pairs for each of amplified fragments derived from one and the other of two complementary strands constituting one fragment of the sample DNA.
[19] The method according to [18], wherein, preferably, the step (2) includes mapping read 1 and read 2 of each of the read sequence pairs on a reference sequence, and classifying read sequence pairs into the same group such that a region between the beginning of read 1 and the beginning of read 2 in the reference sequence is the same thereamong.
[20] The method according to [18], wherein, preferably, the step (2) includes collecting read sequence pairs such that the beginning of one read sequence included in each of the read sequence pairs is at the same position in the reference sequence, then collecting, from the collected read sequence pairs, read sequence pairs such that the

beginning of the other read sequence included in each of the read sequence pairs is at the same position in the reference sequence, and classifying the collected read sequence pairs into the same group.

[21] The method according to [19] or [20], wherein, preferably, the step (3) includes collecting, from each of the groups of read sequence pairs, at least one read sequence pair derived from each of the two complementary strands constituting one fragment of the sample DNA, and building consensus of sequence information among read sequences included in the collected read sequence pairs.

[22] A method for detecting a mutation in genomic DNA, the method comprising:

preparing a sequencing library by the method according to any one of [1] to [11] with use of genomic DNA as sample DNA; and
sequencing the sequencing library.

[23] The method according to [22], wherein, preferably, the sequencing is performed by the method according to any one of [13] to [21].

[24] The method according to [22] or [23], wherein, preferably, the mutation is a base-pair substitution mutation.

## Examples

Hereinafter, Examples will be shown to more specifically describe the present invention.

## Reference Example 1: Sequencing and mutation analysis

The flows of a sequencing method and mutation analysis used in Comparative Examples and Examples described later will be described in the following. Fundamentally, the highly accurate sequencing method utilizing information on complementary strands, which is described in Patent Literature 4, was used. Specifically, a library was subjected to sequencing, and read pairs expected to be derived from the same DNA fragment were collected. Then, a consensus read sequence (a consensus read sequence considering complementary strands) was generated among read sequences expected to be derived from two complementary strands (hereinafter, referred to as strand A and strand B) of the DNA fragment. Acquired consensus read sequences considering complementary strands were used for mutation analysis.

### 1) Sequencing utilizing information on complementary strands

The sequencer HiSeq from Illumina, Inc. was used. A library for the sequencer HiSeq contains PCR products derived from both of two complementary strands of each fragment of sample DNA. Hence, the library was subjected to sequencing to generate read 1 and read 2 for each of the two complementary strands.

To identify read sequences for strands complementary to each other, label sequences for complementary strands (adapter sequences attached to a TruSeq from Illumina, Inc.) were linked to each terminal of each fragment of sample DNA before PCR. Subsequently, PCR with primers which specifically bind to the adapter sequences was performed to produce PCR products including the adapter sequences, and the PCR products were used as a library for sequencing. In the sequencer HiSeq, each of the amplified products is bound onto a flow cell, which is used in sequencing, through annealing of the adapter sequence included in the amplified product with an oligo DNA fragment on the flow cell, and sequenced.

In sequencing, a pair of two read sequences (read 1, read 2) was acquired for each amplified fragment (derived from a fragment of sample DNA) contained in each PCR product in the library. Here, the read sequence containing sequence information on reading out of the sequence of one strand of the amplified fragment from the 5'-terminal side to the 3'-terminal side was read 1 (R1), and the read sequence containing sequence information on reading out of the sequence of the same strand from the 3'-terminal side to the 5'-terminal side was read 2 (R2).

### 2) Edit of read sequences and extraction of information on complementary strands

Adapter sequences and bases such as those of low quality were trimmed from the read sequences acquired in 1), and the resultants were then mapped on the reference sequence. Schematic Diagram 1 shows a conceptual diagram illustrating location of read pairs derived from two complementary strands of a fragment of sample DNA in a reference sequence when the read pairs are mapped on the reference sequence. For reference, two complementary strands of a fragment of sample DNA from which the read pairs are derived are illustrated in Schematic Diagram 1. A region between the beginning of read 1 and the beginning of read 2 in the reference sequence is the same among read pairs derived from strands complementary to each other. Therefore, read pairs expected to be derived from the same fragment of sample DNA were collected on the basis of the position of each read pair mapped on the reference sequence.

[Chem. 3]

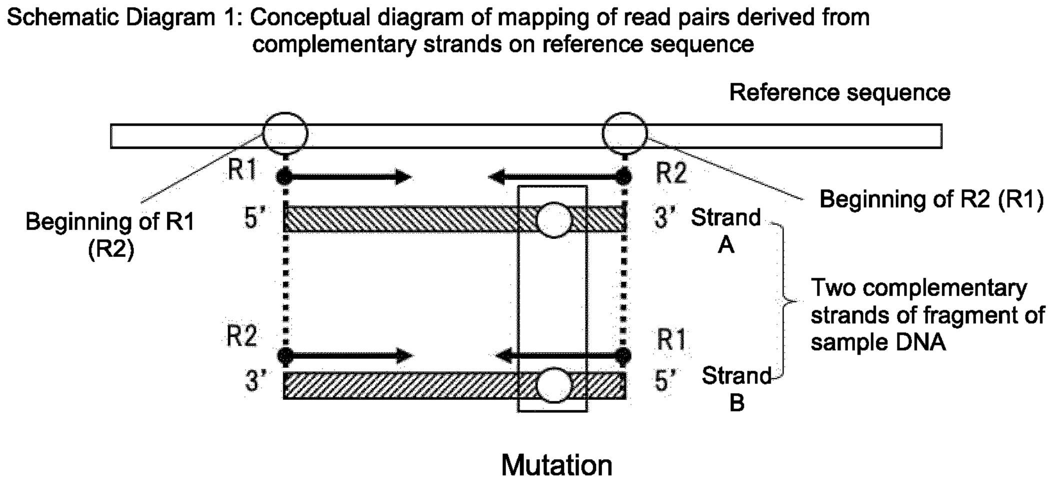


For the present method, "estimated fragment" refers to a region from the beginning of read 1 (read 2) to the beginning of read 2 (read 1) mapped on a reference sequence, in other words, a region between the beginning of read 1 and the beginning of read 2 when read pairs (read 1, read 2) are mapped on the reference sequence; and "group" for the estimated fragment refers to a group of read pairs to give a common estimated fragment (Schematic Diagram 2).

[Chem. 4]

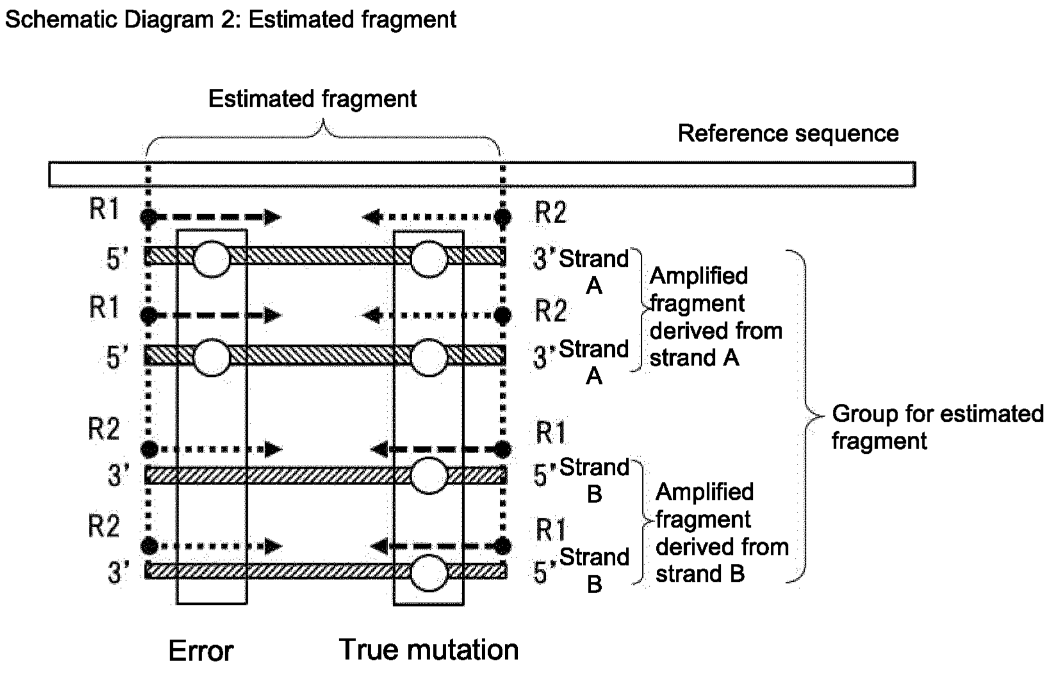


Subsequently, from the group for an estimated fragment, combination of the read pairs from one and the other of two strands complementary to each other was acquired as a set of read pairs.

Amplified fragments obtained from a fragment of sample DNA may possess mutations originally included in the fragment of sample DNA in both strands, and may additionally include a base substitution only in one strand due to oxidative modification of the fragment of sample DNA or the like. Such a case is illustrated in Schematic Diagrams 1 and 2. The fragment of sample DNA shown in Schematic Diagram 1 possesses one base substitution due to mutation (true mutation) in each strand. On the other hand, the amplified fragments derived from the fragment of sample DNA shown in Schematic Diagram 2 possess a base substitution due to mutation (true mutation) in both strands, and additionally include a base substitution generated in the course of sample preparation (error) only in one strand. These true mutation and error have been read out into read 1 and read 2 of each read pair. In the present method, the true mutation fixed in both strands was distinguished from the error generated only in one strand and the true mutation was extracted, through use of sequence information from complementary strands contained in the set of read pairs.

In the present method, a consensus read sequence considering complementary strands was generated from each set of collected read pairs. In generating consensus read sequences considering complementary strands, read pairs to give a common estimated fragment were first collected, and the collected read pairs were classified into read pairs derived from strand A and read pairs derived from strand B. Subsequently, combination of one or more read pairs derived from strand A and one or more read pairs derived from strand B was acquired as a set of read pairs, and a consensus read sequence considering complementary strands was generated by using the set of read pairs. The number of read pairs derived from strand A or strand B which were included in the set of read pairs was not particularly limited, and it was only required to include at least one or more read pairs derived from strand A and at least one or more read pairs derived from strand B. Even in the case that the number of read pairs derived from strand A was two and the number of read pairs derived from strand B was two, for example, or in the case that the number of read pairs derived from strand A was three and the number of read pairs derived from strand B was one, a consensus read sequence considering complementary strands was generated by building consensus among them.

Schematic Diagram 3 below shows, as an example, a more detail procedure from making collections of read pairs to generating consensus read sequences considering complementary strands. In the present method, read pairs derived from each complementary strand were first mapped on a reference sequence as illustrated in Schematic Diagram 3 (1). Then, a group of read pairs whose left ends (ends closest to the 5'-terminal in the reference sequence) were present at the same position in the reference sequence was acquired as a first collection (2). Subsequently, read pairs whose right ends (ends closest to the 3'-terminal in the reference sequence) were present at the same position in the reference sequence were separated from the first collection and acquired as a second collection (3). This second collection was a collection of read pairs to give a common estimated fragment. The second collection was then classified into a group derived from strand A (group F) and a group derived from strand B (group R) (4). Here, the group derived from strand A and the group derived from strand B could be distinguished on the basis of information on label sequences acquired in sequencing. In the present method, sequencing reaction was performed by using a flow cell which recognizes the label sequence in each adapter sequence added to each fragment of sample DNA and allows it to bind to the flow cell. After amplification of fragments in the flow cell, the label sequence in the adapter sequence added in the 5'-terminal side was specifically cut to unify the direction of sequencing for read 1 and read 2 for each amplified fragment, and read pairs were classified into group F and group R on the basis of information on the label sequence. Group F and group R were each a collection of read pairs derived from either one of two complementary strands constituting a DNA fragment. Hence, a consensus read sequence considering complementary strands was generated by building consensus between group F and group R (5).

[Chem. 5]

Schematic Diagram 3: Procedure to generate consensus read sequences considering complementary strands from read pairs

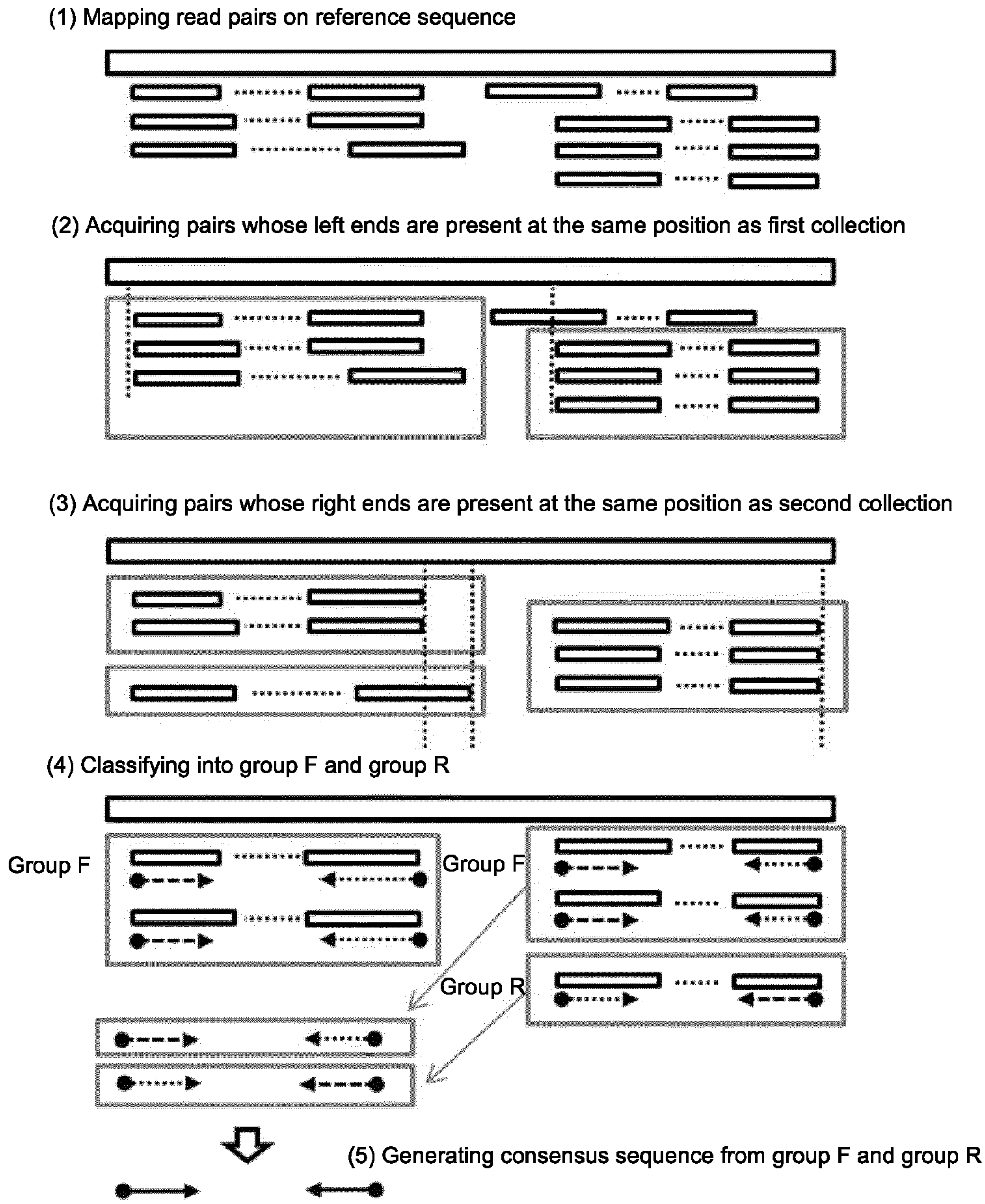


By generating consensus read sequences considering complementary strands, mutations present in both strands in common were acquired as true mutations while mutations generated only in one strand were excluded as errors.

3) Mutation analysis

Mutations in the target genome were detected by re-mapping the consensus read sequences considering complementary strands acquired in 2) on the reference sequence. The specific procedure to detect mutated bases from the consensus read sequences considering complementary strands re-mapped on the reference sequence was in accordance with a procedure described in WO/2018/150513.

4) Software, program

Schematic Diagram 4 illustrates a flow of edit of read sequences, extraction of information on complementary strands, and mutation analysis. In analyses, the software Cutadapt, the software Bowtie2, the software Samtools, and

programs written in the programming language Python were used. First, adapter sequences and bases such as those of low quality were trimmed from Fastq files (read 1 and read 2) derived from each library by using the software Cutadapt. Thereafter, each of the Fastq files derived from each library was subjected to mapping on a reference sequence by using the software Bowtie2 to provide a file in Sam format. The read of each file in Sam format was sorted by using the software Samtools, and a group for an estimated fragment was then generated and sets of read pairs were collected therefrom to generate consensus read sequences considering complementary strands, by using programs written in the programming language Python. The acquired consensus read sequences considering complementary strands were re-mapped on the reference sequence by using the software Bowtie2, and mutation analysis was performed by using the software Samtools and programs written in the programming language Python.

[Chem. 6]

Schematic Diagram 4: Analysis flow

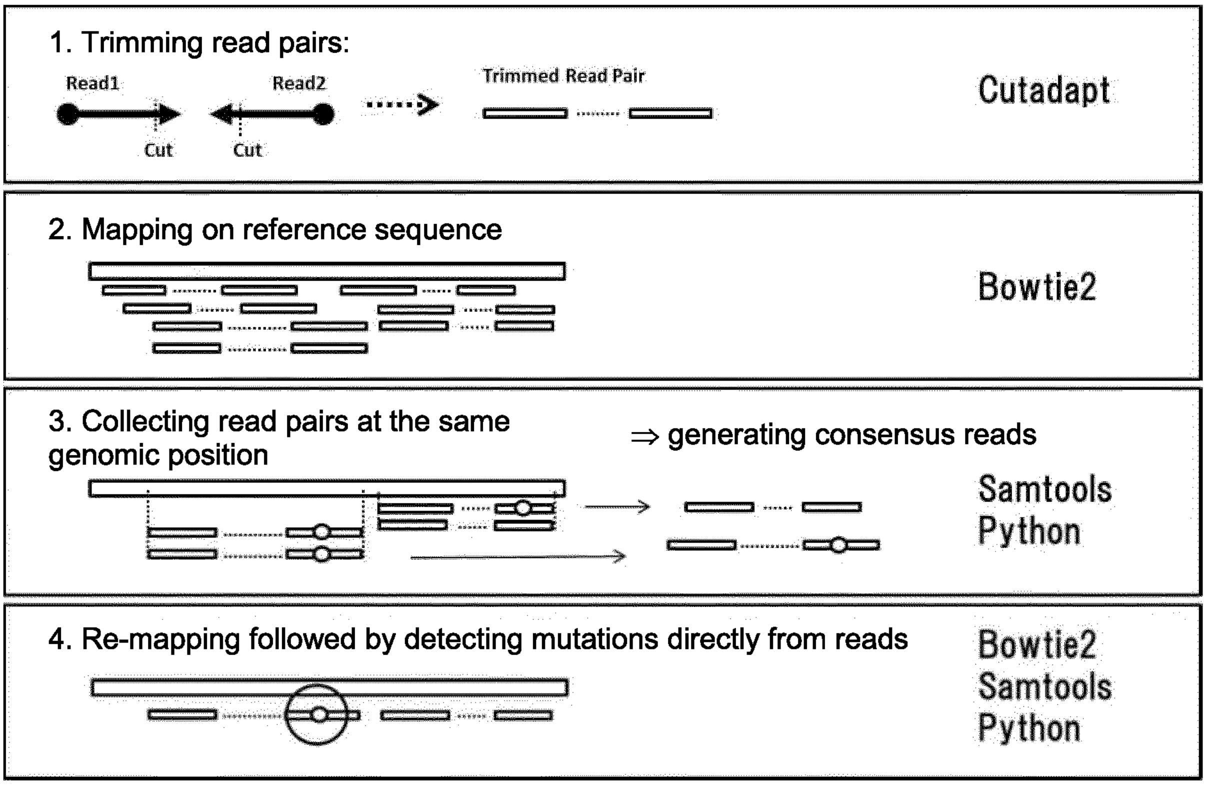


Comparative Example 1: Error reduction by removing bases at both terminals of read pairs

The presence of errors derived from terminal single-stranded protruding sites in fragments of fresh genomic DNA was examined by using the sequencing method in Reference Example 1. In addition, the error reduction effect of a method of removing bases from both terminals of read pairs, an existing improving method for terminal errors, was examined.

1) Sample DNA

Genomic DNA of the LT-2 TA100 strain of Salmonella typhimurium (hereinafter, also referred to as "TA100 strain", simply) exposed to dimethylsulfoxide (DMSO; produced by Wako Pure Chemical Industries, Ltd.) was used as sample DNA.

Exposure of the TA100 cell line to DMSO was performed in accordance with the Ames pre-incubation method (Mol. Mech. Mutagen., 455:29-60, 2000, Sci. Rep. 8 (1) :9583) . TA100 strain was seeded in 2 mL of Nutrient Broth No. 2 (produced by Oxoid Limited), and subjected to shaking culture at 37°C and 180 rpm for 4 hours to afford pre-culture solution with an OD660 value of 1.0 or higher. Into test tubes, 100 µL of DMSO, 500 µL of S9 mix (produced by IEDA TRADING Corporation), and 100 µL of the pre-culture solution were added, and the resultants were subjected to shaking

culture at 100 rpm in a water bath at 37°C for 20 minutes (DMSO-exposed cells). After shaking culture for 20 minutes, the test tubes each containing a culture solution were taken out of the water bath, and 50 μL of each culture solution was added to 2 mL of Nutrient Broth solution (containing 18.5% S9 mix) aliquoted in advance, and the resultant was further cultured in an incubator at 37°C and 180 rpm for 14 hours. After the culture, the bacterial suspensions were collected and centrifuged at 7,500 rpm for 5 minutes, and the supernatants were removed to collect the cells. From the DMSO-exposed cells, total DNA was collected by using a DNeasy Blood & Tissue Kit (produced by QIAGEN) in accordance with the recommended protocol. The double-stranded DNA concentration of each DNA sample obtained was measured by using a Qubit3.0 Fluorometer (produced by Thermo Fisher Scientific) with a Qubit (TM) dsDNA BR Assay Kit attached thereto.

2) Preparation of sequencing library

A TruSeq Nano DNA Library Prep Kit (produced by Illumina, Inc., hereinafter abbreviated as TruSeq) was used for library preparation from the sample DNA. The recommended protocol of the TruSeq is composed of fragmentation of DNA, End Repair (blunting of single-stranded protruding terminals of double-stranded DNA fragments), A-tailing (addition of adenine to 3'-terminals of double-stranded DNA fragments), Adapter ligation (addition of adapters to both terminals of double-stranded DNA fragments), and PCR enrichment (concentration of library DNA by PCR amplification). From DNA derived from the DMSO-exposed cells obtained in 1), a plurality of samples each of which was to give 120 ng of DNA were prepared, and the samples were each fragmented into pieces having a length of approximately 350 bp on average by using a DNA Shearing System ME220 (produced by Covaris Inc.) in accordance with the recommended protocol. The fragmented DNA obtained was subjected to End Repair, A-tailing, and Adapter Ligation. The reaction solution resulting from Adapter Ligation was purified in accordance with the recommended protocol to afford DNA such that an adapter was added to each terminal of each double-stranded DNA fragment (adapter-ligated DNA). The concentration of the adapter-ligated DNA was measured by using a High Sensitivity D5000 Kit in an Agilent 4200 TapeStation (produced by Agilent Technologies).

On the basis of Patent Literature 4, the initial amount of the adapter-ligated DNA (the amount of input DNA) for use in PCR under the optimum conditions was estimated to be 78 amol (15.6 amol/Mbp). With considering this, serial dilution was performed with Resuspension buffer attached to the TruSeq to afford 25 μL of diluted solution containing 78 amol of the adapter-ligated DNA. The diluted solution obtained was subjected to PCR enrichment in accordance with the recommended protocol. In view of the amount of input DNA being 78 amol and the amount of DNA necessary for sequencing, PCR was performed in 15 cycles. DNA was purified from the reaction solution in accordance with the recommended protocol to prepare a library. The concentration of the library DNA was measured by using a High Sensitivity D1000 Kit in an Agilent 4200 TapeStation.

3) Sequencing and mutation analysis

The library prepared in 2) was subjected to sequencing with the read length set to 2 × 100 bp to acquire sequencing data of approximately 10 Gbp (approximately 50 M read pairs) on average per library. After consensus read sequences considering complementary strands were generated from the sequencing data acquired and mapped on a reference sequence, mutated bases were detected. Sequencing, generation of consensus read sequences considering complementary strands, and mutation analysis were performed in accordance with the procedure in Reference Example 1. For the reference sequence, the genomic sequence of the LT-2 strain of S. typhimurium (hereinafter, also expressed as LT-2 strain, simply) obtained from GenBank (www.ncbi.nlm.nih.gov/genbank/) was used (GenBank assembly accession: GCA_000006945.2).

4) Calculation of mutation frequencies

For each library, all bases targeted in analysis in all of the consensus read sequences considering complementary strands mapped on the reference sequence were classified by the corresponding base (A, T, G, and C) in the reference sequence into four groups by using a program written in Python. Subsequently, the total number of bases was counted and mutated bases as compared with the reference sequence were detected for each group. The mutations detected were classified into six mutation patterns (AT → TA, AT → CG, AT → GC, and GC → TA, GC → CG, GC → AT), and the mutation frequency for each mutation pattern was calculated. Further, each mutation pattern was classified by the corresponding base in the reference sequence on which read sequences have been mapped into two mutation patterns, and the mutation frequency for each mutation pattern was calculated. More specifically, AT → TA was classified into A → T and T → A, AT → CG into A → C and T → G, AT → GC into A → G and T → C, GC → TA into G → T and C → A, GC → CG into G → C and C → G, and GC → AT into G → A and C → T, and the mutation frequency for each of the 12 mutation patterns was calculated.

5) Error reduction by removal of bases from both terminals of read pairs

In accordance with the procedure in Reference Example 1, the consensus read sequences considering complementary strands acquired in 3) were re-mapped on the reference sequence to generate a file in Sam format. In the file in Sam format, 0 bases (control), 10 bases, or 20 bases at each terminal of read pairs were excluded from targets of mutation analysis by lowering the quality values with a program written in Python. Thereafter, mutation analysis was performed in accordance with the procedure in Reference Example 1. The mutation frequency for each of the 12 mutation patterns shown in 4) was calculated.

6) Results and discussion

Figure 1 shows the mutation frequencies for the six mutation patterns calculated in 4) in the sample DNA. From the result that the mutation frequency of GC base pairs was higher than that of AT base pairs, the presence of errors due to the oxidative modification of guanine was expected. Figure 2 shows the mutation frequencies for the 12 mutation patterns calculated from read pairs removed of both terminals in 5). In mutations of GC base pairs (GC $\rightarrow$ TA, GC $\rightarrow$ CG), G $\rightarrow$ T and G $\rightarrow$ C mutations were more frequently detected than C $\rightarrow$ A and C $\rightarrow$ G mutations. True mutations should be detected evenly among the bases G and C. The result that mutations of guanine were frequently detected suggests that those mutations are not true mutations but errors caused by mutation of bases due to oxidative modification or the like. The mutation frequencies for G $\rightarrow$ T and G $\rightarrow$ C decreased in a manner depending on the number of bases removed from each terminal of read pairs. This result indicated that errors due to the mutation of guanine were abundant around both terminals of read pairs. Therefore, the mutation of guanine due to oxidative modification or the like at terminal single-stranded sites of DNA fragments was inferred to be a primary cause for the errors.

Subsequently, for GC $\rightarrow$ TA and GC $\rightarrow$ CG mutations, the difference in mutation frequency between the mutation of G and the mutation of C was calculated, and the error reduction rate given by removal of both terminals of read pairs was determined on the basis of the following equation:

$$\text{Error reduction rate (\%)} = (A-B)/A \times 100$$

A: Difference in mutation frequency between G and C without removal of bases from both terminals (control)
B: Difference in mutation frequency between G and C with removal of bases from both terminals

Table 1 shows error reduction rates. The error reduction rate for 10-base removal was <30%, and that for 20-base removal was as low as about 40%. The method of removing five bases from each terminal, which was reported by Kennedy et al. (Non Patent Literature 3), was expected to give much lower error reduction effect than 10-base removal. Those results indicate that terminal single-stranded protruding moieties of DNA fragments were not sufficiently removed in a successful manner by removal of 20 bases from each terminal. Increasing the number of bases to be removed is expected to further reduce errors, whereas removal of many bases from read pairs lowers the data efficiency because the number of bases available for mutation analysis decreases. Thus, removal of both terminals of read pairs from targets of mutation analysis is not an effective approach for reducing errors due to oxidative modification or the like at terminal single-stranded sites of DNA fragments.

[Table 1]

| **Mutation pattern** | **Error reduction rate (%)** | |
|---|---|---|
| | **Removal of 10 bases from each terminal** | **Removal of 20 bases from each terminal** |
| **GC to TA** | **27.5** | **40.2** |
| **GC to CG** | **26.2** | **40.0** |

Example 1: Error reduction by library preparation method using single-strand-specific nuclease

The error reduction effect of single-strand-specific nuclease treatment for DNA fragments was evaluated.

1) Sample DNA

DMSO-exposed cells were prepared with the same procedure as in 1) of Comparative Example 1, and TA100

strain was exposed to 3-methylcholanthrene (3-MC) with the same procedure. In DMSO, 3-MC (produced by Sigma-Aldrich Co. LLC., CASRN.56-49-5) was dissolved. Into test tubes, 100 μL of the 3-MC solution, 500 μL of S9 mix (produced by IEDA TRADING Corporation), and 100 μL of the pre-culture solution for TA100 strain were added (amount of 3-MC: 1000 gg/tube), and the resultants were subjected to shaking culture at 100 rpm in a water bath at 37°C for 20 minutes (3-MC-exposed cells). Cells were collected from the bacterial suspensions, and DNA was extracted with the same procedure as in 1) of Comparative Example 1.

2) Ames test

For the Ames test, a bacterial suspension exposed to 3-MC under the above-described conditions was prepared. Thereto, 2 mL of top agar (containing 1% NaCl, 1% agar, 0.05 mM histidine, and 0.05 mM biotin) warmed to 45°C was added and the resultant was stirred by using a Vortex, and poured onto a minimal glucose agar medium (Tesmedia (R) AN; produced by Oriental Yeast Co., Ltd.) to form a layer. The resulting plate was subjected to culturing at 37°C for 48 hours, and the number of colonies observed was counted.

3) Preparation of sequencing libraries

I) Fragmentation of sample DNA

From DNA derived from the DMSO-exposed cells or the 3-MC-exposed cells, a plurality of samples each of which was to give 60 ng or 100 ng of DNA were prepared, and the samples were each fragmented into pieces having a length of approximately 350 bp on average by using a DNA Shearing System ME220. The fragments of each sample were divided into two groups. For the group without nuclease treatment (nontreatment group), the DNA fragments were suspended in Resuspension buffer attached to the TruSeq for End Repair in the subsequent step in accordance with the recommended protocol to afford 60 μL of eluate. For the group with nuclease treatment (treatment group), the DNA fragments were purified through steps of adsorbing on Sample Purification Beads attached to the TruSeq (hereinafter, also expressed as beads, simply), washing twice with 80% ethanol water, and drying in accordance with the recommended protocol. In the subsequent elution operation, the beads were suspended in distilled water (DW, produced by NIPPON GENE CO., LTD.) to afford 30 μL of DNA eluate containing DNA fragments.

II) Nuclease treatment

S1 nuclease (Promega Corporation, catalog No.: M5761), Mung Bean Nuclease (MBN) (Takara Bio Inc., catalog No.: 2420A), or $RecJ_f$ (New England Biolabs, catalog No.: M0264L) was used as the single-strand-specific nuclease. The activity values (numbers of units) of the enzymes were defined as follows.

- S1 nuclease: an enzymatic activity such that 1 μg of acid-soluble substance is generated per minute at 37°C in a mixed solution of 30 mM sodium acetate (pH 4.6, 25°C), 50 mM NaCl, 1 mM $ZnCl_2$, 5% glycerol, and 0.5 mg/mL denatured calf thymus DNA was defined as 1 U.
- MBN: an enzymatic activity such that 1 μg of acid-soluble decomposition product is generated per minute at 37°C and pH 5.0 with thermally denatured calf thymus DNA as a substrate was defined as 1 U.
- RecJf: an amount of the enzyme necessary for generating 0.5 ng of deoxyribonucleotide soluble in trichloroacetic acid per minute at 37°C in 50 μL of whole reaction solution (containing 1× NE Buffer 2 and 1.5 μg of sonicated [$^3$H]-labeled single-stranded E. coli DNA) was defined as 1 U.

II-1) S1 nuclease treatment

To the DNA eluate obtained in I), 4 μL of 10× Reaction Buffer attached to the S1 nuclease was added. The S1 nuclease was appropriately diluted with 1× Reaction Buffer, 1, 3, 10, 30, 100, or 300 U thereof was added to the DNA eluate, and DW was added thereto to a total volume of 40 μL. For the sample to which 1000 U of the S1 nuclease was to be added, 4.6 μL of 10× Reaction Buffer was added to 30 μL of the DNA eluate obtained by beads purification, and 12 μL of the stock solution of the S1 nuclease was added thereto to a total volume of 46 μL. Each reaction solution with the S1 nuclease was incubated at 30°C for 30 minutes. Assuming that the whole amount of DNA (60 ng) present at the beginning of library preparation existed in each reaction solution, the numbers of units of the S1 nuclease per 1 ng of DNA were 0.02, 0.05, 0.17, 0.50, 1.67, 5.00, and 16.7 U/ng. For inactivation of the S1 nuclease in each reaction solution, 3 μL of 0.5 M EDTA (pH 8.0) (produced by NIPPON GENE CO., LTD.) was added, and the resultant was incubated at 70°C for 10 minutes. To purify DNA from the inactivated reaction solution, beads attached to the TruSeq in an amount equal to that of the reaction solution were added and a purification operation was carried out in accordance

with the recommended protocol, and the resultant was suspended in Resuspension buffer attached to the TruSeq to afford 60 μL of eluate (S1 nuclease treatment group).

II-2) MBN treatment

To the DNA eluate obtained in I), 5 μL of 10× Mung Bean Nuclease Buffer attached to the MBN was added. The MBN was appropriately diluted with 1× Mung Bean Nuclease Buffer, and 3, 10, 30, or 100 U thereof was added to the DNA eluate to a total volume of 50 μL. Each reaction solution with the MBN was stirred, and incubated at 37°C for 10 minutes. For inactivation of the MBN in each enzymatic reaction solution, 3 μL of 0.5 M EDTA (pH 8.0) was added, and the resultant was incubated at 65°C for 10 minutes. Assuming that 100 ng of DNA fragments existed in each reaction solution, the numbers of units per 1 ng of DNA were 0.03, 0.1, 0.3, and 1.0 U/ng. To purify DNA from the inactivated reaction solution, beads attached to the TruSeq in an amount equal to that of the reaction solution were added and a purification operation was carried out in accordance with the recommended protocol, and the resultant was suspended in Resuspension buffer attached to the TruSeq to afford 60 μL of eluate (MBN treatment group).

II-3) $RecJ_f$ treatment

To the DNA eluate obtained in I), 5 μL of 10× NE Buffer 2 attached to the $RecJ_f$ was added. The $RecJ_f$ was appropriately diluted with 1× NE Buffer 2, and 3, 10, 30, or 100 units thereof was added to the DNA eluate to a total volume of 50 μL. Each reaction solution with the $RecJ_f$ was stirred, and incubated at 37°C for 60 minutes. Assuming that 100 ng of DNA fragments existed in each reaction solution, the numbers of units per 1 ng of DNA were 0.03, 0.1, 0.3, and 1.0 U/ng. For inactivation of the $RecJ_f$ in each enzymatic reaction solution, incubation was performed at 65°C for 20 minutes. To purify DNA from the inactivated reaction solution, beads attached to the TruSeq in an amount equal to that of the reaction solution were added and a purification operation was carried out in accordance with the recommended protocol, and the resultant was suspended in Resuspension buffer attached to the TruSeq to afford 60 μL of eluate ($RecJ_f$ treatment group).

III) End Repair, A-tailing, Adapter Ligation, and PCR enrichment

With the same procedure as in 2) of Comparative Example 1, End Repair, A-tailing, and Adapter Ligation were performed for the nontreatment group, S1 nuclease treatment group, MBN treatment group, and $RecJ_f$ treatment group obtained in II) in accordance with the recommended protocol of the TruSeq. Each reaction solution resulting from Adapter Ligation was purified in accordance with the recommended protocol to afford DNA such that an adapter was added to each terminal of each double-stranded DNA fragment (adapter-ligated DNA). The concentration of the adapter-ligated DNA was measured by using a High Sensitivity D5000 Kit in an Agilent 4200 TapeStation (produced by Agilent Technologies). Subsequently, PCR enrichment was performed with the same procedure as in 2) of Comparative Example 1 to give libraries.

4) Sequencing and mutation analysis

Each library prepared in 3) was subjected to sequencing with the read length set to 2 × 150 bp to acquire sequencing data of approximately 15 Gbp (approximately 50 M read pairs) on average per library. Generation of consensus read sequences considering complementary strands and mutation detection were performed with the sequencing data acquired. The sequencing, generation of consensus read sequences considering complementary strands, and mutation analysis were performed with the procedures in Reference Example 1.

5) Calculation of mutation frequencies

The mutation frequency for each of the six mutation patterns and 12 mutation patterns was calculated with the same procedure as in 4) of Comparative Example 1. Subsequently, for GC → TA and GC → CG mutations, the difference in mutation frequency between substitution of G and substitution of C was calculated, and the error reduction rate for each nuclease treatment group was determined on the basis of the following equation:

$$\text{Error reduction rate (\%)} = (A-B)/A \times 100$$

A: Difference in mutation frequency between G and C in nontreatment group (0 U/ng)
B: Difference in mutation frequency between G and C in nuclease treatment group with given number of units

6) Calculation of data efficiency

The data efficiency of each library was calculated from the number of read pairs in consensus read sequences considering complementary strands in the library used in the mutation analysis and the total number of read pairs read out in sequencing of the library (amount of sequencing data).

Data efficiency (%) = (number of read pairs in consensus read sequences considering complementary strands)/(amount of sequencing data) × 100

7) Mean number of read pairs per group

For each of consensus read sequences considering complementary strands generated in 4), the number of read pairs per group for an estimated fragment was counted, the number of groups including an equal number of read pairs was counted, and the mean number of read pairs was calculated:

Mean number of read pairs = {$\Sigma_i$ (i × (number of groups including i read pairs))}/(total number of groups) wherein i denotes the number of read pairs included in a group.

8) Results and discussion

I) Number of revertants in Ames test

Table 2 shows the number of revertant colonies after exposure to 3-MC. The data shown include measurements for three plates and mean values thereof. The number of revertant colonies was found to increase through exposure to 3-MC, and this result demonstrated that mutations had been introduced into the genome of TA100 strain through exposure to 3-MC.

[Table 2]

| Number of revertant colonies in Ames test | | | | |
|---|---|---|---|---|
| 3-MC concentration (μg/plate) | Number of revertants | | | |
| | 1 | 2 | 3 | Mean |
| 0 (DMSO) | 112 | 93 | 97 | 101 |
| 10 | 887 | 881 | 1279 | 1016 |
| 20 | 1539 | 1484 | 1509 | 1511 |
| 100 | 1519 | 1539 | 1797 | 1618 |

II) Error reduction effects of single-strand-specific nucleases

II-1) S1 nuclease

Figure 3 shows mutation frequencies for the six mutation patterns in the DMSO-exposed library. In the nontreatment group (S1 nuclease: 0 U/ng), the mutation frequency of GC base pairs was high as with the case of Comparative Example 1. The S1 nuclease treatment groups (S1 nuclease: from 0.2 to 16.7 U/ng), by contrast, exhibited reduction in mutation frequency in a manner depending on the number of units, and the error reduction effect saturated at 0.17 U/ng.

Next, frequencies for the 12 mutation patterns in the same library are shown in Figures 4 and 5. In the nontreatment group, G → T and G → C mutations were more frequently detected than C → A and C → G mutations as with the case of Comparative Example 1. In the S1 nuclease treatment groups, the mutation frequencies for G → T and G → C decreased as the number of units increased. Table 3 shows error reduction rates for GC → TA and GC → CG. The reduction of mutation frequency saturated at 0.17 U/ng or more, and the bias of mutation frequency between G and C was substantially improved. This was inferred to be caused by removal of guanine which had been present in single-stranded sites in fragments of the sample DNA and subjected to oxidative modification through specific decomposition of the single-stranded sites by the S1 nuclease. It was confirmed that S1 nuclease treatment at 0.17 U/ng or more allows errors due to the oxidative modification of bases in terminal single-stranded sites in DNA fragments to be removed.

[Table 3]

| **Error reduction rate (%)** | | | | | | | | |
|---|---|---|---|---|---|---|---|---|
| **S1 nuclease (U/ng DNA in reaction solution)** | | **0.02** | **0.05** | **0.17** | **0.50** | **1.67** | **5.00** | **16.7** |
| **Mutation pattern** | **GC to TA** | **52.8** | **35.3** | **83.1** | **69.9** | **81.7** | **84.5** | **79.6** |
| | **GC to CG** | **30.4** | **50.6** | **84.9** | **87.5** | **86.0** | **98.3** | **98.1** |

II-2) Mung Bean Nuclease

Figure 6 shows mutation frequencies for the six mutation patterns in the DMSO-exposed library with MBN treatment. In the MBN treatment groups (from 0.03 to 1.00 U/ng), the mutation frequencies decreased in a manner depending on the number of units. Next, frequencies for the 12 mutation patterns in the same library are shown in Figures 7 and 8. The MBN treatment groups exhibited considerable reduction in mutation frequency for G → C, and considerable reduction in difference in mutation frequency between G → C and C → G. Although reduction in mutation frequency was found for G → T, the reduction was smaller than that in the case of S1 nuclease, and the difference in mutation frequency between G → T and C → A was still present even for larger numbers of units. Table 4 shows error reduction rates for GC → TA and GC → CG. For GC → CG, error reduction effect was exhibited at 0.03 U/ng or more, and the bias of mutation frequency between G and C was largely improved at 0.10 U/ng or more. For GC → TA, on the other hand, the difference in mutation frequency between G and C was reduced, but the effect was small. This was considered to be, in part, due to the result that the mutation frequency for GC → TA in the DMSO-exposed library was lower than that in the results shown in II-1. Mean values of mutation frequency for G → T and C → A in DNA (n = 3) prepared by exposing to DMSO under the same conditions were $0.177 \times 10^{-6}$ and $0.042 \times 10^{-6}$, respectively. The error reduction rates for the mean values were found to be 11.4% (0.03 U/ng), 40.2% (0.10 U/ng), 15.6% (0.30 U/ng), and 57.8% (1.00 U/ng). Thus, the error reduction effect of MBN was confirmed, though the effect was smaller than that of S1 nuclease.

[Table 4]

| **Error reduction rate (%)** | | | | | |
|---|---|---|---|---|---|
| **MBN (U/ng DNA in reaction solution)** | | **0.03** | **0.10** | **0.30** | **1.00** |
| **Mutation pattern** | **GC to TA** | **-33.5** | **9.8** | **-27.2** | **36.4** |
| | **GC to CG** | **63.3** | **90.6** | **81.2** | **97.7** |

II-3) $RecJ_f$

Figure 9 shows mutation frequencies for the six mutation patterns in the DMSO-exposed library with $RecJ_f$ treatment. The result for the nontreatment group was in common with that for the MBN treatment groups. The $RecJ_f$ treatment groups (from 0.03 to 1.00 U/ng) exhibited reduction in mutation frequency in a manner depending on the number of units. Next, frequencies for the 12 mutation patterns in the same library are shown in Figures 10 and 11. In the RecJf treatment groups, reduction in mutation frequency were found for G → T and G → C, and reduction in difference in mutation frequency was exhibited between G → T and C → A and between G → C and C → G, but the effects were smaller than those of S1 nuclease. Table 5 shows error reduction rates for GC → TA and GC → CG. As with the case of II-2, in view of the low mutation frequency for GC → TA in the DMSO-exposed library, comparison was made with mean values of mutation frequency for G → T and C → A in DNA (n = 3) prepared by exposing to DMSO under the same conditions. The error reduction rates calculated by using these mean values were -10.8% (0.03 U/ng), 35.2% (0.10 U/ng), 54.1% (0.30 U/ng), and 62.3% (1.00 U/ng). Thus, $RecJ_f$ exhibited an error reduction effect comparable to

that of MBN for GC → TA, and an error reduction effect was found for GC → CG, though the error reduction effect was smaller than those of S1 nuclease and MBN. Error reduction effect was seemingly exhibited at 0.10 U/ng or more for both GC → TA and GC → CG.

[Table 5]

| **Error reduction rate (%)** | | | | | |
|---|---|---|---|---|---|
| **$RecJ_f$ (U/ng DNA in reaction solution)** | | **0.03** | **0.10** | **0.30** | **1.00** |
| **Mutation pattern** | **GC to TA** | **-66.9** | **2.3** | **30.8** | **43.3** |
| | **GC to CG** | **-30.6** | **8.4** | **51.5** | **60.0** |

III) Improvement of increase rate of mutation frequency by 3-MC exposure

III-1) S1 nuclease

Figures 12 and 13 show results of calculation of mutation frequencies for the six mutation patterns in the DMSO-exposed library (DMSO control) and the 3-MC-exposed library (3MC) by the method in 5) with different numbers of units of S1 nuclease. In the nontreatment group (control, 0 U/ng), as compared with the DMSO control, no distinct increase in mutation frequency was detected for any of the mutation patterns in 3-MC, whereas, for the S1 nuclease treatment groups, distinct increase in mutation frequency was found for GC → TA in 3-MC. This mutation pattern was consistent with a mutation pattern detected in the liver of transgenic mice exposed to 3-MC (Environ. Mol. Mutagen., 2000, 36:266-273). These results presumably indicated that the S1 nuclease treatment reduced sequencing errors derived from guanine in single strands, whereas true mutations were detected. Table 6 shows GC → TA mutation frequency increase rates (SN ratios) against the DMSO control in 3-MC. It was suggested that S1 nuclease treatment at 0.17 U/ng or more reduces sequencing errors, thereby allowing low-frequency mutations induced by mutagen exposure to be detected.

[Table 6]

| **GC → TA mutation frequency increase rate (3MC/DMSO control)** | | | | | | | | |
|---|---|---|---|---|---|---|---|---|
| S1 nuclease (U/ng DNA in reaction solution) | 0 | 0.02 | 0.05 | 0.17 | 0.50 | 1.67 | 5.00 | 16.7 |
| SN ratio | 1.27 | 2.1 | 1.97 | 2.72 | 3.75 | 3.35 | 1.63 | 3.59 |

III-2) Mung Bean Nuclease

As in III-1, Figure 14 shows results for the MBN treatment groups. In the MBN treatment groups, increase in mutation frequency was found for GC → TA in 3-MC, as with the case of the S1 nuclease treatment groups. Table 7 shows GC → TA mutation frequency increase rates (SN ratios) against the DMSO control in 3-MC. The SN ratio in the nontreatment group (0 U/ng) in this experiment was higher than that in III-1. This was because the mutation frequency for GC → TA in the DMSO control was lower than that in III-1, and the mutation frequency for GC → TA in 3-MC was higher than that in III-1. In view of this, a DMSO control and 3-MC (each n = 3) without MBN treatment were prepared under the same conditions, and mean values of mutation frequency for GC → TA were calculated for them, and the SN ratio was determined from the mean values. The results showed that the mean values for GC → TA in the DMSO control and 3-MC were $0.109 \times 10^{-6}$ and $0.176 \times 10^{-6}$, respectively, giving an SN ratio of 1.61. It was expected from the result that 0.10 U/ng or more of MBN provides an improved SN ratio.

[Table 7]

| **GC → TA mutation frequency increase rate (3MC/DMSO control)** | | | | | |
|---|---|---|---|---|---|
| **MBN (U/ng DNA in reaction solution)** | **0** | **0.03** | **0.10** | **0.30** | **1.00** |
| **SN ratio** | **2.04** | **1.60** | **1.81** | **1.34** | **1.92** |

III-3) $RecJ_f$

As in III-1, Figure 15 shows results for the RecJf treatment groups. In the RecJf treatment groups, increase in

mutation frequency was also found for GC → TA in 3-MC, as with the cases of the S1 nuclease and MBN treatment groups. Table 8 shows GC → TA mutation frequency increase rates (SN ratios) against the DMSO control in 3-MC. In light of the SN ratio (1.61) of the mean values of mutation frequency calculated in III-2), it was expected that 0.10 U/ng or more of $RecJ_f$ exhibits error reduction effect.

[Table 8]

| **GC → TA mutation frequency increase rate (3MC/DMSO control)** | | | | | |
|---|---|---|---|---|---|
| **$RecJ_f$ (U/ng DNA in reaction solution)** | **0** | **0.03** | **0.10** | **0.30** | **1.00** |
| **SN ratio** | **2.04** | **1.48** | **2.14** | **1.68** | **1.94** |

IV) Data efficiency and mean number of read pairs

While sequencing in the present Example was performed under conditions with the amount of input DNA being 78 amol, which were estimated to be the optimum conditions (see Patent Literature 4), the nuclease treatments may have affected the optimum conditions for sequencing. For this reason, whether the sequencing in the present Example was performed under the optimum conditions was evaluated on the basis of the data efficiency and the mean number of read pairs (Patent Literature 4) in the sequencing. Tables 9 to 11 show results of calculation of data efficiency and mean numbers of read pairs in the libraries treated with different numbers of units of S1 nuclease, MBN, or $RecJ_f$. While the optimum conditions for sequencing calculated in Patent Literature 4 were such that the data efficiency is from about 5 to 10% and the mean number of read pairs is approximately 2, similar results were obtained in the present Example. Accordingly, the influence of each nuclease treatment on the sequencing conditions was inferred to be small, thus resulting in achievement of sequencing under nearly optimum conditions in the present Example.

[Table 9]

| DMSO-exposed library | | | | | | | | |
|---|---|---|---|---|---|---|---|---|
| S1 nuclease (U/ng DNA in reaction solution) | 0 | 0.02 | 0.05 | 0.17 | 0.50 | 1.67 | 5.00 | 16.7 |
| Data efficiency (%) | 8.0 | 8.3 | 8.3 | 8.0 | 8.5 | 8.8 | 8.9 | 8.9 |
| Mean number of read pairs | 1.9 | 2.0 | 2.0 | 2.2 | 1.9 | 2.0 | 2.1 | 1.9 |
| 3-MC-exposed library | | | | | | | | |
| S1 nuclease (U/ng DNA in reaction solution) | 0 | 0.02 | 0.05 | 0.17 | 0.50 | 1.67 | 5.00 | 16.7 |
| Data efficiency (%) | 8.3 | 7.9 | 7.7 | 8.2 | 8.5 | 8.5 | 8.8 | 8.0 |
| Mean number of read pairs | 2.4 | 2.2 | 2.0 | 2.0 | 2.1 | 2.2 | 2.3 | 2.2 |

[Table 10]

| DMSO-exposed library | | | | | |
|---|---|---|---|---|---|
| MBN (U/ng DNA in reaction solution) | 0 | 0.03 | 0.10 | 0.30 | 1.00 |
| Data efficiency (%) | 8.2 | 8.8 | 8.6 | 8.9 | 9.0 |
| Mean number of read pairs | 2.4 | 2.4 | 2.1 | 2.2 | 2.3 |
| 3-MC-exposed library | | | | | |
| MBN (U/ng DNA in reaction solution) | 0 | 0.03 | 0.10 | 0.30 | 1.00 |
| Data efficiency (%) | 8.3 | 8.6 | 8.5 | 8.8 | 8.7 |
| Mean number of read pairs | 2.7 | 2.5 | 2.1 | 2.2 | 2.2 |

[Table 11]

| DMSO-exposed library | | | | | |
|---|---|---|---|---|---|
| $RecJ_f$ (U/ng DNA in reaction solution) | 0 | 0.03 | 0.10 | 0.30 | 1.00 |
| Data efficiency (%) | 8.2 | 8.6 | 7.4 | 8.6 | 8.8 |
| Mean number of read pairs | 2.4 | 2.4 | 2.8 | 2.6 | 2.3 |
| 3-MC-exposed library | | | | | |
| $RecJ_f$ (U/ng DNA in reaction solution) | 0 | 0.03 | 0.10 | 0.30 | 1.00 |
| Data efficiency (%) | 8.2 | 8.4 | 7.4 | 8.4 | 8.5 |
| Mean number of read pairs | 2.7 | 2.4 | 2.5 | 2.4 | 2.3 |

Example 2: Evaluation of influence on mutation analysis

In the present Example, to evaluate the influence of single-strand-specific nuclease treatment for DNA fragments on mutation analysis, examination was carried out on 1) the comprehensiveness of consensus read sequences considering complementary strands for the genome of LT-2 strain, and 2) misrecognition of different DNA fragments as identical fragments (misrecognition of fragments). The read pairs and consensus read sequences considering complementary strands obtained for each sample in Example 1 were used. S1 nuclease, MBN, and $RecJ_f$ were used as the single-strand-specific nuclease.

1) Comprehensiveness of consensus read sequences considering complementary strands for genome of LT-2 strain

The coverage of sequencing over the genome was examined to evaluate whether specific sites of the genome were specifically sequenced. Pieces of information on coverage at different genomic positions were extracted from consensus read sequences considering complementary strands of the DMSO-exposed library for the nontreatment group and S1 nuclease treatment groups, MBN treatment groups, and $RecJ_f$ treatment groups, and partitioned into genomic regions each consisting of about 100 bases by programs written in the programming language Python, and the coverage in each region was determined and normalized (the sum total of coverage is equal to 1) to construct a histogram. Further, covered rates (the proportion of genomic positions where the coverage was 1 or more), mean coverage, standard deviation of coverage (SD of coverage), and coefficients of variation (CV) in mapping on the genome of LT-2 strain were calculated.

$$\text{Coefficient of variation (CV) (\%)} = \text{(standard deviation of coverage)}/\text{(mean coverage)} \times 100$$

Figure 16 shows histograms of coverage for the nontreatment group and the S1 nuclease treatment groups at 0.17 U/ng or more, in which sequencing error reduction effect clearly appeared. The part with no coverage, which roughly ranges from the genomic position 800000 to 900000 and is observed in all the data in common, is a site where the uvrB gene is deleted in TA100 strain (J. Appl. Toxicol., 2017, 37:1125-1128). A histogram almost comparable to that for the nontreatment group was found for any of the S1 nuclease treatment groups, regardless of the number of units. The top of Table 12 shows covered rates, mean coverage, SD of coverage, and CV in the nontreatment group and S1 nuclease treatment groups when consensus read sequences considering complementary strands for the groups were mapped on the genome of LT-2 strain. It was confirmed that covered rates and CV almost comparable with those in the nontreatment group are given even for increased numbers of units of S1 nuclease. Figures 17 and 18 show histograms of coverage for the nontreatment group, and for the MBN treatment group and $RecJ_f$ treatment group, respectively, at 1.00 U/ng. A histogram almost comparable to that for the nontreatment group was found for both the MBN treatment group and the $RecJ_f$ treatment group. The bottom of Table 12 shows covered rates, mean coverage, SD of coverage, and CV in the nontreatment group, the MBN treatment group, and the $RecJ_f$ treatment group when consensus read sequences considering complementary strands for the groups were mapped on the genome of LT-2 strain. It was confirmed that covered rates and CV almost comparable with those in the nontreatment group are given for both the MBN treatment group and the $RecJ_f$ treatment group. The demonstrated results determined that single-strand-specific nuclease treatment for fragments of sample DNA causes almost no bias with respect to genomic regions to be sequenced.

[Table 12]

| S1 nuclease (U/ng DNA in reaction solution) | Covered rate (%) | Mean coverage | SD of coverage | CV (%) |
|---|---|---|---|---|
| 0 | 97.51 | 140.5 | 37.07 | 26.38 |
| 0.17 | 97.52 | 171.8 | 43.97 | 25.59 |
| 0.50 | 97.51 | 176.9 | 45.97 | 25.99 |
| 1.67 | 97.50 | 184.8 | 48.63 | 26.31 |
| 5.00 | 97.52 | 218.4 | 58.62 | 26.84 |
| 16.7 | 97.50 | 187.8 | 57.56 | 30.64 |
| | | | | |
| Nuclease (U/ng DNA in reaction solution) | Covered rate (%) | Mean coverage | SD of coverage | CV (%) |
| 0U | 97.54 | 263.1 | 66.81 | 25.40 |
| 1.00 (MBN) | 97.51 | 261.6 | 67.55 | 25.82 |
| 1.00 (RecJf) | 97.51 | 231.7 | 59.45 | 25.66 |

2) Misrecognition of fragments

If read pairs derived from different cells are accidentally mapped on the same position in a reference sequence in generating consensus read sequences considering complementary strands, the read pairs are misrecognized as read pairs derived from the same fragment of double-stranded DNA. At that time, if a read pair with a mutation has been obtained from DNA of one cell and a read pair without any mutation has been obtained from DNA of another cell, the true mutation is disadvantageously excluded as an error. Such misrecognition of different DNA fragments as identical fragments (misrecognition of fragments) can be minimized by adjusting the amount of input DNA in the process of amplification of DNA fragments in library preparation on the basis of the genome size of a target of analysis and adjusting the diversity of adapter-ligated DNA in the library. The amount of input DNA was 78 amol for all the libraries in Example 1, and hence misrecognition of fragments is normally negligible. In the present Example, examination was carried out on whether treatment with single-strand-specific nuclease makes misrecognition of fragments more frequent.

In the present analysis, index information in each adapter sequence was used for identification of sample DNA. A DMSO-exposed library and a 3-MC-exposed library were prepared by using adapter sequences differing in index information, and sequencing data were acquired. From the beginning of each of the Fastq files (read 1, read 2) for the libraries, 25 M reads were extracted, data of read 1 were combined and data of read 2 were combined, generating Fastq files each consisting of 50 M reads with two pieces of index information for read 1 and read 2. In this way, sequencing data containing a mixture of read pairs derived from different genomic DNAs were generated for different numbers of units of single-strand-specific nuclease. These data were mapped on a reference sequence, and groups of read pairs were generated in accordance with the method in Reference Example 1. From the groups, groups including two or more read pairs were extracted, and the proportion of cases with inclusion of read pairs derived from different genomic DNAs (proportion of cases with inclusion of different indexes = frequency of misrecognition of fragments) was calculated on the basis of the index information of read pairs in each group.

$$\text{Proportion of cases with inclusion of different indexes (\%)} = (\text{number of groups with different pieces of index information})/(\text{number of groups including two or more read pairs}) \times 100$$

I) S1 nuclease

Figure 19 and Table 13 show proportions of cases with inclusion of different indexes, in other words, frequencies of misrecognition of fragments, in the S1 nuclease treatment groups at different numbers of units. The proportion of

cases with inclusion of different indexes increased as the number of units of S1 nuclease increased. Because two pieces of index information were used in the present Example, about half of the actual occurrences of misrecognition of fragments were inferred to be detected, and hence a value approximately twice a calculated proportion of cases with inclusion of different indexes was estimated to be the actual frequency of misrecognition. For S1 nuclease treatment at 0.17 U/ng or more, which substantially reduced sequencing errors, the frequency of misrecognition of fragments was about 7% or higher, which was so problematic as to affect mutation frequencies.

[Table 13]

| S1 nuclease (U/ng DNA in reaction solution) | 0 | 0.02 | 0.05 | 0.17 | 0.50 | 1.67 |
|---|---|---|---|---|---|---|
| Proportion (%) of cases with inclusion of different indexes | 1.53 | 1.60 | 1.77 | 3.56 | 7.44 | 10.32 |

II) MBN

Figure 20 and Table 14 show frequencies of misrecognition of fragments in the MBN treatment groups at different numbers of units. In the case of MBN, the proportion of cases with inclusion of different indexes also increased as the number of units increased. The estimated frequency of misrecognition (a value approximately twice the proportion of cases with inclusion of different indexes) at 0.10 U/ng or more was about 6% or higher, which was so problematic as to affect mutation detection.

[Table 14]

| MBN (U/ng DNA in reaction solution) | 0 | 0.03 | 0.10 | 0.30 | 1.00 |
|---|---|---|---|---|---|
| Proportion (%) of cases with inclusion of different indexes | 1.58 | 1.83 | 3.16 | 6.30 | 12.65 |

III) $RecJ_f$

Figure 21 and Table 15 show frequencies of misrecognition of fragments in the $RecJ_f$ treatment groups at different numbers of units. The proportion of cases with inclusion of different indexes slightly increased as the number of units increased, however, the degree of increase was not so large as to affect mutation detection.

[Table 15]

| $RecJ_f$ (U/ng DNA in reaction solution) | 0 | 0.03 | 0.10 | 0.30 | 1.00 |
|---|---|---|---|---|---|
| Proportion (%) of cases with inclusion of different indexes | 1.58 | 1.42 | 1.29 | 1.52 | 1.81 |

3) Results and discussion

The results in Example 1 confirmed that errors in sequencing can be reduced by treating DNA with S1 nuclease, MBN, or $RecJ_f$ before end repair. Thus, it was demonstrated that the single-strand-specific nucleases have error reduction effect in common. The error reduction effects were in the order of S1 nuclease > MBN > $RecJ_f$. This was considered to be, in part, because single-strand-specific exonuclease (RecJf) is incapable of decomposing single-stranded moieties each sandwiched by double strands, while single-strand-specific endonuclease (S1 nuclease and MBN) is capable of decomposing them. On the other hand, the results in Example 2 revealed that S1 nuclease and MBN used in combination with the highly accurate sequencing method cause increased frequency of misrecognition of fragments to affect mutation frequencies. The increased frequencies of misrecognition were considered to be due to the sequence specificity of the S1 nuclease and MBN activities. Specifically, residual single-stranded sequences, which are difficult to decompose with S1 nuclease or MBN, at terminals of DNA fragments cause accidental matching of both terminal sites of read pairs to increase the probability of being mapped on the same position in a reference sequence, resulting in increased frequencies of misrecognition. To solve this problem, two approaches were contemplated: (i) further decreasing the amount of input DNA; and (ii) S1 nuclease or MBN treatment of fragments followed by additional treatment of the fragments with a single-strand-specific nuclease of different specificity. The efficacies of these approaches were examined with use of S1 nuclease in the subsequent Examples. On the other hand, $RecJ_f$ did not substantially increase frequencies of misrecognition, and hence was considered to be applicable without affecting mutation detection.

Example 3: Influence of amount of input DNA on frequency of misrecognition of fragments

The frequency of misrecognition of fragments, in other words, the probability that read pairs are accidentally mapped on the same position in a reference sequence, can be reduced by decreasing the diversity of sample DNA in a library. In view of this, examination was performed in the present Example to determine whether accidental overlapping of reads can be reduced by decreasing the amount of input DNA in library preparation further below 78 amol.

1) Preparation of sequencing library

Genomic DNAs derived from the DMSO-exposed cells and the 3-MC-exposed cells prepared in Comparative Example 1 and Example 1 were used as sample DNAs. A plurality of samples each of which was to give 120 ng of DNA were prepared, and libraries subjected to S1 nuclease treatment were produced in accordance with the method described in Example 1. With considering reduction of sequencing errors and the frequency of misrecognition of fragments, the number of units of S1 nuclease was set to 0.08 U/ng (DNA) or 0.25 U/ng (DNA). In the process of PCR enrichment of adapter-ligated DNA, the amount of input DNA was set to 39 or 20 amol, and PCR amplification was performed for the DNA at 39 amol in 16 cycles and for the DNA at 20 amol in 17 cycles, with considering the amounts of DNAs in PCR products, thereby preparing libraries.

2) Sequencing and calculation of frequency of misrecognition of fragments

The libraries were subjected to sequencing in the same manner as in Example 1. Subsequently, the frequencies of misrecognition of fragments (proportions of cases with inclusion of different indexes) were calculated with the same procedure as in Example 2.

3) Results and discussion

Figure 22 and Table 16 show the frequencies of misrecognition of fragments. As with the case of 2) of Example 2, a value approximately twice a value in Table 16 was estimated to be the actual frequency of misrecognition. As described in Examples in Patent Literature 4, the frequency of misrecognition of fragments was successfully reduced by decreasing the amount of input DNA. In the case of treating with 0.08 U/ng of S1 nuclease, setting the amount of input DNA to 39 amol or less gave an actual frequency of misrecognition of about 5% or lower, successfully reducing the probability of missing a mutation as much as possible. Likewise, in the case of 0.25 U/ng of S1 nuclease, setting the amount of input DNA to 20 amol or less gave a frequency of misrecognition of 5% or lower.

[Table 16]

| Proportion (%) of cases with inclusion of different indexes | | | | |
|---|---|---|---|---|
| S1 nuclease (U/ng DNA in reaction solution) | | 0 | 0.08 | 0.25 |
| Amount of input DNA | 39 amol | 0.68 | 2.69 | 4.32 |
| | 20 amol | 0.31 | 1.29 | 2.05 |

The misrecognition of fragments in Example 2 and the results in the present Example demonstrated that the incidence of misrecognition of fragments increases in accordance with the enzyme level (U/ng), but the incidence of misrecognition of fragments can be reduced by selecting a proper amount of input DNA. The increase rate of misrecognition of fragments under S1 nuclease treatment was defined as the following equation, and calculated for each enzyme level, as shown in Table 17, on the basis of the results in Example 2.

Increase rate of misrecognition of fragments = [frequency of misrecognition of fragments (%) with S1 nuclease (U/ng) treatment]/[frequency of misrecognition of fragments (%) without S1 nuclease treatment]

[Table 17]

| S1 nuclease (U/ng DNA in reaction solution) | 0.017 | 0.050 | 0.167 | 0.500 | 1.67 |
|---|---|---|---|---|---|
| Increase rate of misrecognition of fragments | 1.04 | 1.16 | 2.32 | 4.85 | 6.72 |

As shown in Table 17, the influence on the frequency of misrecognition of fragments was negligible at enzyme levels of 0.05 U/ng or less, but the incidence of misrecognition of fragments increased at enzyme levels of more than

0.05 U/ng. For example, at enzyme levels in the range of more than 0.05 U/ng and 0.167 U/ng or less, the increase rate of misrecognition of fragments as compared with the case without S1 nuclease treatment was inferred to be about twice. Since the frequency of misrecognition of fragments depends on the amount of input DNA as described above, the proper range of the amount of input DNA at enzyme levels of more than 0.05 U/ng was considered to be about 1/2 or less of that without S1 nuclease treatment, specifically, to be 125 amol/Mbp or less, being 1/2 of 250 amol/Mbp. Similarly, at enzyme levels in the range of more than 0.167 U/ng and 0.5 U/ng or less, the increase rate of misrecognition of fragments as compared with the case without S1 nuclease treatment was inferred to be about four times, and the proper range of the amount of input DNA was considered to be 62.5 amol/Mbp or less. At enzyme levels of more than 0.5 U/ng, the increase rate of misrecognition of fragments as compared with the case without S1 nuclease treatment was inferred to be eight times or less, and the proper range of the amount of input DNA was estimated to be 31.3 amol/Mbp or less.

In addition, proper conditions for library preparation and sequencing were successfully derived by combining the relationship between increase rates of concentration for S1 nuclease treatment and increase rates of misrecognition of fragments and the relationship between amounts of input DNA and increase rates of misrecognition of fragments. As shown in Table 17, for example, the 10-fold increase of the enzyme level in S1 nuclease treatment from 0.17 U/ng to 1.67 U/ng resulted in about 3-fold increase in the frequency of misrecognition of fragments. Therefore, the frequency of misrecognition of fragments with increase in the enzyme level of S1 nuclease can be expressed as [$3^{\log \text{S1 nuclease (U/ng)}}$], wherein S1 nuclease (U/ng) > 0.05, and log denotes common logarithm. On the other hand, the results in the present Example found such a tendency that the frequency of misrecognition of fragments doubled as the amount of input DNA doubled. In light of the two results, conditions for library preparation and sequencing in the case that the enzyme level of S1 nuclease is more than 0.05 U/ng is reflected by an index represented by the following equation:

$$\text{Index} = \text{amount of input DNA in PCR (amol/Mbp sample DNA)} \times 3^{\log \text{S1 nuclease (U/ng)}}$$

wherein S1 nuclease (U/ng) > 0.05, and log denotes common logarithm.

Table 18 shows numerical values of the index under different conditions. In view of the proper range of conditions obtained through the examinations in the above Examples, the value of the index which allows sequencing under preferable conditions was considered to be 60 or less, more preferably 30 or less, even more preferably 15 or less, even more preferably 7.5 or less.

[Table 18]

| S1 nuclease (U/ng DNA in reaction solution) | | 0.08 | 0.17 | 0.25 | 0.50 | 1.67 |
|---|---|---|---|---|---|---|
| Amount of input DNA (amol) / sample DNA (Mbp) | 500 | 152.8 | 212.7 | 258.1 | 359.2 | 638.0 |
| | 250 | 76.4 | 106.3 | 129.0 | 179.6 | 319.0 |
| | 125 | 38.2 | 53.2 | 64.5 | 89.8 | 159.5 |
| | 62.5 | 19.1 | 26.6 | 32.3 | 44.9 | 79.7 |
| | 31.3 | 9.55 | 13.3 | 16.1 | 22.5 | 39.9 |
| | 15.6 | 4.77 | 6.65 | 8.06 | 11.2 | 19.9 |
| | 7.81 | 2.39 | 3.32 | 4.03 | 5.61 | 9.97 |
| | 3.91 | 1.19 | 1.66 | 2.02 | 2.81 | 4.98 |
| | 1.95 | 0.60 | 0.83 | 1.01 | 1.40 | 2.49 |

The results in Example 2 found that MBN also exhibited frequencies of misrecognition comparable to those for S1 nuclease at numbers of units comparable to those for S1 nuclease, and hence the relational equation and proper range of conditions derived above were expected to be directly applicable. On the other hand, the influence of the number of units of $RecJ_f$ on the frequency of misrecognition of fragments was negligible, as shown in Example 2.

Example 4: Influence of different nuclease treatments on frequency of misrecognition of fragments

The increase in the frequency of misrecognition of fragments caused by S1 nuclease treatment was inferred to be due to residual single strands, which are difficult to decompose with S1 nuclease, at terminals of DNA fragments.

Therefore, the frequency of misrecognition was considered to be improved by treatment of DNA fragments with S1 nuclease followed by additional treatment of the DNA fragments with a single-strand-specific nuclease of different specificity. In contrast to the endonuclease S1 nuclease, $RecJ_f$ has 5' → 3' exonuclease activity to decompose from the 5'-terminal of a single strand. In the present Example, examination was performed on the influence of S1 nuclease treatment of DNA fragments followed by additional treatment of the DNA fragments with $RecJ_f$ on the frequency of misrecognition of fragments.

1) Preparation of sequencing library

Genomic DNAs derived from the DMSO-exposed cells and the 3-MC-exposed cells prepared in Comparative Example 1 and Example 1 were used as sample DNAs. A plurality of samples each of which was to give 100 ng of DNA were prepared, and DNA eluates each containing 30 μL of fragments of sample DNA were obtained in accordance with the method described in 3) I) of Example 1.
Subsequently, the fragments were treated with 30 U (0.3 U/ng) of S1 nuclease in accordance with the method described in 3) II-1) of Example 1. After addition of EDTA and thermal denaturation, beads were added, DNA was purified from each reaction solution, and the resultants were divided into two groups. For the $RecJ_f$-treatment-free group, the beads were suspended in Resuspension buffer attached to the TruSeq to prepare 60 μL of eluate. For the $RecJ_f$ treatment group, the beads were suspended in distilled water to afford 30 μL of eluate, which was then subjected to $RecJ_f$ (3 (0.03), 10 (0.1), 30 (0.3), 100 (1.0) U (U/ng)) treatment in accordance with the method described in 3) II-3) of Example 1. After thermal denaturation, beads were added to each reaction solution for purification of DNA, and suspended in Resuspension buffer attached to the TruSeq to prepare 60 μL of eluate. Libraries were prepared from the obtained eluates in accordance with the recommended protocol of the TruSeq. In the process of PCR enrichment of adapter-ligated DNA, the amount of input DNA was set to 78 amol, and amplification was performed in 15 cycles.

2) Sequencing and calculation of frequency of misrecognition of fragments

The libraries were subjected to sequencing in the same manner as in Example 1. Subsequently, the frequencies of misrecognition of fragments (proportions of cases with inclusion of different indexes) were calculated with the same procedure as in Example 2.

3) Results and discussion

Figure 23 and Table 19 show frequencies of misrecognition for fragments subjected to treatment with 0.30 U/ng of S1 nuclease followed by treatment with $RecJ_f$ at different numbers of units. The frequency of misrecognition of fragments decreased as the number of units of $RecJ_f$ increased, though the decrease was slight. This was considered to be because single-stranded moieties left undecomposed after decomposition by S1 nuclease were decomposed by $RecJ_f$, which has different sequence specificity. Accordingly, the frequency of misrecognition of fragments was expected to be decreased by combination treatment with single-strand-specific nucleases differing in sequence specificity.

[Table 19]

| **$RecJ_f$ (U/ng DNA in reaction solution)** | **0** | **0.03** | **0.10** | **0.30** | **1.00** |
|---|---|---|---|---|---|
| **Proportion (%) of cases with inclusion of different indexes** | **6.03** | **6.14** | **5.85** | **5.80** | **5.43** |

Figure 24 shows mutation frequencies for the six mutation patterns in the DMSO-exposed library. In the present experiment, errors were sufficiently decreased even only with S1 nuclease treatment at 0.30 U/ng ($RecJ_f$: 0 U/mg), and hence it was considered that the frequency of misrecognition was not largely decreased by the additional treatment with $RecJ_f$.

## Claims

**1.** A method for preparing a sequencing library, the method comprising:

fragmenting sample DNA; and
treating prepared fragments of the sample DNA with a single-strand-specific nuclease to remove single-stranded moieties from the fragments, wherein

the sample DNA is DNA extracted from a living cell, DNA extracted from a frozen cell, or a stored sample of any of the DNAs.

**2.** The method according to claim 1, wherein the single-strand-specific nuclease is a single-strand-specific endonuclease, a single-strand-specific exonuclease, or a combination thereof.

**3.** The method according to claim 2, wherein the treating with the single-strand-specific nuclease includes treating the fragments of the sample DNA with a single-strand-specific endonuclease and then further treating the fragments of the sample DNA with a single-strand-specific exonuclease.

**4.** The method according to claim 2 or 3, wherein the single-strand-specific endonuclease is S1 nuclease or Mung Bean Nuclease.

**5.** The method according to claim 2 or 3, wherein the single-strand-specific endonuclease is S1 nuclease in an amount of 0.02 U/ng or more per 1 ng of the fragments of the sample DNA.

**6.** The method according to claim 2 or 3, wherein the single-strand-specific endonuclease is Mung Bean Nuclease in an amount of 0.02 U/ng or more per 1 ng of the fragments of the sample DNA.

**7.** The method according to claim 2 or 3, wherein the single-strand-specific exonuclease is $RecJ_f$.

**8.** The method according to claim 2 or 3, wherein the single-strand-specific exonuclease is $RecJ_f$ in an amount of 0.10 U/ng or more per 1 ng of the fragments of the sample DNA.

**9.** The method according to any one of claims 1 to 8, further comprising subjecting the fragments of the sample DNA treated with the single-strand-specific nuclease to one or more treatments selected from the group consisting of end repair, addition of a base to a terminal, and amplification.

**10.** The method according to claim 9, wherein

the amplification is PCR, the single-strand-specific nuclease is S1 nuclease, and
if the number of units (U/ng) of the S1 nuclease per 1 ng of the fragments of the sample DNA is 0.05 U/ng or less, the amount of input DNA in the PCR per 1 Mbp of the sample DNA is 250 amol or less, or
if the number of units (U/ng) of the S1 nuclease per 1 ng of the fragments of the sample DNA is more than 0.05 U/ng, an index represented by the following equation:

$$\text{Index} = \text{amount of input DNA in the PCR (amol/Mbp sample DNA)} \times 3^{\log \text{S1 nuclease (U/ng)}}$$

wherein S1 nuclease (U/ng) > 0.05, and log denotes common logarithm,
is 60 or less.

**11.** The method according to claim 9, wherein

the amplification is PCR, the single-strand-specific nuclease is Mung Bean Nuclease, and
if the number of units (U/ng) of the Mung Bean Nuclease per 1 ng of the fragments of the sample DNA is 0.05 U/ng or less, the amount of input DNA in the PCR per 1 Mbp of the sample DNA is 250 amol or less, or
if the number of units (U/ng) of the Mung Bean Nuclease per 1 ng of the fragments of the sample DNA is more than 0.05 U/ng, an index represented by the following equation:

$$\text{Index} = \text{amount of input DNA in the PCR (amol/Mbp sample DNA)} \times 3^{\log \text{Mung Bean Nuclease (U/ng)}}$$

wherein Mung Bean Nuclease (U/ng) > 0.05, and log denotes common logarithm, is 60 or less.

**12.** A method for sequencing DNA, the method comprising sequencing a sequencing library prepared by the method according to any one of claims 1 to 11.

**13.** The method according to claim 12, wherein the method for sequencing comprises:

(1) sequencing the library to generate one or more read sequences for each of a plurality of amplified fragments contained in the library, and acquiring a plurality of read sequences for the plurality of amplified fragments;
(2) collecting, from the plurality of read sequences acquired, read sequences containing sequence information on the same region in the sample DNA used in preparation of the library into a group to generate one or more groups of read sequences; and
(3) building consensus of sequence information among read sequences included in each of the groups of read sequences.

**14.** The method according to claim 13, wherein the step (1) includes generating one or more read sequences for each of amplified fragments derived from one and the other of two complementary strands constituting one fragment of the sample DNA.

**15.** The method according to claim 14, wherein the step (2) includes classifying read sequences to be mapped on the same position in a reference sequence into the same group.

**16.** The method according to claim 15, wherein the step (3) includes collecting, from each of the groups of read sequences, at least one read sequence derived from each of the two complementary strands constituting one fragment of the sample DNA, and building consensus of sequence information among the collected read sequences.

**17.** The method according to claim 13, wherein

the plurality of read sequences in the step (1) includes a plurality of read sequence pairs each consisting of read 1 and read 2, wherein
read 1 is a read sequence containing sequence information on reading out of the sequence of one strand of two complementary strands constituting one of the amplified fragments, from the 5'-terminal side to the 3'-terminal side, and
read 2 is a read sequence containing sequence information on reading out of the sequence of the one strand from the 3'-terminal side to the 5'-terminal side;
the step (2) includes collecting, from the read sequence pairs acquired, read sequence pairs containing sequence information on the same region in the sample DNA into a group to generate one or more groups of read sequence pairs; and
the step (3) includes building consensus of sequence information among read sequences included in each of the groups of read sequence pairs.

**18.** The method according to claim 17, wherein the step (1) includes generating one or more read sequence pairs for each of amplified fragments derived from one and the other of two complementary strands constituting one fragment of the sample DNA.

**19.** The method according to claim 18, wherein the step (2) includes mapping read 1 and read 2 of each of the read sequence pairs on a reference sequence, and classifying read sequence pairs into the same group such that a region between the beginning of read 1 and the beginning of read 2 in the reference sequence is the same thereamong.

**20.** The method according to claim 18, wherein the step (2) includes collecting read sequence pairs such that the beginning of one read sequence included in each of the read sequence pairs is at the same position in the reference sequence, then collecting, from the collected read sequence pairs, read sequence pairs such that the beginning of the other read sequence included in each of the read sequence pairs is at the same position in the reference sequence, and classifying the collected read sequence pairs into the same group.

**21.** The method according to claim 19 or 20, wherein the step (3) includes collecting, from each of the groups of read sequence pairs, at least one read sequence pair derived from each of the two complementary strands constituting one fragment of the sample DNA, and building consensus of sequence information among read sequences included in the collected read sequence pairs.

**22.** A method for detecting a mutation in genomic DNA, the method comprising:

preparing a sequencing library by the method according to any one of claims 1 to 11 with use of genomic DNA as sample DNA; and
sequencing the sequencing library.

**23.** The method according to claim 22, wherein the sequencing is performed by the method according to any one of claims 13 to 21.

Figure 1

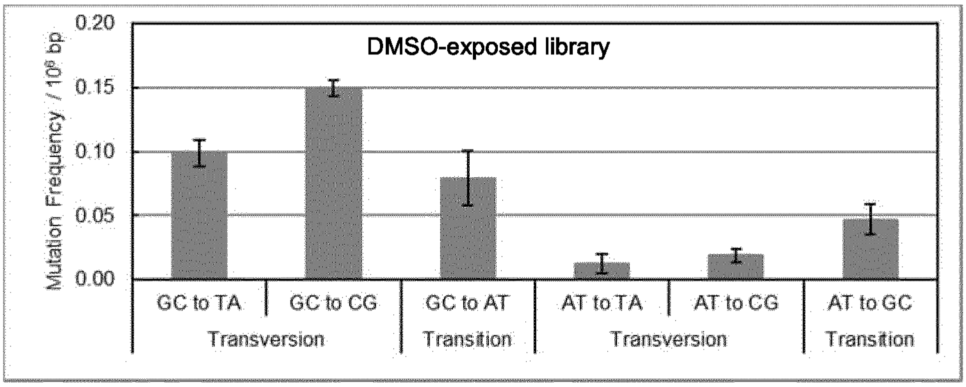


Figure 2

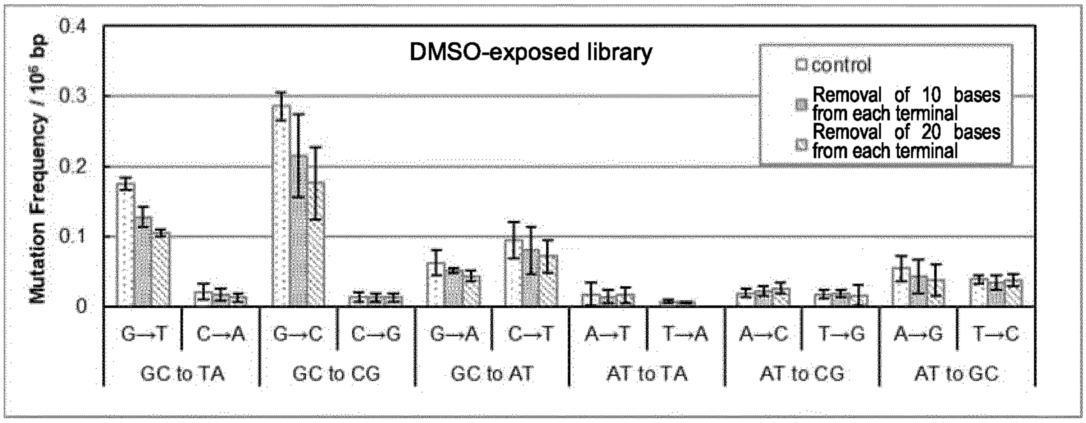


Figure 3

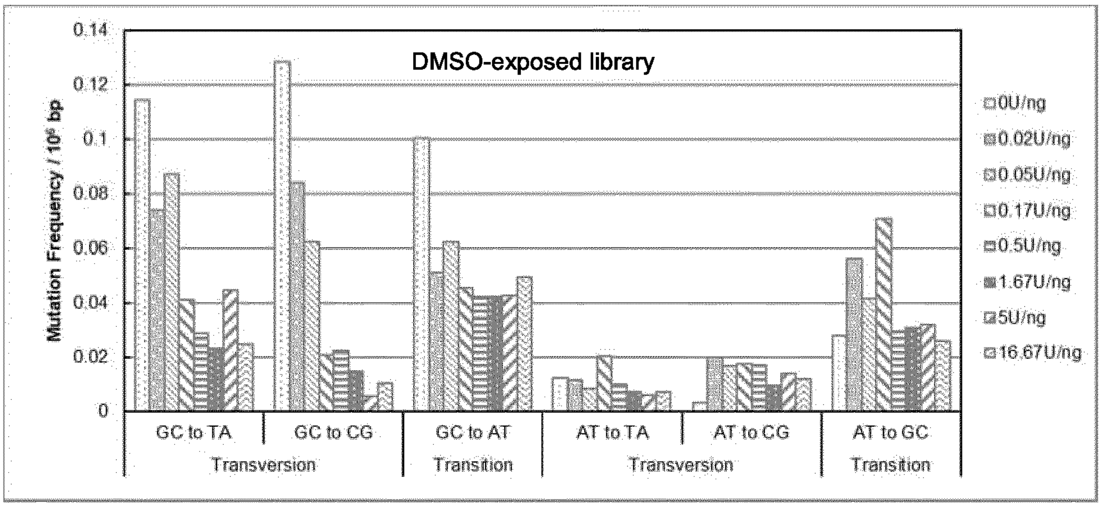

Figure 4

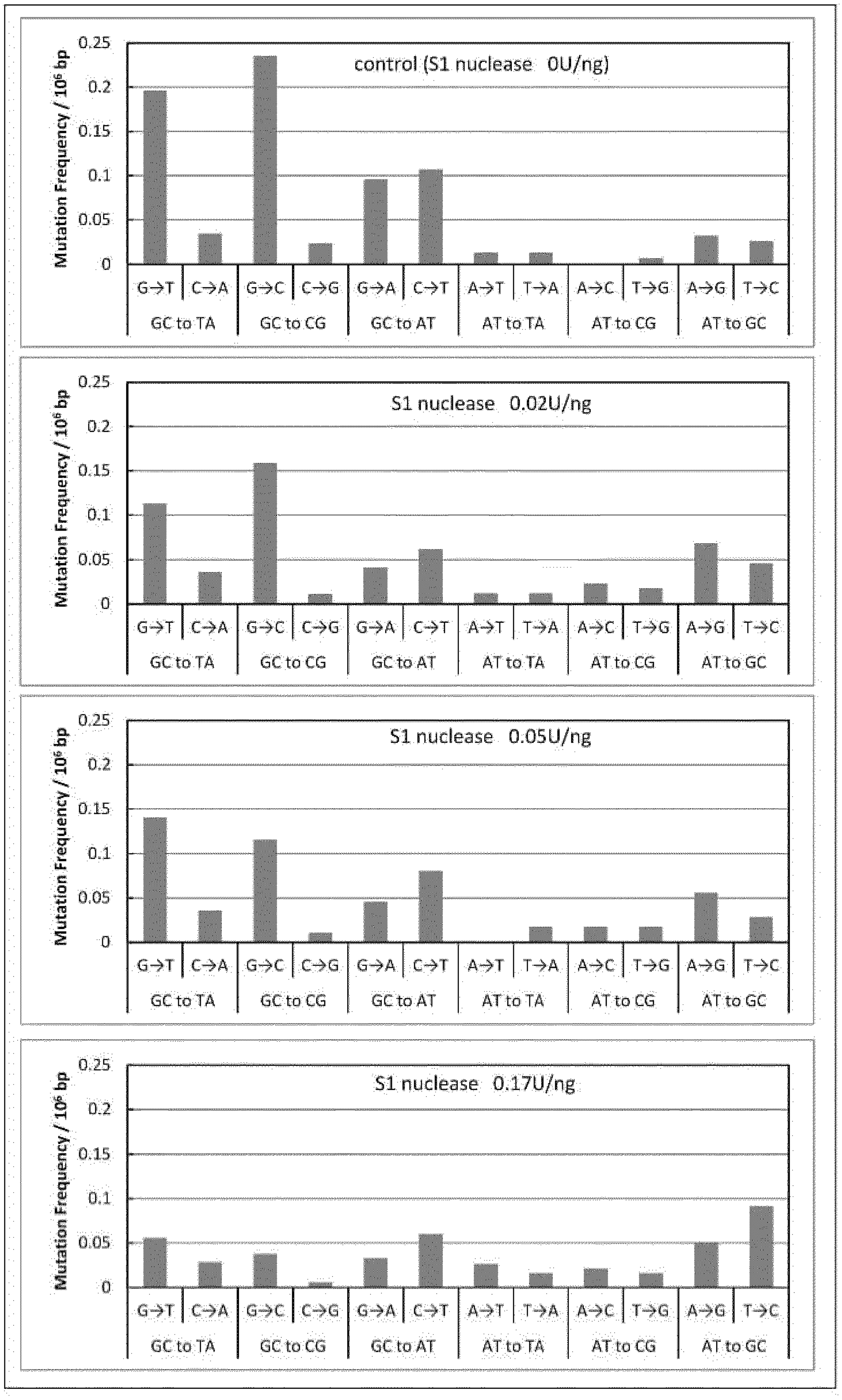
control (S1 nuclease 0U/ng)
S1 nuclease 0.02U/ng
S1 nuclease 0.05U/ng
S1 nuclease 0.17U/ng
Mutation Frequency / 10⁶ bp
0.25
0.2
0.15
0.1
0.05
0
G→T
C→A
G→C
C→G
G→A
C→T
A→T
T→A
A→C
T→G
A→G
T→C
GC to TA
GC to CG
GC to AT
AT to TA
AT to CG
AT to GC

Figure 5

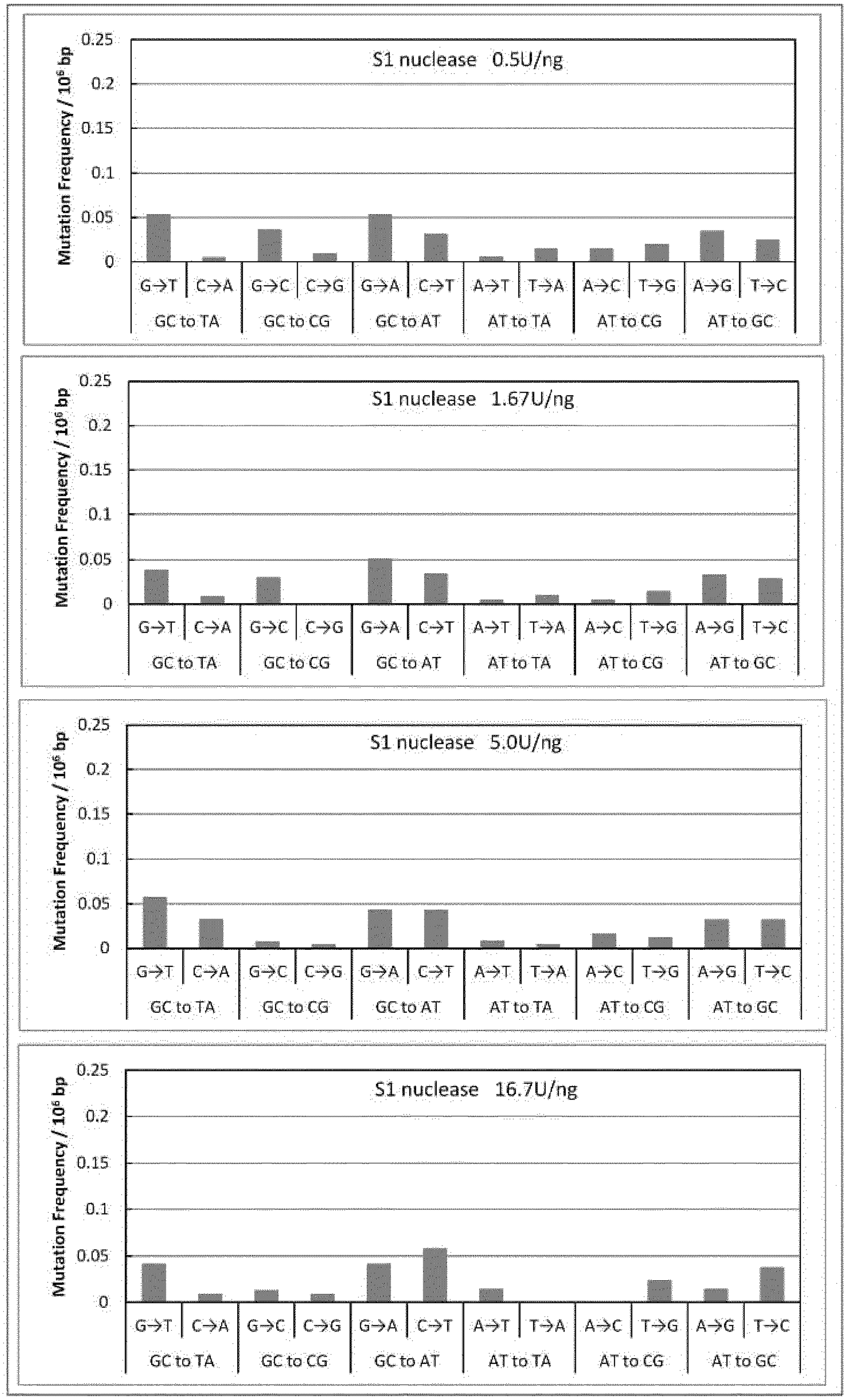
S1 nuclease 0.5U/ng
S1 nuclease 1.67U/ng
S1 nuclease 5.0U/ng
S1 nuclease 16.7U/ng
Mutation Frequency / 10⁶ bp
0.25
0.2
0.15
0.1
0.05
0
G→T
C→A
G→C
C→G
G→A
C→T
A→T
T→A
A→C
T→G
A→G
T→C
GC to TA
GC to CG
GC to AT
AT to TA
AT to CG
AT to GC

Figure 6

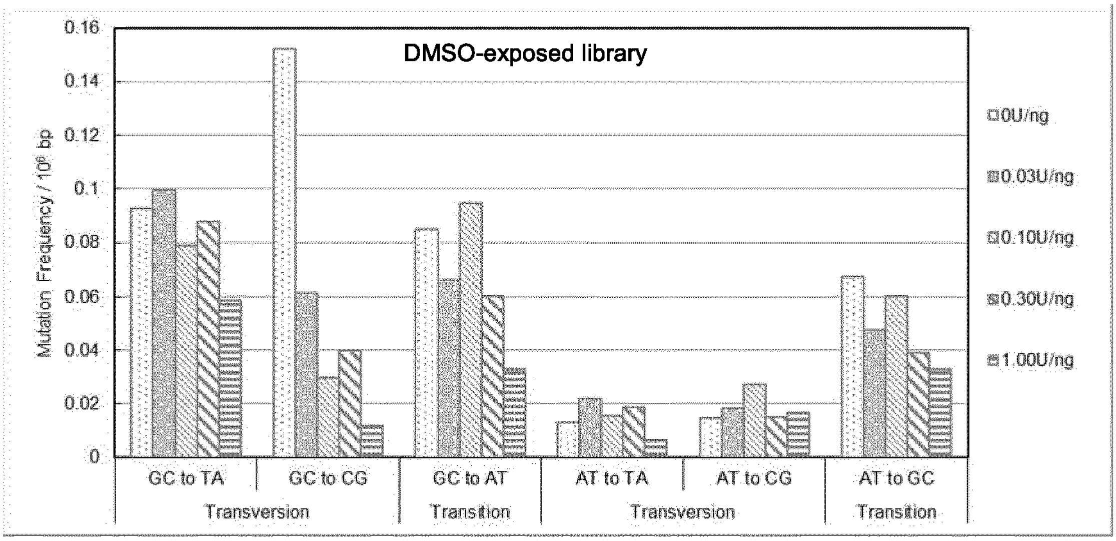
DMSO-exposed library
Mutation Frequency / $10^6$ bp
0.16
0.14
0.12
0.1
0.08
0.06
0.04
0.02
0
0U/ng
0.03U/ng
0.10U/ng
0.30U/ng
1.00U/ng
GC to TA
GC to CG
GC to AT
AT to TA
AT to CG
AT to GC
Transversion
Transition
Transversion
Transition

Figure 7

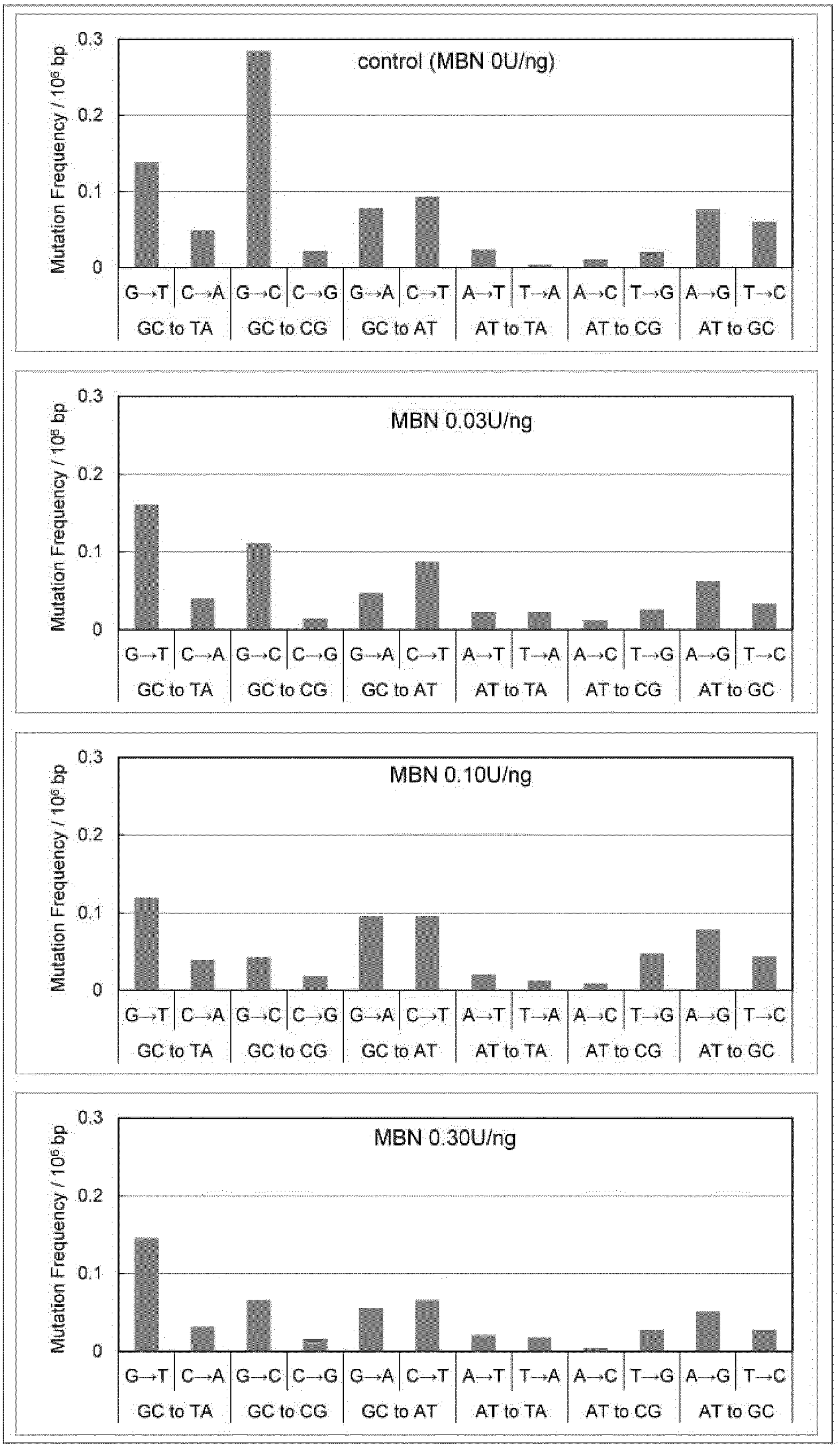
control (MBN 0U/ng)
MBN 0.03U/ng
MBN 0.10U/ng
MBN 0.30U/ng
Mutation Frequency / 10⁶ bp
0
0.1
0.2
0.3
G→T
C→A
G→C
C→G
G→A
C→T
A→T
T→A
A→C
T→G
A→G
T→C
GC to TA
GC to CG
GC to AT
AT to TA
AT to CG
AT to GC

Figure 8

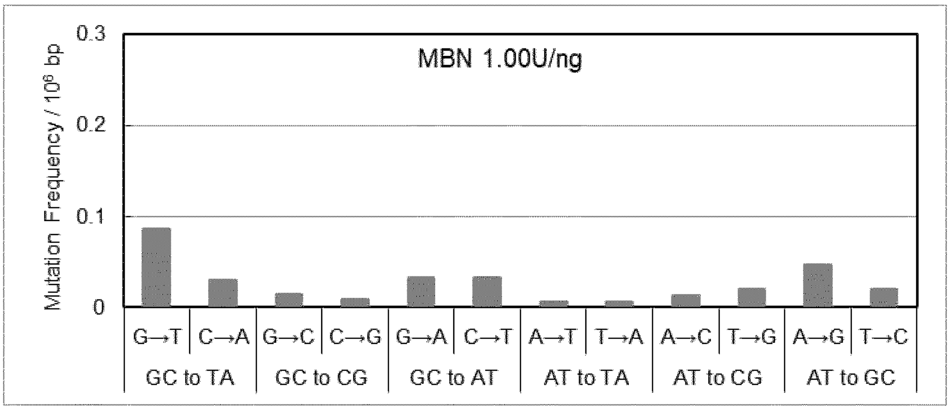


Figure 9

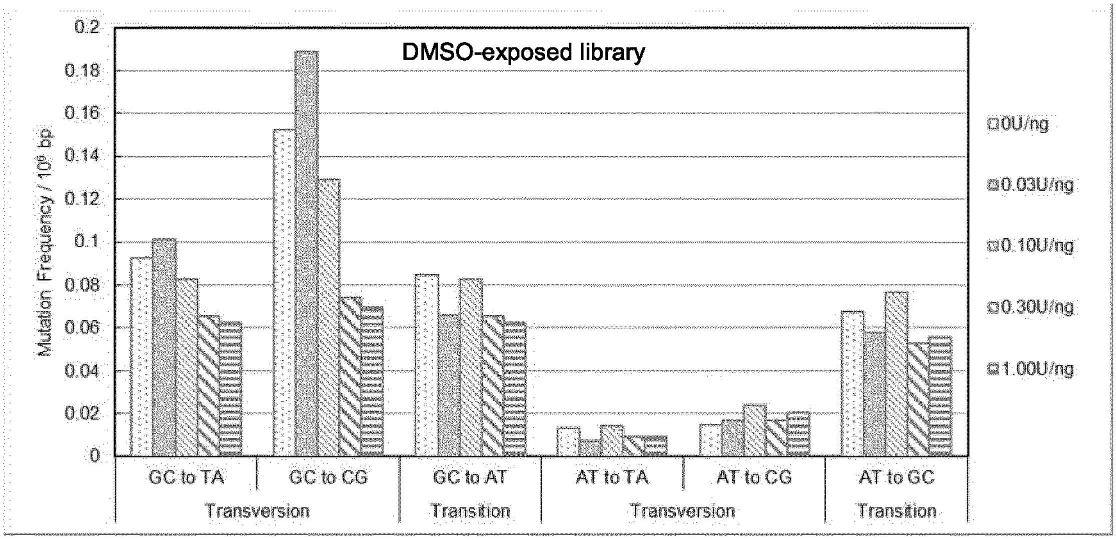

Figure 10

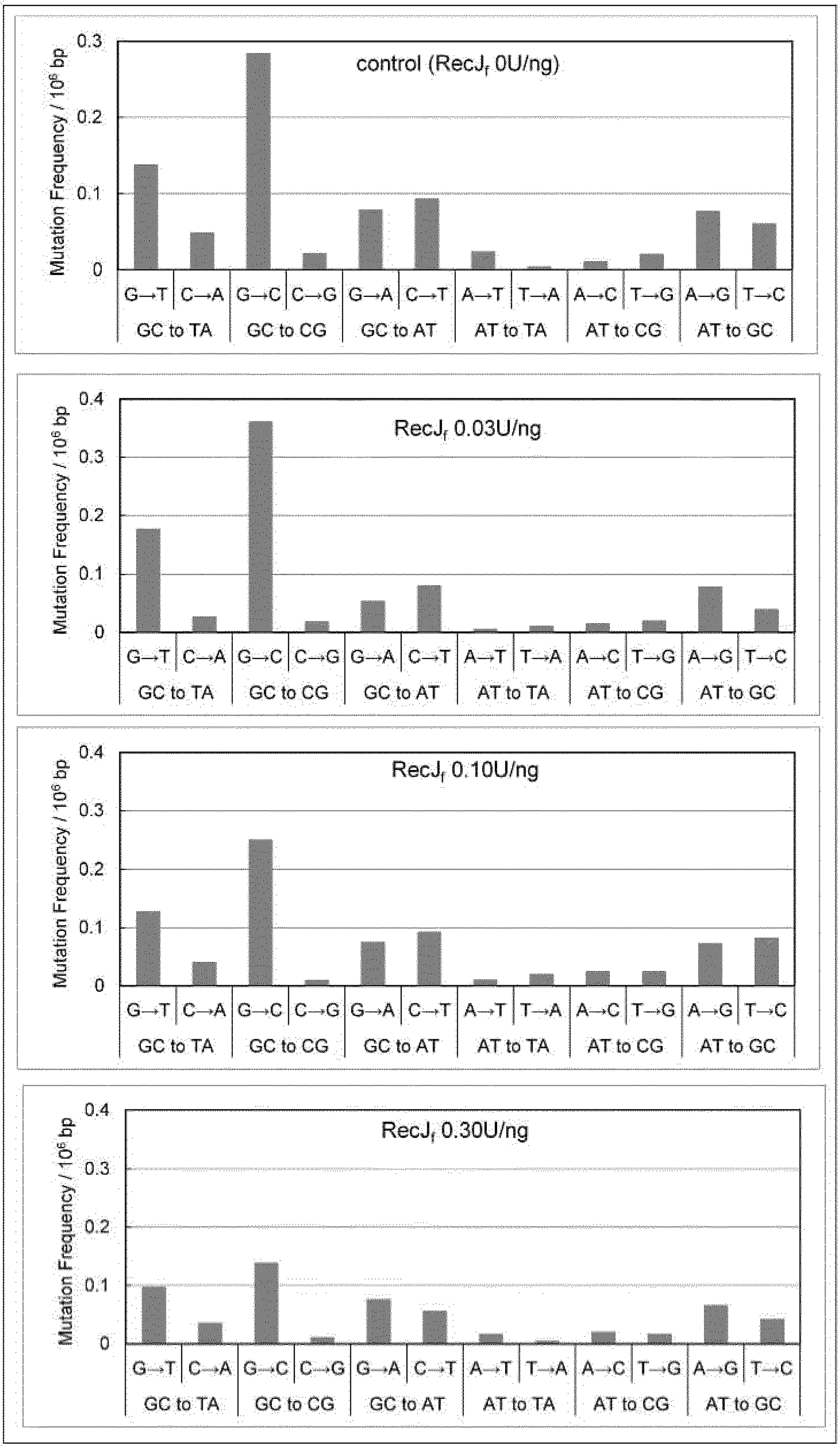
control (RecJ$_f$ 0U/ng)
RecJ$_f$ 0.03U/ng
RecJ$_f$ 0.10U/ng
RecJ$_f$ 0.30U/ng
Mutation Frequency / 10$^6$ bp
G→T
C→A
G→C
C→G
G→A
C→T
A→T
T→A
A→C
T→G
A→G
T→C
GC to TA
GC to CG
GC to AT
AT to TA
AT to CG
AT to GC

Figure 11

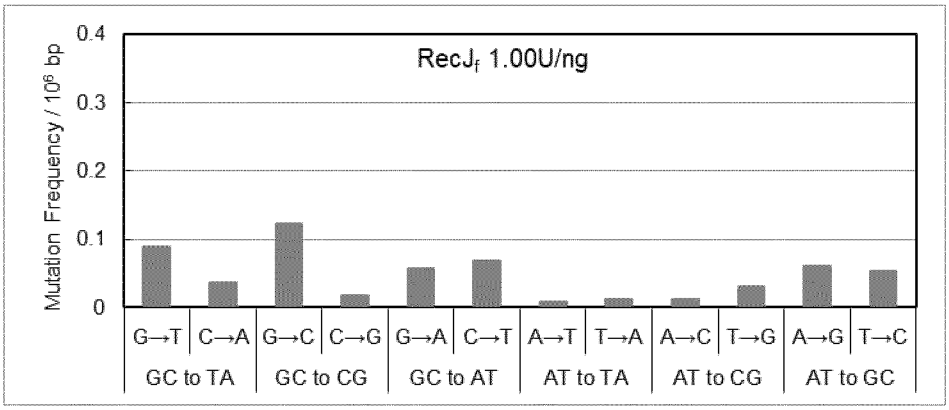


Figure 12

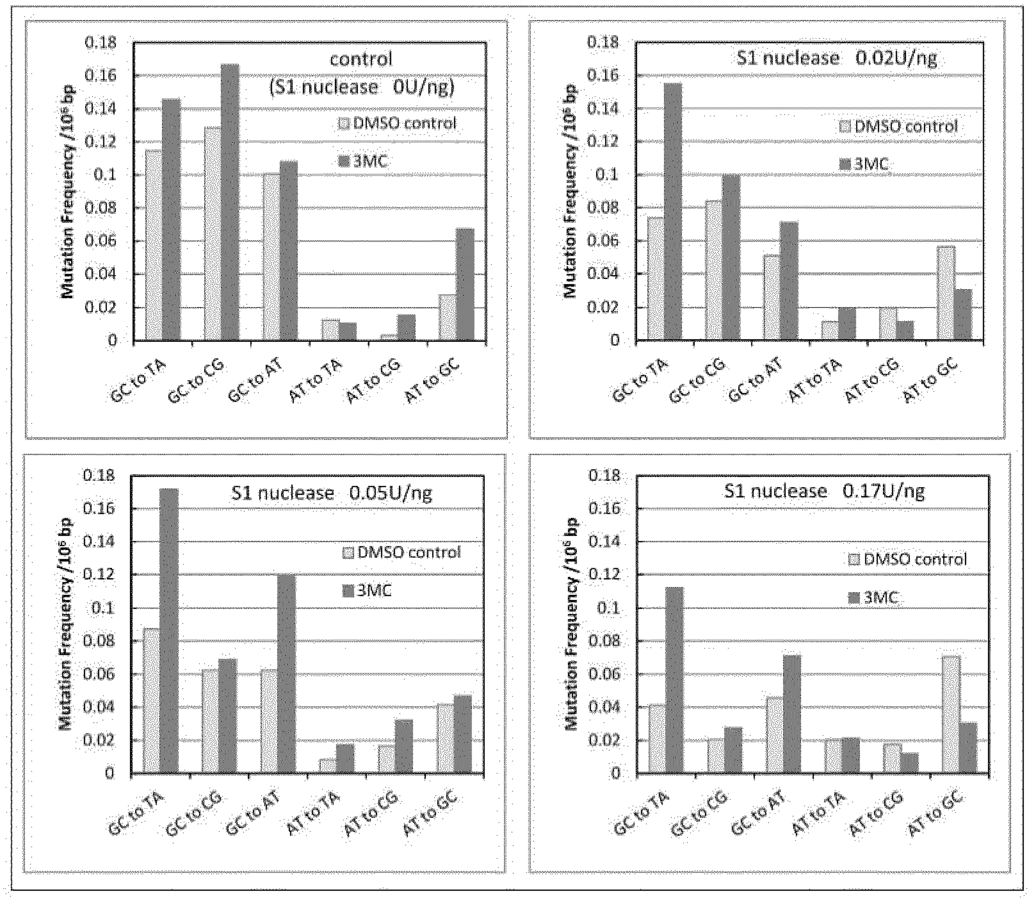

Figure 13

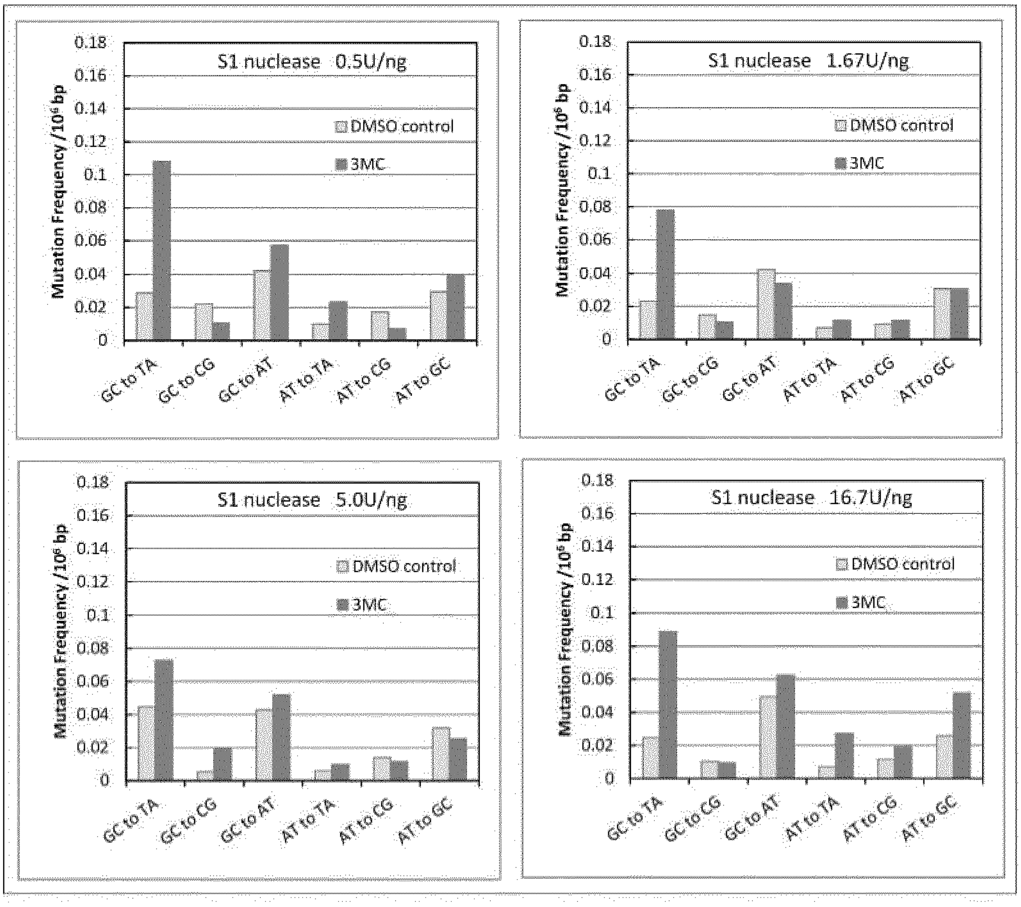
S1 nuclease 0.5U/ng
S1 nuclease 1.67U/ng
S1 nuclease 5.0U/ng
S1 nuclease 16.7U/ng
Mutation Frequency /10⁶ bp
DMSO control
3MC
GC to TA
GC to CG
GC to AT
AT to TA
AT to CG
AT to GC
0
0.02
0.04
0.06
0.08
0.1
0.12
0.14
0.16
0.18

Figure 14

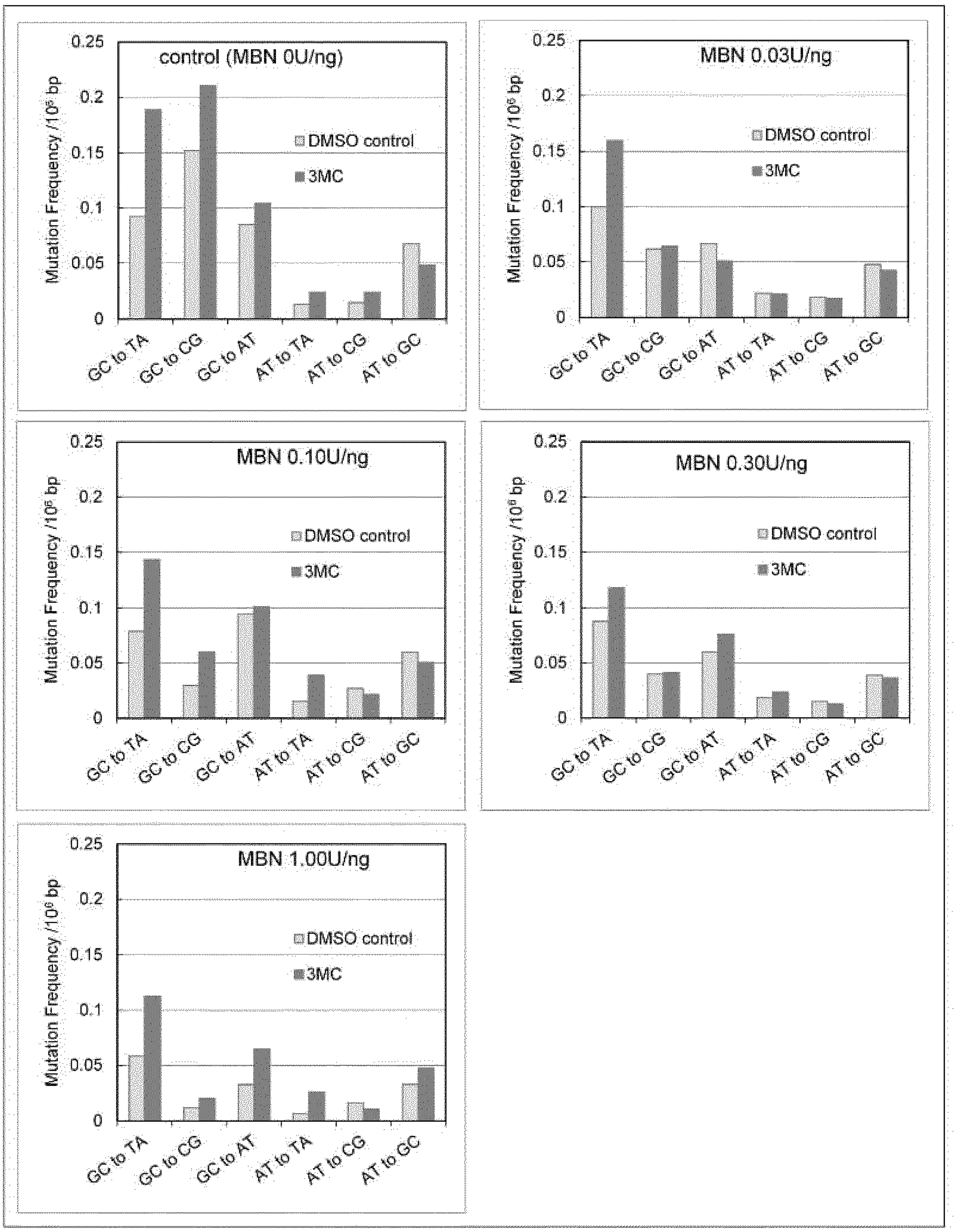
control (MBN 0U/ng)
MBN 0.03U/ng
MBN 0.10U/ng
MBN 0.30U/ng
MBN 1.00U/ng
DMSO control
3MC
Mutation Frequency /10⁶ bp
0.25
0.2
0.15
0.1
0.05
0
GC to TA
GC to CG
GC to AT
AT to TA
AT to CG
AT to GC

Figure 15

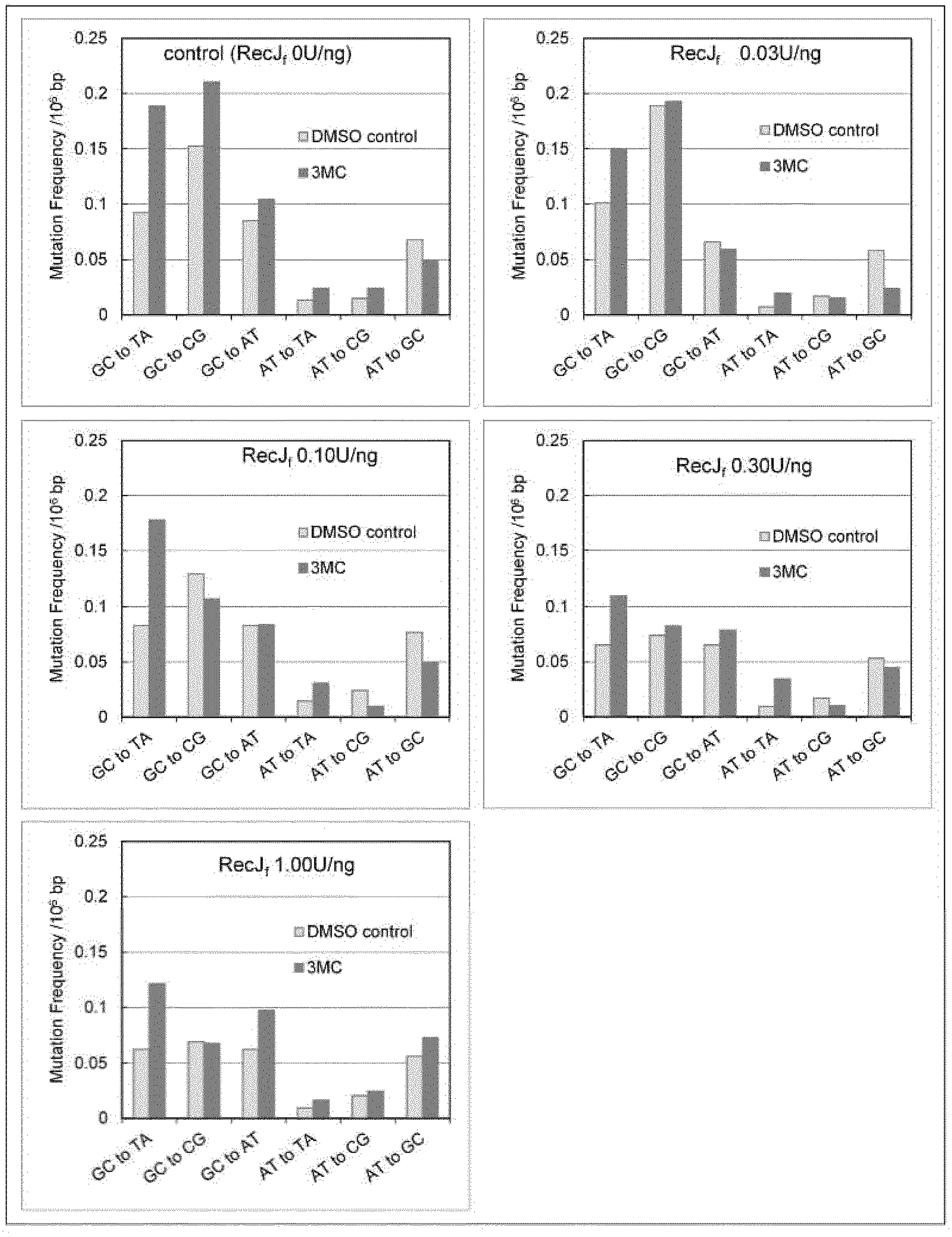
control (RecJf 0U/ng)
RecJf 0.03U/ng
RecJf 0.10U/ng
RecJf 0.30U/ng
RecJf 1.00U/ng
Mutation Frequency /10^6 bp
DMSO control
3MC
0.25
0.2
0.15
0.1
0.05
0
GC to TA
GC to CG
GC to AT
AT to TA
AT to CG
AT to GC

Figure 16

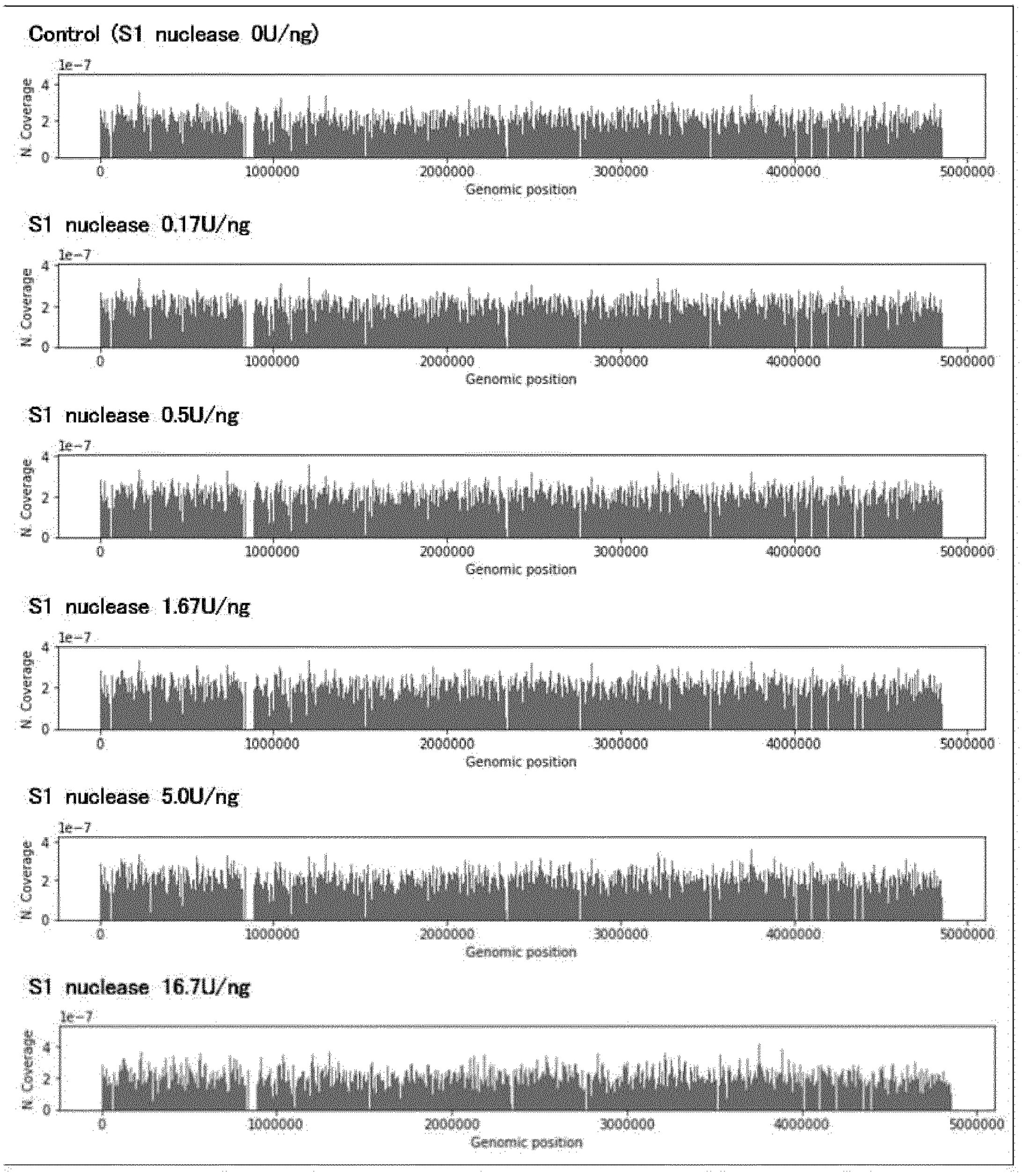
Control (S1 nuclease 0U/ng)
S1 nuclease 0.17U/ng
S1 nuclease 0.5U/ng
S1 nuclease 1.67U/ng
S1 nuclease 5.0U/ng
S1 nuclease 16.7U/ng
N. Coverage
1e−7
0
2
4
Genomic position
0
1000000
2000000
3000000
4000000
5000000

Figure 17

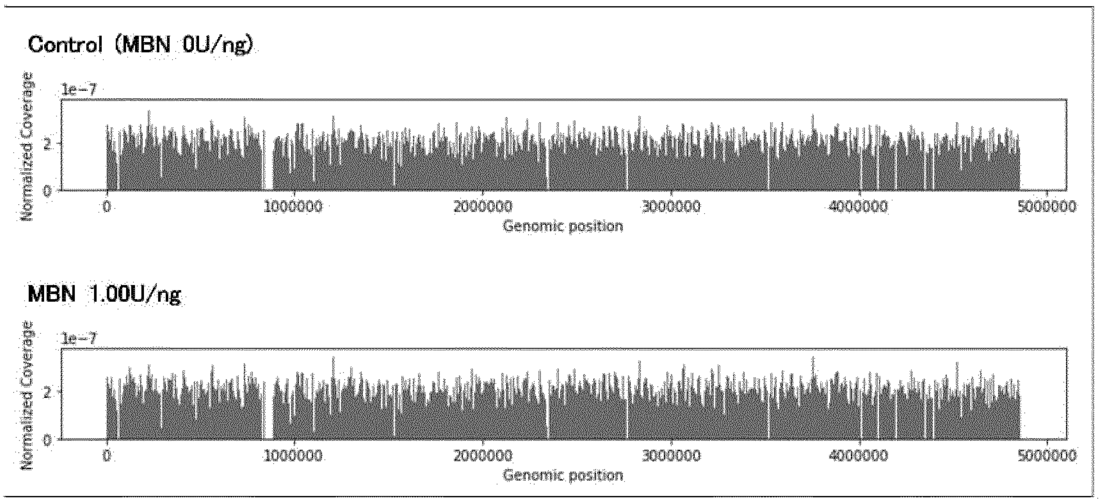


Figure 18

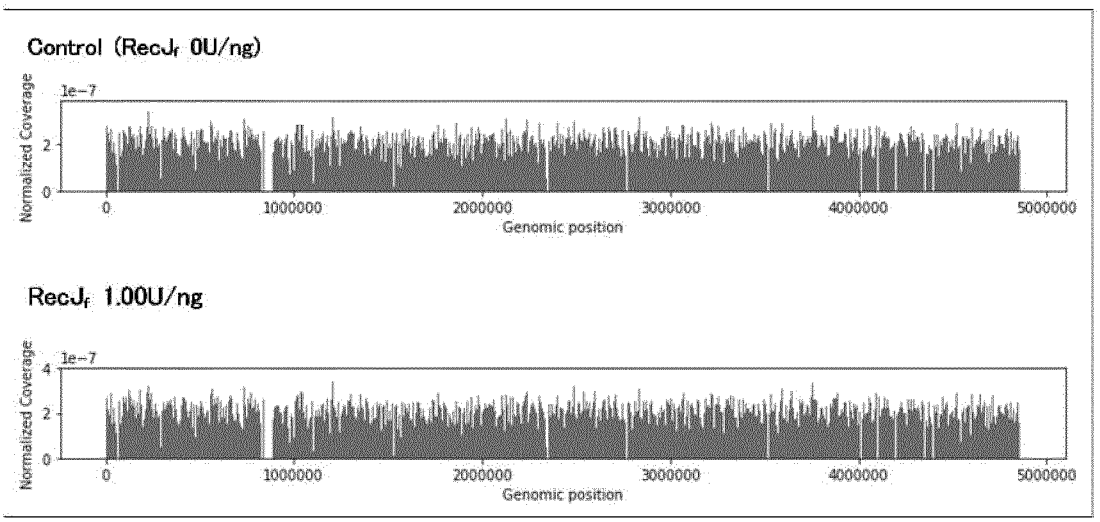

Figure 19

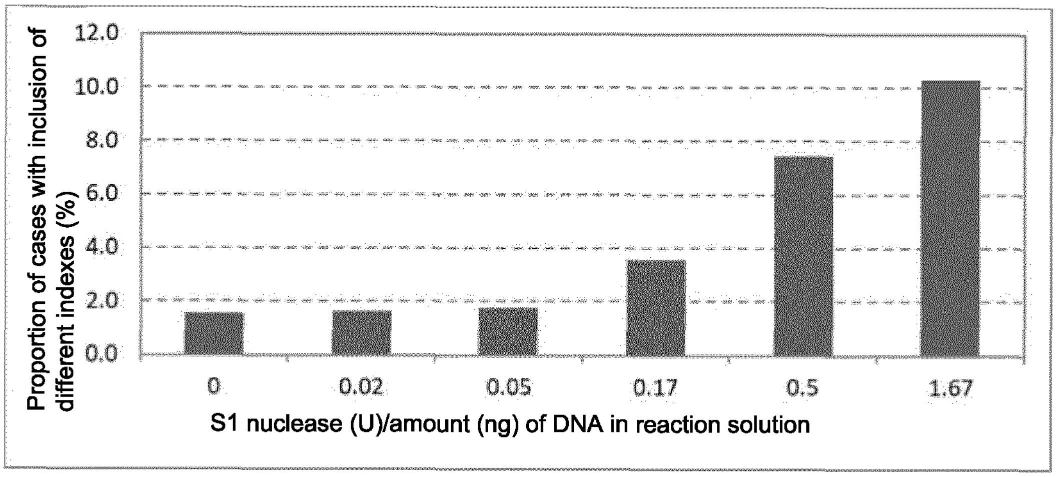


Figure 20

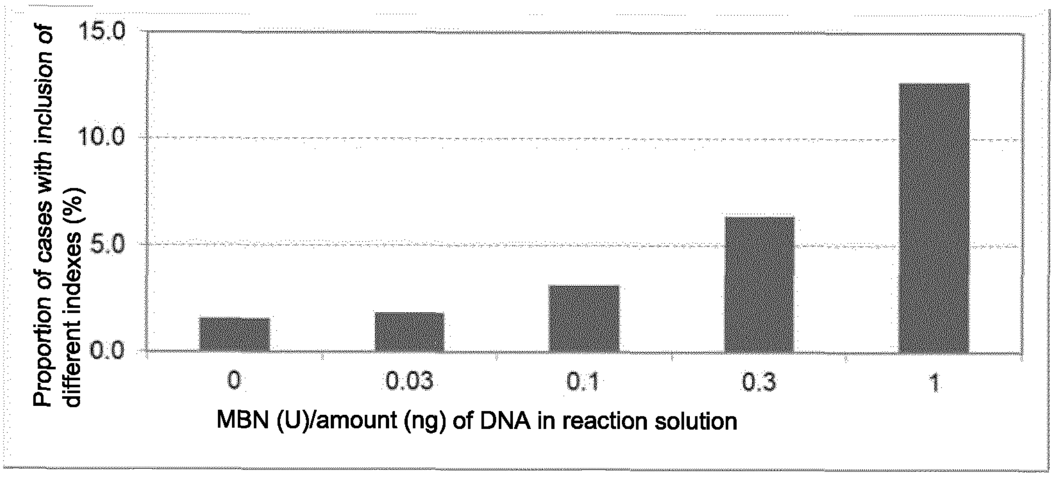

Figure 21

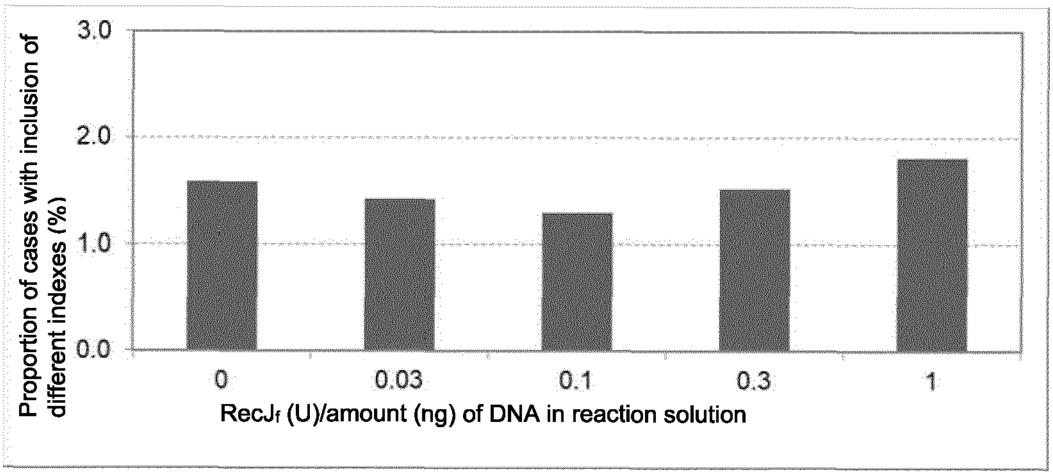


Figure 22

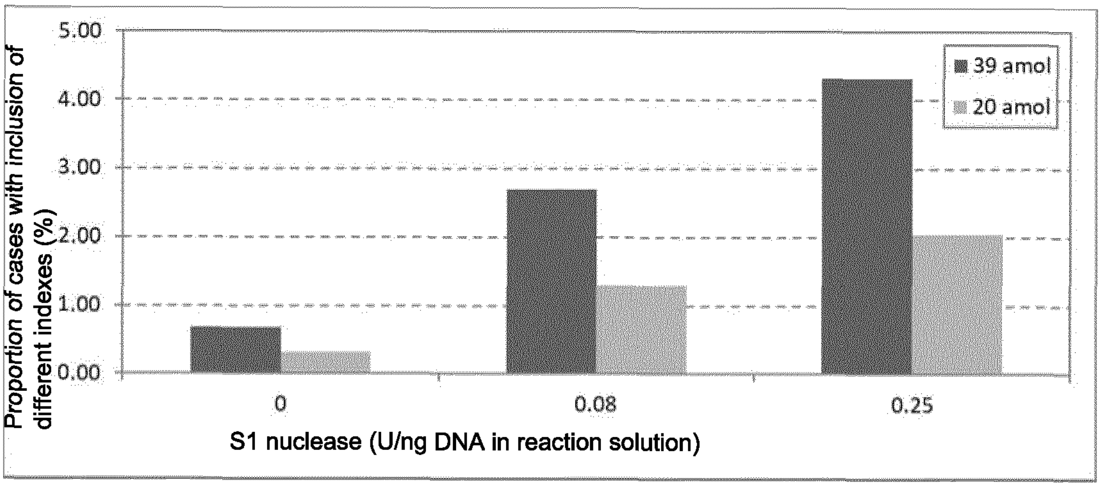

Figure 23

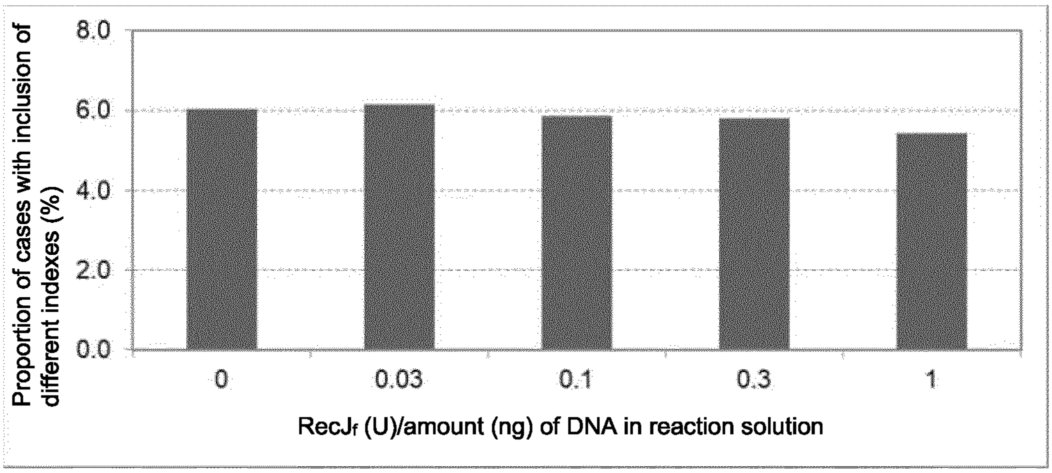


Figure 24

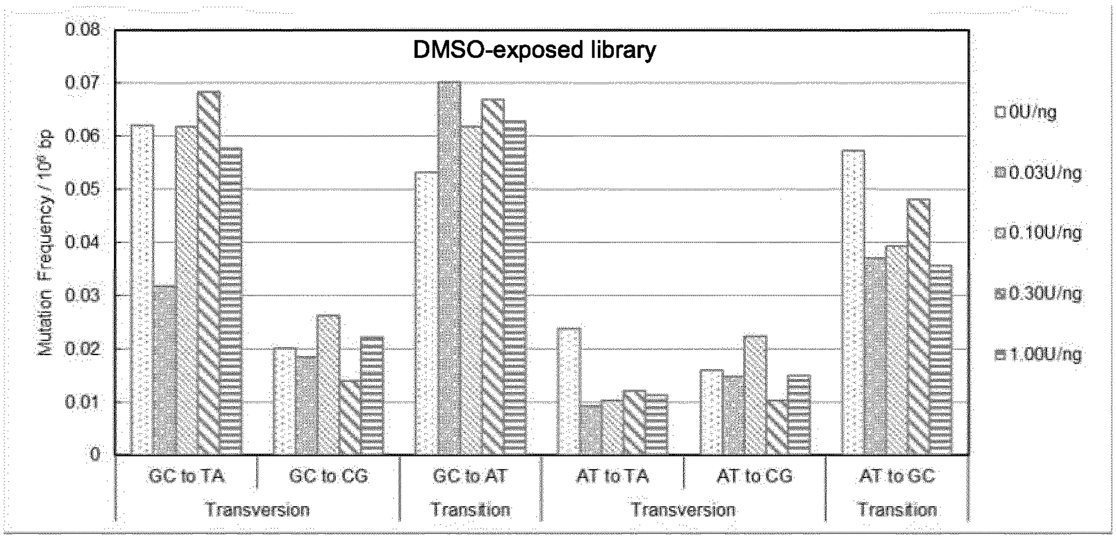

# INTERNATIONAL SEARCH REPORT

International application No.
PCT/JP2020/042487

A. CLASSIFICATION OF SUBJECT MATTER
C40B 40/06(2006.01)i; C12Q 1/48(2006.01)i; C12Q 1/6827(2018.01)i; C12Q 1/6869(2018.01)i; C12N 9/16(2006.01)i; G01N 33/48(2006.01)i; G01N 33/50(2006.01)i
FI: C40B40/06; G01N33/48 P; G01N33/50 P; C12Q1/6869 Z; C12Q1/48 Z; C12Q1/6827 Z; C12N9/16 Z
According to International Patent Classification (IPC) or to both national classification and IPC

B. FIELDS SEARCHED
Minimum documentation searched (classification system followed by classification symbols)
C40B40/06; C12Q1/48; C12Q1/6827; C12Q1/6869; C12N9/16; G01N33/48; G01N33/50

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2005/042781 A2 (AGENCOURT BIOSCIENCE CORPORATION) 12 May 2005 (2005-05-12) example 1 | 1-12<br>1-23 |
| Y | JP 2019-193612 A (KAO CORP.) 07 November 2019 (2019-11-07) claims 1-29 | 1-23 |
| A | WO 2015/118077 A1 (FRAUNHOFER-GESELLSCHAFT ZUR FORDERUNG DER ANGEWANDTEN FORSCHUNG E. V.) 13 August 2015 (2015-08-13) claims 1-18, p. 25 | 1-23 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 January 2021 (07.01.2021) | 19 January 2021 (19.01.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/042487

| Patent Documents referred in the Report | Publication Date | Patent Family | Publication Date |
|---|---|---|---|
| WO 2005/042781 A2 | 12 May 2005 | US 2006/0024681 A1<br>example 1<br>EP 1682680 A2 | |
| JP 2019-193612 A | 07 Nov. 2019 | WO 2019/208827 A1<br>claims 1-29 | |
| WO 2015/118077 A1 | 13 Aug. 2015 | JP 2017-509324 A<br>claims 1-18,<br>paragraph [0090]<br>US 2016/0348164 A1<br>EP 3102702 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015057985 A
- WO 2019126803 A
- WO 2013142389 A
- WO 2019208827 A
- WO 2018150513 A

### Non-patent literature cited in the description

- *Nucleic Acids Research,* 2019, vol. 47 (2), e12
- *Nucleic Acids Research,* 2013, vol. 41 (6), e67
- *Nature Protocols,* 2014, vol. 9 (11), 2586-2606
- *PNAS,* 2012, vol. 109 (36), 14508-14513
- *Mol. Mech. Mutagen.,* 2000, vol. 455, 29-60
- *Sci. Rep.,* vol. 8 (1), 9583
- *CHEMICAL ABSTRACTS,* 56-49-5
- *Environ. Mol. Mutagen.,* 2000, vol. 36, 266-273
- *J. Appl. Toxicol.,* 2017, vol. 37, 1125-1128